(19) Europäisches Patentamt
European Patent Office
Office européen des brevets

(11) EP 2 760 878 B1

(12) **EUROPÄISCHE PATENTSCHRIFT**

(45) Veröffentlichungstag und Bekanntmachung des Hinweises auf die Patenterteilung:
**28.10.2015 Patentblatt 2015/44**

(21) Anmeldenummer: **12766636.0**

(22) Anmeldetag: **24.09.2012**

(51) Int Cl.:
***C07J 43/00*** *(2006.01)* ***A61K 31/58*** *(2006.01)*
***A61P 5/24*** *(2006.01)*

(86) Internationale Anmeldenummer:
**PCT/EP2012/068803**

(87) Internationale Veröffentlichungsnummer:
**WO 2013/045407 (04.04.2013 Gazette 2013/14)**

(54) **ESTRA-1,3,5(10),16-TETRAEN-3-CARBOXAMID-DERIVATE, VERFAHREN ZU IHRER HERSTELLUNG, PHARMAZEUTISCHE PRÄPARATE, DIE DIESE ENTHALTEN, SOWIE DEREN VERWENDUNG ZUR HERSTELLUNG VON ARZNEIMITTELN**

ESTRA-1,3,5(10),16-TETRAENE-3-CARBOXAMIDE DERIVATIVES, PROCESS FOR PREPARATION THEREOF, PHARMACEUTICAL PREPARATIONS COMPRISING THEM, AND USE THEREOF FOR PRODUCTION OF MEDICAMENTS

DÉRIVÉS D'ESTRA-1,3,5(10),16-TÉTRAÈNE-3-CARBOXAMIDE, PROCÉDÉ POUR LEUR FABRICATION, PRÉPARATIONS PHARMACEUTIQUES CONTENANT CES SUBSTANCES AINSI QUE LEUR UTILISATION DANS LA FABRICATION DE MÉDICAMENTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.09.2011 DE 102011083725**

(43) Veröffentlichungstag der Anmeldung:
**06.08.2014 Patentblatt 2014/32**

(73) Patentinhaber:
- **Bayer Intellectual Property GmbH
40789 Monheim (DE)**
- **Bayer Pharma Aktiengesellschaft
13353 Berlin (DE)**

(72) Erfinder:
- **BOTHE, Ulrich
13187 Berlin (DE)**
- **BARAK, Naomi
12049 Berlin (DE)**
- **BUSEMANN, Matthias
13467 Berlin (DE)**
- **FISCHER, Oliver Martin
12099 Berlin (DE)**
- **ROTGERI, Andrea
13503 Berlin (DE)**
- **GASHAW, Isabella
San Francisco, CA 94158-0000 (US)**
- **HARTUNG, Ingo
13158 Berlin (DE)**
- **MARQUARDT, Tobias
42115 Wuppertal (DE)**

(74) Vertreter: **BIP Patents
c/o Bayer Intellectual Property GmbH
Alfred-Nobel-Straße 10
40789 Monheim am Rhein (DE)**

(56) Entgegenhaltungen:
**WO-A1-2008/065100 US-A- 5 604 213
US-B1- 6 541 463**

- **DELUCA D ET AL: "Inhibitory effects of fluorine-substituted estrogens on the activity of 17beta-hydroxysteroid dehydrogenases", MOLECULAR AND CELLULAR ENDOCRINOLOGY, ELSEVIER IRELAND LTD, IE, Bd. 248, Nr. 1-2, 27. März 2006 (2006-03-27), Seiten 218-224, XP027882680, ISSN: 0303-7207 [gefunden am 2006-03-27] in der Anmeldung erwähnt**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



**(Forts. nächste Seite)**

- **PENNING T M ET AL: "Structure-function aspects and inhibitor design of type 5 17beta-hydroxysteroid dehydrogenase (AKR1C3)", MOLECULAR AND CELLULAR ENDOCRINOLOGY, ELSEVIER IRELAND LTD, IE, Bd. 171, Nr. 1-2, 22. Januar 2001 (2001-01-22), Seiten 137-149, XP027339511, ISSN: 0303-7207 [gefunden am 2001-01-22] in der Anmeldung erwähnt**
- **DESCOMPS BERNARD ET AL: "Etude du site actif de la 17-.beta.-hydroxysteroide: NAD(P) oxydoreductase soluble du placenta humain", BULLETIN DE LA SOCIETE DE CHIMIE BIOLOGIGUE, MASSON, PARIS, FR, Bd. 51, Nr. 12, 1. Januar 1970 (1970-01-01), Seiten 1591-1611, XP008126557, ISSN: 0037-9042**

## Beschreibung

Die Erfindung betrifft AKR1C3 Inhibitoren und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Blutungsbeschwerden und Endometriose.

AKR1C3 ist ein multifunktionales Enzym und katalysiert unter anderem die Reduktion von 4-Androsten-3,17-dion (einem schwachen Androgen) zu Testosteron (einem potenten Androgen) und von Estron (einem schwachen Estrogen) zu 17β-Estradiol (einem starken Estrogen). Zusätzlich wird die Reduktion von Prostaglandin (PG) H2 zu PGF2α und PGD2 zu 9α,11β-PGF2 inhibiert (T. M. Penning et. al., 2006, 'Aldo-keto reductase (AKR) 1 C3: Role in prostate disease and the development of specific inhibitors', Molecular and Cellular Endocrinology 248(1-2), 182 -191).

Die lokale Bildung von Estradiol (E2) spielt eine zentrale Rolle für die Initiierung und das Fortschreiten von Brustkrebserkrankungen und Endometriose. Die Reduktion der Gewebespiegel von Estrogenen und insbesondere von Estradiol wird erreicht durch die therapeutische Gabe von Aromataseinhibitoren (um die Bildung von Estrogenen aus Androgenen zu inhibieren) und von Sulfataseinhibitoren (um die Bildung von Estron aus Estronsulfat zu blockieren). Beide Therapieansätze haben aber den Nachteil, dass systemische Estrogenspiegel radikal reduziert werden (A. Oster et. al., J. Med. Chem. 2010, 53, 8176-8186). Kürzlich wurde experimentell nachgewiesen, dass endometriotische Läsionen in der Lage sind lokal Estradiol zu synthetisieren (B. Delvoux et al., J Clin Endocrinol Metab. 2009, 94, 876-883). Für den Subtyp der ovariellen Endometriose wurde eine Überexpression der AKR1C3 mRNA beschrieben (T. Smuc et al., Mol Cell Endocrinol. 2009 Mar 25;301(1-2): 59-64).

An der Identifizierung von neuen Inhibitoren des Enzyms Aldo-keto reductase 1C3 (AKR1C3) (Synonyme: Typ 5 17β-hydroxysteroid Dehydrogenase oder Prostaglandin F Synthase) besteht großer Bedarf, da Inhibitoren ein Potential für die Behandlung von hormonabhängigen Erkrankungen wie zum Beispiel Endometriose aber auch für die Behandlung von hormonunabhängigen Erkrankungen haben (M.C. Byrns, Y. Jin, T.M. Penning, Journal of Steroid Biochemistry and Molecular Biology (2010); A. L. Lovering et. al., Cancer Res 64(5), 1802-1810). Neben der Endometriose zählen dazu auch Prostatakrebs (K. M. Fung et al., Endocr Relat Cancer 13(1), 169-180), Prostatahyperplasie (R. O. Roberts et al., Prostate 66(4), 392-404), Endometriumskarzinom (T. L. Rizner et al., Mol Cell Endocrinol 2006 248(1-2), 126-135), polyzystisches ovarielles Syndrom (K. Qin et al., J Endocrinol Metab 2006, 91(1), 270-276), Lungenkarzinom (Q. Lan et al., Carcinogenesis 2004, 25(11), 2177-2181), non-Hodgkin-Lymphom (Q. Lan et al., Hum Genet 2007, 121(2), 161-168), Haarausfall (L. Colombe et al., Exp Dermatol 2007, 16(9), 762-769), Adipositas (P. A. Svensson et al., Cell Mol Biol Lett 2008, 13(4), 599-613), Blasenkarzinom (J. D. Figueroa, Carcinogenesis 2008, 29(10), 1955-1962), chronische myeloische Leukäme (J. Birthwistle, Mutat Res 2009, 662(1-2), 67-74), Nierenzellkarzinom (J. T. Azzarello, Int J Clin Exp Pathol 2009, 3(2), 147-155), Brustkrebs (M. C. Byms, J Steroide Biochem Mol Biol 2010, 118(3), 177-187), die frühzeitige Geschlechtsreife (C. He, Hum Genet 2010, 128(5), 515-527) und die chronisch-obstruktive Lungenerkrankung (S. Pierrou, Am J Respir Crit Care 2007, 175(6), 577-586).

Einige Inhibitoren von AKR1C3 sind bekannt (Übersichtsartikel: Joanna M Day, Helena J Tutill, Atul Purohit und Michael J Reed, Endocrine-Related Cancer (2008) 15, 665-692). Als steroidale Substanz wurde zum Beispiel EM-1404 basierend auf dem Estratrien-Gerüst mit einer Spirolactoneinheit an Position 17 beschrieben (F. Labrie et al. US Patent 6,541,463, 2003).

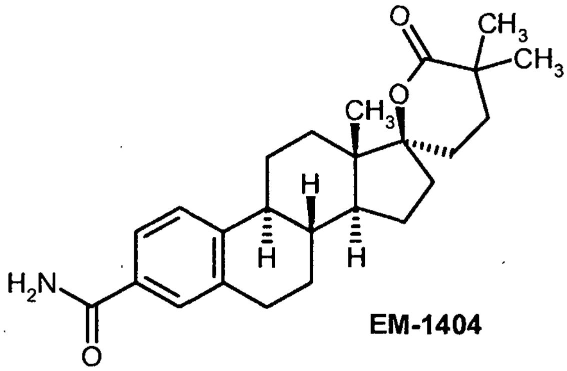

**EM-1404**

Weitere steroidale Substanzen mit Lactoneinheit findet man in P. Bydal, Van Luu-The, F. Labrie, D. Poirier, European Journal of Medicinal Chemistry 2009, 44, 632-644. Fluorierte Estratrienderivate wurden beschrieben in D. Deluca, G. Moller, A. Rosinus, W. Elger, A. Hillisch, J. Adamski, Mol. Cell. Endocrinol. 2006, 248, 218 - 224.

Bei den erfindungsgemäßen Verbindungen handelt es sich um Substanzen basierend auf einem Estra-1,3,5(10),16-tetraen-Gerüst substituiert mit einem aromatischen Heterocyclus an Position 17 und einer Aminocarbonyl-Gruppe an Position 3. In S. E. Barrie et al. US 5604213 werden 17-(3-Pyridyl)estra-1,3,5(10),16-tetraen-3-ol Derivate

als 17α-Hydroxylase/C17-20 Lyase (Cyp17A1) Inhibitoren beschrieben. Insbesondere wird über die Substanz 17-(3-Pyridyl)estra-1,3,5(10),16-tetraen-3-ol berichtet. Es werden jedoch in US 5604213 keine 17-(3-Pyridyl)estra-1,3,5(10),16-tetraen-Derivate substituiert mit einer Aminocarbonyl-Gruppe an 3-Position beschrieben. Die hier beanspruchten, erfindungsgemäßen Verbindungen weisen zusätzlich als funktionelle Gruppe eine Carboxylgruppe auf, wodurch sich ein weiterer struktureller Unterschied zu den in US 5604213 beschriebenen Substanzen ergibt. Überraschenderweise wurde nun gefunden, dass die hier beanspruchten, erfindungsgemäßen Verbindungen potente Inhibitoren von AKR1C3 (vergl. Beispiel 31) sind, dabei aber nur sehr schwach oder gar nicht Cyp17A1 inhibieren (vergl. Beispiel 32).

Estra-1,3,5(10),16-tetraen-Derivate substituiert mit einer Aminocarbonyl ($-CONH_2$) Gruppe an 3-Position werden beschrieben in US2005/0203075. Diese Derivate sind jedoch nicht mit einem Heterocyclus an Position 17 des Estra-1,3,5(10),16-tetraen-Gerüstes substituiert.

Eine Übersicht über 17-Pyridyl- und 17-Pyrimidylandrostan-Derivate, die als Cyp17A1 Inhibitoren beschrieben werden, findet man in V. M. Moreira et al. Current Medicinal Chemistry, 2008 Vol. 15, No. 9.

Aufgabe der vorliegenden Erfindung ist es alternative, als AKR1C3 Inhibitoren wirkende Stoffe zur Verfügung zu stellen. Die hier beanspruchten, neuen AKR1C3 Inhibitoren zeigen zusätzlich im Vergleich zum bekannten AKR1C3 Inhibitor EM-1404 eine deutlich verbesserte Wasserlöslichkeit (vergl. Beispiel 33). Dadurch ist die Formulierbarkeit der erfindungsgemäßen Verbindungen in wässrigen Applikationsmedium verbessert.

Gegenstand der vorliegenden Erfindung sind Verbindungen der Formel (I):

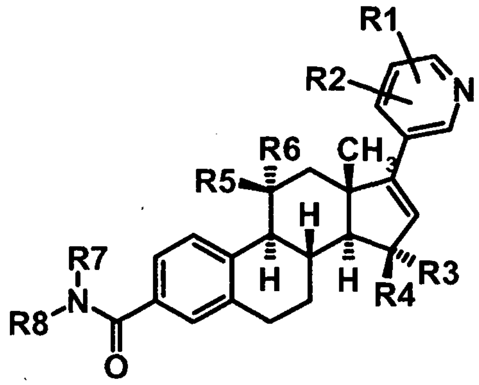

(I)

worin

| | |
|---|---|
| R1 und R2 | unabhängig voneinander Wasserstoff, Fluor, Chlor, Nitril, Trifluormethyl, Pentafluorethyl, Methoxy, Ethoxy, Trifluormethoxy, $-OCH_2CF_3$, $CH_3SO_2$-, $CH_3CH_2SO_2$-, $-(C{=}O)CH_3$, Carboxyl, $C_1$-$C_4$-Alkyl, Hydroxyl, $-CH_2OH$, $-C(CH_3)_2OH$, $-CONH_2$, - (C=O)NHAlkyl, $-(C{=}O)N(CH_3)_2$, $-SO_2NH_2$, $-SO_2NHCH_3$, $-SO_2N(CH_3)_2$ oder den Ersatz einer C-H Gruppe im Pyridinring durch ein Stickstoffatom und |
| R3 und R4 | Wasserstoff oder<br>R3 Hydroxyl, Fluor, Methoxy oder Ethoxy und R4 Wasserstoff oder<br>R3 Wasserstoff und R4 für Hydroxyl, Fluor, Methoxy oder Ethoxy und |
| R5 und R6 | Wasserstoff oder<br>R5 Fluor, Hydroxyl, Methoxy oder Ethoxy und R6 Wasserstoff oder<br>R5 Wasserstoff und R6 Fluor und |
| R7 | Wasserstoff, $C_1$-$C_4$-Alkyl, $C_3$-$C_6$-Cycloalkyl, Cyclopropylmethyl, Trifluormethyl oder 2,2,2-Trifluorethyl und |
| R8 | $-CR^aR^b-COOH$ wobei<br>$R^a$ und $R^b$ unabhängig voneinander Wasserstoff, Methyl, Ethyl oder<br>$R^a$ und $R^b$ gemeinsam $-(CH_2)_n-$ mit n = 2, 3, 4 oder 5, wobei bis zu 4 Wasserstoffatome der $CH_2$-Gruppen durch Fluoratome ersetzt sein können oder<br>$R^a$ und $R^b$ gemeinsam $-CH_2-O-CH_2-$, $-CH_2-NH-CH_2-$, $-CH_2-N(CH_3)-CH_2-$, - $CH_2CH_2-O-CH_2CH_2-$, $-CH_2CH_2-NH-CH_2CH_2-$, $-CH_2CH_2-N(CH_3)-CH_2CH_2-$oder<br>$R^a$ Wasserstoff, Methyl oder Ethyl und $R^b$ gemeinsam mit R7 $-(CH_2)_n-$ mit n = 1, 2, 3, 4, wobei einzelne |

oder bis zu 4 Wasserstoffatome der $CH_2$-Gruppen durch Fluoratome ersetzt sein können oder $R^a$ gemeinsam mit R7 $-CH_2-O-CH_2CH_2-$, $-CH_2-N(CH_3)-CH_2CH_2-$ und $R^b$ Wasserstoff, Methyl oder Ethyl oder $-CR^cR^d-CR^eR^f-COOH$ wobei

$R^c$, $R^d$, $R^e$ $R^f$ Wasserstoff oder,

$R^c$, $R^d$ unabhängig voneinander Methyl, Ethyl oder gemeinsam $-(CH_2)_n-$ mit n = 2, 3, 4, 5 oder $-CH_2CH_2-O-CH_2CH_2-$ und $R^e$, $R^f$ Wasserstoff oder

$R^c$, $R^d$ Wasserstoff und $R^e$, $R^f$ unabhängig voneinander Methyl, Ethyl oder gemeinsam $-(CH_2)n-$ mit n = 2, 3, 4, 5, $-CH_2CH_2-O-CH_2CH_2-$, $-CH_2CH_2-NH-CH_2CH_2-$, $-CH_2CH_2-N(CH_3)-CH_2CH_2-$ oder $-CH_2-O-CH_2-$ oder

$R^c$ Methyl, Ethyl, Trifluorethyl und $R^d$, $R^e$ und $R^f$ Wasserstoff oder

$R^c$, $R^d$ und $R^f$ Wasserstoff und $R^e$ Methyl, Ethyl, Trifluormethyl, Hydroxyl oder Methoxy oder

$R^c$ und $R^e$ gemeinsam $-(CH_2)_n-$ mit n = 1,2,3 oder 4 und $R^d$ und $R^f$ Wasserstoff oder

$-CH_2-CH_2-CHR^g-COOH$ wobei

$R^g$ Wasserstoff oder

$R^g$ und R7 gemeinsam $-CH_2-$ oder $-CH_2CH_2-$

bedeuten und deren Salze, Solvate und Solvate der Salze.

Ebenfalls Gegenstand der Erfmdung sind Verbindungen der Formel (II) und der Formel (III)

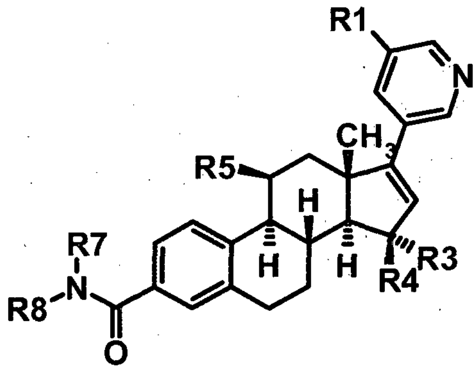

(II)

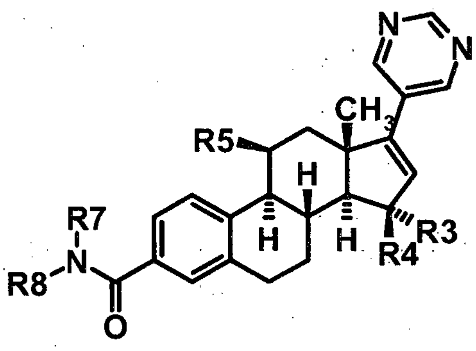

(III)

worin

R1 Wasserstoff, Fluor, Chlor, Nitril, Methoxy, Ethoxy, Trifluormethoxy, Methyl, Ethyl, Trifluormethyl, $-(C=O)CH_3$ und

R3 und R4 Wasserstoff oder
R3 Hydroxyl und R4 Wasserstoff oder
R3 Wasserstoff und R4 Hydroxyl und

R5 Wasserstoff oder Fluor und

R7 Wasserstoff oder $C_1$-$C_4$-Alkyl und

R8 $-CR^aR^b$ -COOH wobei
$R^a$ und $R^b$ unabhängig voneinander Wasserstoff, Methyl oder Ethyl oder
$R^a$ und $R^b$ gemeinsam $-(CH_2)_n-$ mit n = 2, 3, 4 oder 5 oder
$R^a$ Wasserstoff und $R^b$ gemeinsam mit R7 $-(CH_2)_n$ mit n = 3 oder 4 oder
$-CR^cR^d-CR^cR^f$-COOH wobei
$R^c$, $R^d$, $R^e$ $R^f$ Wasserstoff oder
$R^c$, $R^d$ Wasserstoff und $R^e$, $R^f$ unabhängig voneinander Methyl, Ethyl oder gemeinsam $-(CH_2)n-$ mit n = 2, 3, 4, 5 oder $-CH_2CH_2-O-CH_2CH_2-$ oder $R^c$ Methyl oder Ethyl und $R^d$, $R^e$ und $R^f$ Wasserstoff oder
$R^c$ und $R^e$ gemeinsam $-(CH_2)_n-$ mit n = 1, 2, 3 oder 4 und $R^d$ und $R^f$ Wasserstoff oder
$-CH_2-CH_2-CHR^g$-COOH wobei
$R^g$ Wasserstoff oder
$R^g$ und R7 gemeinsam $-CH_2CH_2-$

bedeuten und deren Salze, Solvate und Solvate der Salze.

Auch Gegenstand der Erfindung sind Verbindungen der Formel (II) und der Formel (III) worin

R1 Wasserstoff, Fluor, Chlor, Nitril, Methoxy, Trifluormethyl und

R3 und R4 Wasserstoff oder
R3 Hydroxyl und R4 Wasserstoff oder
R3 Wasserstoff und R4 Hydroxyl und

R5 Wasserstoff oder Fluor und

R7 Wasserstoff, Methyl oder Ethyl und

R8 $-CR^aR^b$-COOH wobei
$R^a$ und $R^b$ unabhängig voneinander Wasserstoff, Methyl oder Ethyl oder
$R^a$ Wasserstoff und $R^b$ gemeinsam mit R7 $-(CH_2)_n-$ mit n = 3 oder 4 oder
$-CR^cR^d-CR^eR^f$-COOH wobei
$R^c$, $R^d$, $R^e$ $R^f$ Wasserstoff oder
$R^c$, $R^d$ Wasserstoff und $R^e$, $R^f$ unabhängig voneinander Methyl oder Ethyl oder gemeinsam $-(CH_2)n-$ mit n = 2, 4, 5 oder $-CH_2CH_2-O-CH_2C$ oder
$R^c$ Methyl und $R^d$, $R^e$ und $R^f$ Wasserstoff oder
$R^c$ und $R^e$ gemeinsam $-(CH_2)_n-$ mit n = 3 oder 4 und $R^d$ und $R^f$ Wasserstoff oder
$-CH_2-CH_2-CHR^9$-COOH wobei
$R^g$ Wasserstoff oder
$R^g$ und R7 gemeinsam$-CH_2CH_2-$

bedeuten und deren Salze, Solvate und Solvate der Salze.

Außerdem sind Gegenstand der Erfindung Verbindungen der Formel (II) und der Formel (III) worin

R1 Wasserstoff, Fluor, Methoxy, Trifluormethyl und

R3 und R4 Wasserstoff oder
R3 Hydroxyl und R4 Wasserstoff oder
R3 Wasserstoff und R4 Hydroxyl und

R5 Wasserstoff oder Fluor und

R7 Wasserstoff oder Methyl und

R8 $-CR^aR^b$ -COOH worin
$R^a$ und $R^b$ unabhängig voneinander Wasserstoff oder Methyl oder

$R^a$ Wasserstoff und $R^b$ gemeinsam mit R7 $-(CH_2)_3-$ oder
$-CR^cR^d-CR^eR^f-COOH$
$R^c$, $R^d$, $R^e$ $R^f$ Wasserstoff, oder
$R^c$ und $R^d$ Wasserstoff und $R^e$ und $R^f$ Methyl oder gemeinsam $-(CH_2)n-$ mit n = 2 oder 4 oder $-CH_2CH_2-O-CH_2CH_2-$ oder
$R^c$ Methyl und $R^d$, $R^e$ und $R^f$ Wasserstoff oder
$R^c$ und $R^e$ gemeinsam $-(CH_2)_3-$ und $R^d$ und $R^f$ Wasserstoff oder
$-CH_2-CH_2-CHR^g-COOH$
$R^g$ Wasserstoff oder
$R^g$ und R7 $-CH_2CH_2-$

bedeuten und deren Salze, Solvate und Solvate der Salze.

Weiterhin sind Gegenstand der Erfindung die Verbindungen

4-[({[17-(5-Fluorpyridin-3-yl)estra-1,3,5(10),16-tetraen-3-yl]carbonyl}amino) methyl]-3,4,5,6-tetrahydro-2*H*-pyran-4-carbonsäure

*N*-{[17-(5-Fluorpyridin-3-yl)estra-1,3,5(10),16-tetraen-3-yl]carbonyl}-*N*-methyl-β-alanin

1-[({[17-(5-Fluorpyridin-3-yl)estra-1,3,5(10),16-tetraen-3-yl]carbonyl}amino) methyl]cyclopropan-1-carbonsäure

1-[({[17-(5-Fluorpyridin-3-yl)estra-1,3,5(10),16-tetraen-3-yl]carbonyl}amino)methyl] cyclopentan-1-carbonsäure

3-({[17-(5-Fluorpyridin-3-yl)estra-1,3,5(10),16-tetraen-3-yl]carbonyl}amino)-2,2-dimethylpropansäure

1-{[17-(5-Fluorpyridin-3-yl)estra-1,3,5(10),16-tetraen-3-yl]carbonyl}piperidin-4-carbonsäure

*N*-{[17-(5-Fluorpyridin-3-yl)estra-1,3,5(10),16-tetraen-3-yl]carbonyl}-2-methylalanin

4-({[17-(5-Fluorpyridin-3-yl)estra-1,3,5(10),16-tetraen-3-yl]carbonyl}amino)butansäure

*N*-{[17-(5-Fluorpyridin-3-yl)estra-1,3,5(10),16-tetraen-3-yl]carbonyl}-β-alanin

*N*-{[17-(5-Fluorpyridin-3-yl)estra-1,3,5(10),16-tetraen-3-yl]carbonyl}glycin

(1*R**,2*S**)-2-({[17-(5-Fluorpyridin-3-yl)estra-1,3,5(10),16-tetraen-3-yl]carbonyl} amino)cyclopentan-1-carbonsäure

(*S*)-3-({[17-(5-Fluorpyridin-3-yl)estra-1,3,5(10),16-tetraen-3-yl]carbonyl}amino) butansäure

(*R*)-3-({[17-(5-Fluorpyridin-3-yl)estra-1,3,5(10),16-tetraen-3-yl]carbonyl}amino) butansäure

3-({[17-(5-Methoxypyridin-3-yl)estra-1,3,5(10),16-tetraen-3-yl]carbonyl} amino)-2,2-dimethylpropansäure

*N*-{[17-(5-Methoxypyridin-3-yl)estra-1,3,5(10),16-tetraen-3-yl]carbonyl}-β-alanin

*N*-{[17-(5-Methoxypyridin-3-yl)estra-1,3,5(10),16-tetraen-3-yl]carbonyl}-*N*-methyl-β-alanin

*N*-{[17-(Pyrimidin-5-yl)estra-1,3,5(10),16-tetraen-3-yl]carbonyl}-β-alanin

4-({[7-(Pyrimidin-5-yl)estra-1,3,5(10),16-tetraen-3-yl]carbonyl}amino)butansäure

*N*-Methyl-*N*-{[17-(pyrimidin-5-yl)estra-1,3,5(10),16-tetraen-3-yl]carbonyl}-β-alanin

2,2-Dimethyl-3-({[17-(pyrimidin-5-yl)estra-1,3,5(10),16-tetraen-3-yl]carbonyl}amino) propansäure

*N*-({17-[5-(Trifluormethyl)pyridin-3-yl]estra-1,3,5(10),16-tetraen-3-yl}carbonyl)-β-alanin

*N*-Methyl-*N*-({17-[5-(trifluormethyl)pyridin-3-yl]estra-1,3,5(10),16-tetraen-3-yl} carbonyl)-β-alanin

*N*-{[17-(5-Fluorpyridin-3-yl)estra-1,3,5(10),16-tetraen-3-yl]carbonyl}-L-prolin

*N*-{[17-(5-Fluorpyridin-3-yl)estra-1,3,5(10),16-tetraen-3-yl]carbonyl}-D-prolin

4-({[11β-Fluor-17-(5-fluorpyridin-3-yl)estra-1,3,5(10),16-tetraen-3-yl]carbonyl} amino)butansäure

*N*-{[17-(5-Fluorpyridin-3-yl)-15α-hydroxyestra-1,3,5(10),16-tetraen-3-yl]carbonyl}-*N*-methyl-β-alanin

*N*-{[17-(5-Fluorpyridin-3-yl)-15β-hydroxyestra-1,3,5(10),16-tetraen-3-yl]carbonyl}-*N*-methyl-β-alanin

N-Methyl-N-{[17-(6-methylpyridazin-4-yl)estra-1,3,5(10),16-tetraen-3-yl]carbonyl}-β-alanin

N-Methyl-N-{[17-(pyridin-3-yl)estra-1,3,5(10),16-tetraen-3-yl]carbonyl}-β-alanin

4-({[17-(5-Methoxypyridin-3-yl)estra-1,3,5(10),16-tetraen-3-yl]carbonyl}amino)butansäure

und deren Salze, Solvate und Solvate der Salze.

Es wurde gefunden, dass die erfindungsgegenständlichen Estra-1,3,5[10],16-tetraen-3-carbonylamino Derivate als AKR1C3 Inhibitoren wirken. Über den Großteil des beanspruchten Strukturbereichs zeigen diese Substanzen eine starke Inhibition von AKR1C3 in vitro ($IC_{50}$-Werte kleiner als 50 nM) und überwiegend sogar $IC_{50}$-Werte < 20 nM. Zusätzlich zeigen diese Derivate keine oder eine nur sehr geringe Inhibition von Cyp17A1.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Diastereomere) existieren. In Verbindungen der Formel (I) können sich Stereozentren im Rest R8 (und falls R7 und R8 gemeinsam einen Zyklus bilden, auch in diesem Zyklus) befinden. Die Erfindung umfasst deshalb die Diastereomeren und ihre jeweiligen Mischungen. Aus solchen Mischungen von Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als <u>Salze</u> sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Essigsäure, Ameisensäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als <u>Solvate</u> werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:

$C_1$-$C_{14}$-Alkyl steht für einen linearen oder verzweigten Alkylrest mit 1 bis 4 Kohlenstoffatomen, beispielhaft und vorzugsweise für Methyl, Ethyl, Propyl, Butyl, Isopropyl, tert-Buyl, Isobutyl.

$C_3$-$C_6$-Cycloalkyl Cycloalkylgruppe mit 3 bis 6 Kohlenstoffatomen, wobei auch der Ring teilweise ungesättigt sein kann, beispielhaft und vorzugsweise für Cyclopropyl, Cyclobutyl, Cyclopentyl und Cyclohexyl.

Weiterer Gegenstand der Erfindung sind Verfahren zur Herstellung der erfindungsgemäßen Verbindungen der Formel (I). Die Herstellung der erfindungsgemäßen Verbindungen (I) kann durch die nachfolgenden Syntheseschema verdeutlicht werden:

Eine Teilmenge der erfindungsgemäßen Verbindungen lässt sich ausgehend vom literaturbekannten Methyl-17-oxoestra-1,3,5(10)-trien-3-carboxylat (Steroids, 1995, 60, 3, 299 - 306) herstellen (Syntheseschema 1):

Die Umsetzung zum Intermediat 1 erfolgt durch die Verwendung von Trifluormethansulfonsäureanhydrid oder *N*,*N*-Bis(trifluoromethanesulfonyl)anilin in Gegenwart einer Base wie Pyridin, 2,6-Dimethylpyridin oder 2,6-Di-*tert*-butylpyridin oder in Gegenwart eines tertiären Amins wie Triethylamin oder Diisopropylethylamin oder durch Verwendung von Alkalimetallhexamethylsilazanen oder Lithium-diisopropylamid (LDA) (J. Med. Chem., 1995, 38, 2463 - 2471, J. Org. Chem., 1985, 50, 1990 - 1992, JACS, 2009 ,131, 9014 - 9019, Archiv der Pharmazie (Weinheim, Germany), 2001, 334, 12, 373 - 374). Bevorzugt ist die Umsetzung mit Trifluormethansulfonsäureanhydrid in Gegenwart von 2,6-Di-*tert*-butylpyridin in Dichlormethan.

Die Herstellung der Intermediate 2 erfolgt mit Hilfe der dem Fachmann bekannten *Suzuki*-Reaktion. Hierfür wird das Intermediat 1 mit einer stickstoffhaltigen aromatischen Boronsäure, einem Boronsäureester wie z.B. einem Boronsäurepinakolester, einem *MIDA* Boronat (D. M. Knapp et al. J. Am. Chem. Soc. 2009, 131, 6961) oder mit einem Trifluorboratsalz (G. A. Molander et al., J. Org. Chem. 2009, 74, 973) umgesetzt. Als Katalysatoren kommen eine Vielzahl an palladiumhaltigen Katalysatoren in Betracht, wie zum Beispiel Tetrakis(triphenylphosphin)palladium(0), Bis-(triphenylphophin)-palladium(II)-dichlorid oder [1,3-Bis(2,6-diisopropylphenyl)imidazol-2-yliden](3-chloropyridyl)palladium(II) dichlorid (CAS 905459-27-0). Alternativ kann eine Palladiumhaltige Quelle wie zum Beispiel Palladium(II)-acetat, Palladium(II)-chlorid oder $Pd(dba)_2$ in Kombination mit einem Phosphorhaltigen Liganden wie zum Beispiel Triphenylphosphin, SPhos (D. M. Knapp et. al., J. Am. Chem. Soc. 2009, 131, 6961) oder RuPhos (G. A. Molander, J. Org. Chem. 2009, 74, 973) eingesetzt werden. Bevorzugt ist die Umsetzung mit Boronsäuren in Gegenwart von Tetrakis(triphenylphosphin)palladium(0) oder [1,3-Bis(2,6-diisopropylphenyl)imidazol-2-yliden](3-chloropyridyl)palladium(II) dichlorid.

Die Herstellung der Intermediate 3 erfolgt durch Verseifung des Methylesters nach dem Fachmann bekannten Methoden. Hierfür wird das Intermediat 2 in einem Lösemittel wie Tetrahydrofuran (THF), Methanol oder Dimethylsulfoxid (DMSO) oder in einer Mischung aus Methanol und THF mit Natronlauge oder einer wässrigen Lithiumhydroxidlösung versetzt. Gegebenenfalls wird erwärmt. Bevorzugt ist die Umsetzung in THF und Methanol in Gegenwart von Natronlauge oder wässriger Lithiumhydroxidlösung bei 40°C.

Die Herstellung der Beispielverbindungen erfolgt in zwei Stufen ausgehend von Intermediaten 3 durch eine Amidkupplung mit einem Ester einer Aminosäure und durch nachfolgende Umwandlung des Carbonsäureesters zur Carbonsäure. Für die Amidkupplung (Stufe A) kommen dem Fachmann bekannte Reagenzien wie zum Beispiel *N*,*N*'-Dicyclohexylcarbodiimid (DCC), *N*-[3-(Dimethylamino)propyl]-*N*'-ethylcarbodiimid Hydrochlorid (EDC) [CAS 25952-53-8] oder HATU (O-(7-Azabenzotriazol-1-yl)-*N*,*N*,*N*',*N*'-tetramethyluronium hexafluorophosphonat in Frage. Zusätzlich können als Additive noch Reagenzien wie 1*H*-Benzotriazol-1-ol Hydrat (HOBt Hydrat [CAS 123333-53-9]) oder 4-Dimethylaminopyridin (DMAP) benutzt werden. Als Basen können zum Beispiel Pyridin, Triethylamin oder Diisopropylethylamin verwendet werden. Bevorzugt ist die Umsetzung mittels EDC, HOBt Hydrat und Triethylamin. Für die Umwandlung des Carbonsäureesters in die Carbonsäure (Stufe B) können - falls es sich zum Beispiel um einen Methyl, Ethyl oder Benzylester handelt - Verseifungsmethoden verwendet werden, wie bei der Herstellung der Intermediate 3 beschrieben. Handelt es sich um einen Carbonsäure-*tert*-butylester, so kann dieser nach dem Fachmann bekannten Methoden zum Carbonsäure umgewandelt werden wie zum Beispiel durch die Reaktion mit Trifluoressigsäure in Dichlormethan oder Chloroform oder durch die Reaktion mit Chlorwasserstoff in 1,4-Dioxan. Die Reaktion mit Trifluoressigsäure in Dichormethan ist bevorzugt.

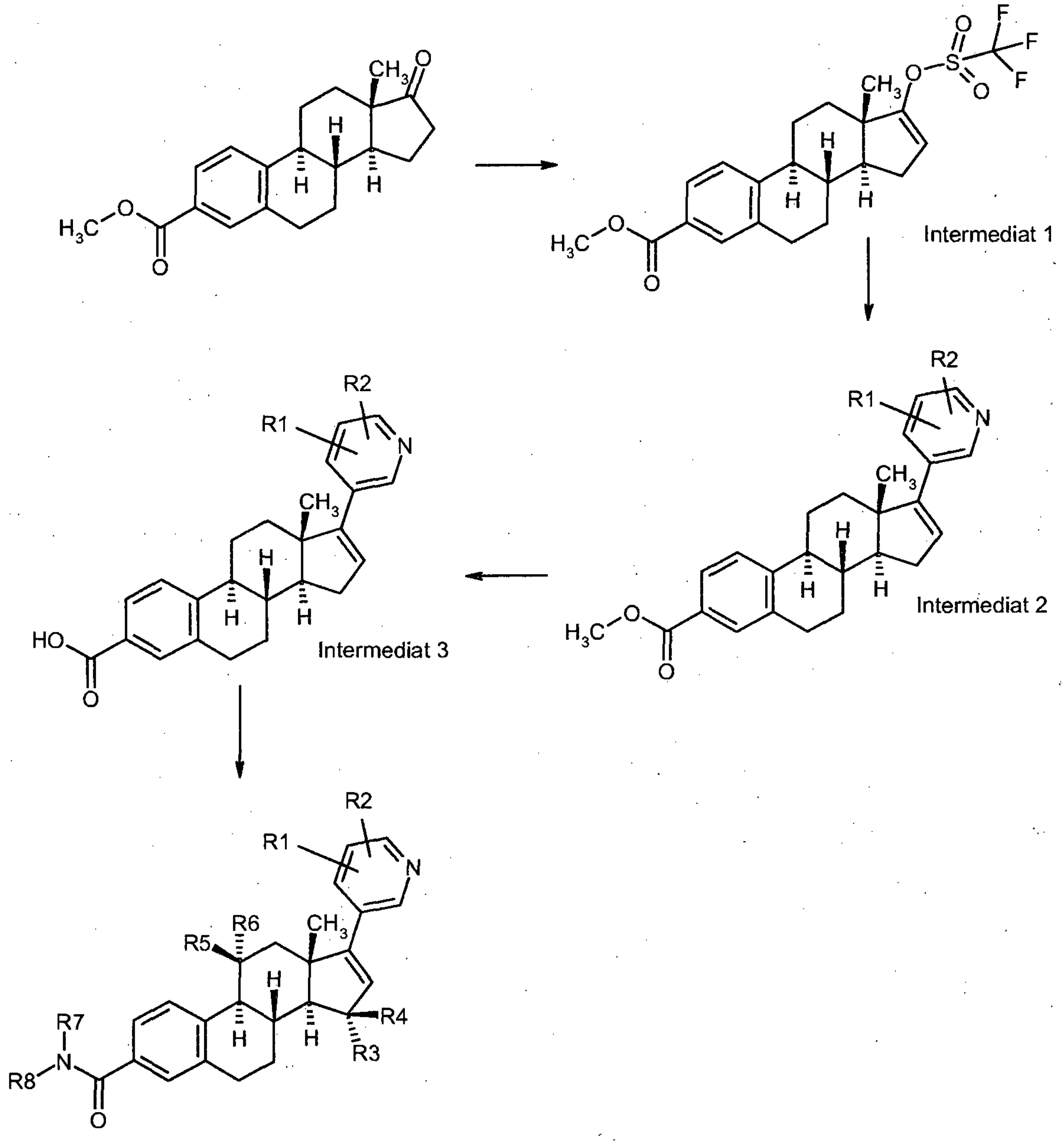


Teilmenge der Beispielverbindungen mit R3, R4, R5, R6 = H

**Syntheseschema 1**

Eine Teilmenge der erfindungsgemäßen Verbindungen der Formel (I) mit R5 = F und R6 = H lässt sich wie in Syntheseschema 2 beschrieben herstellen:

3,11α-Dihydroxyestra-1,3,5(10)-trien-17-on wird mit Essigsäureanhydrid und Pyridin in Gegenwart von 4-Dimethylaminopyridin (DMAP) in Dichlormethan zu Intermediat 4 umgesetzt. Die Umwandlung in das Intermediat 5 erfolgt mit Natriumhydrogencarbonat in Methanol. Die Umsetzung von Intermediat 5 mit 1,1,2,2,3,3,4,4,4-Nonafluorbutan-1-sulfonylfluorid und Kaliumcarbonat führt zu Intermediat 6, welches mit Palladium(II)acetat, 1,3-Bis(diphenylphoshino)propan, Triethylamin in Methanol und DMSO in einem Autoklaven in einer Kohlenmonoxidatmosphäre zu Intermediat 7 umgewandelt wird. Die Umsetzung zu Intermediat 8 erfolgt mit Methoden wie bei der Herstellung von Intermediat 1 beschrieben. Die Transformation von Intermediat 8 zu Intermediat 9 wird mit Methoden, wie bei der Herstellung von Intermediat 2 beschrieben, durchgeführt. Intermediat 9 wird mit Kaliumcarbonat in Methanol zu Intermediat 10 verseift. Die Transformation zum Intermediate 11 wird mit 1,8-Diazabicyclo[5.4.0]undec-7-en und 1,1,2,2,3,3,4,4,4-Nonafluorbutan-1-sulfonylfluorid in THF durchgeführt. Die Verseifung von Intermediat 11 zu Intermediat 12 erfolgt mit Bedingungen wie bei der Herstellung von Intermediat 3 beschrieben. Bevorzugt ist die Umsetzung in THF und Methanol in Gegenwart von wässriger Lithiumhydroxidlösung. Die Herstellung einer Teilmenge der Beispielverbindungen ausgehend von Intermediat 12 erfolgt analog zur Herstellung der Beispielverbindungen ausgehend von Intermediat 3 wie in Syntheseschema 1 beschrieben.

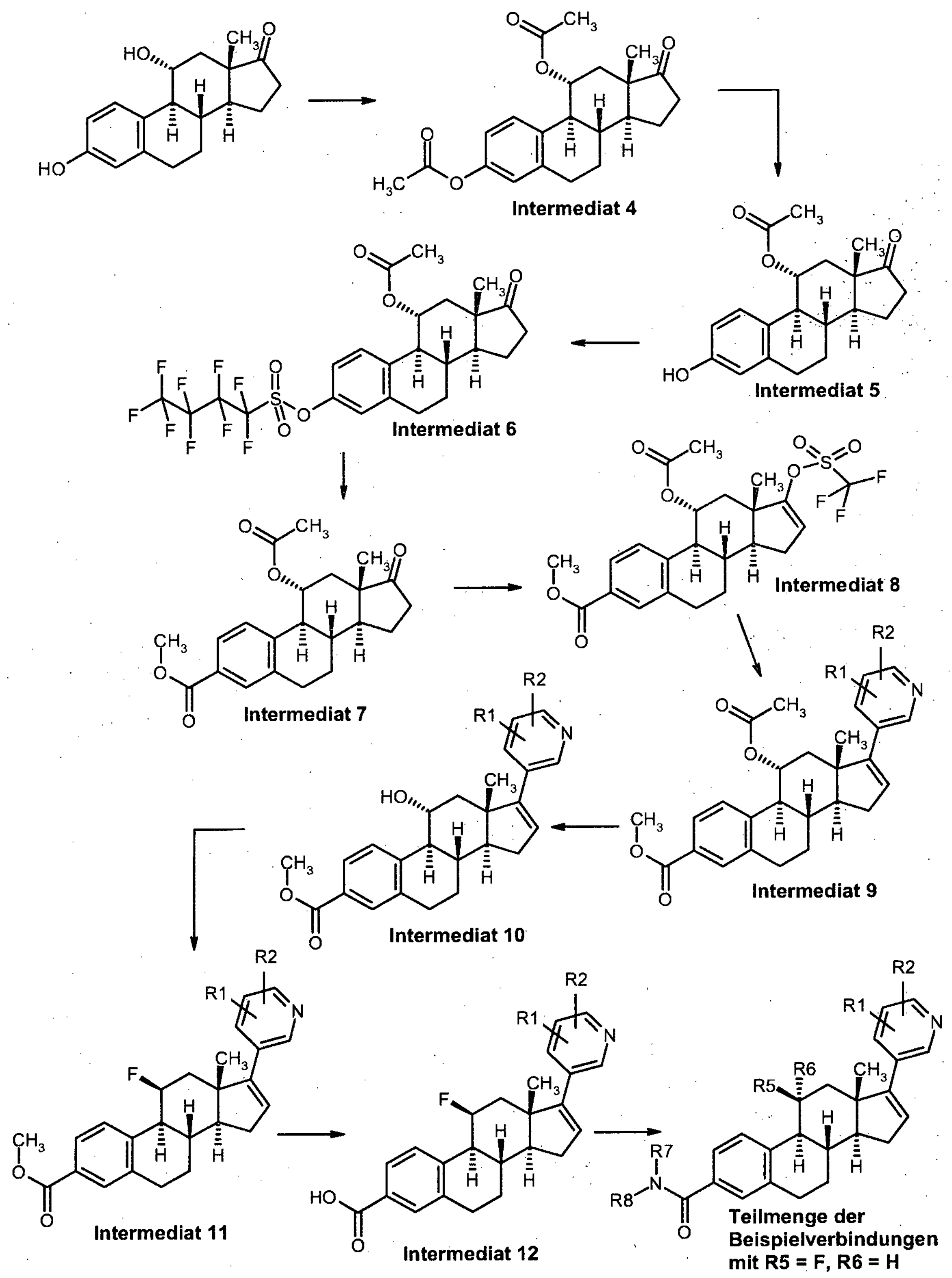
Intermediat 4
Intermediat 5
Intermediat 6
Intermediat 7
Intermediat 8
Intermediat 9
Intermediat 10
Intermediat 11
Intermediat 12
Teilmenge der Beispielverbindungen mit R5 = F, R6 = H

**Syntheseschema 2**

Eine Teilmenge der erfindungsgemäßen Verbindungen der Formel (I) mit der Substituentendefinition R3 = OH und R4 = H oder R3 = H und R4 = OH lässt sich wie in Syntheseschema 3 verdeutlicht herstellen. Die Umsetzung erfolgt mit Mikroorganismen, z.B. bestimmten geeigneten Pilz-Stämmen, die eine regio- und stereoselektive Hydroxylierung ermöglichen. Beispielsweise ist es auf diese Weise möglich, regio- und stereoselektiv Hydroxylgruppen in die 15-Position des Steroidgerüstes einzuführen. Die so erhaltenen 15-OH Derivate sind Beispielverbindungen im Sinne der Erfindung und können ferner auch in chemischen Folgeumsetzungen weiter modifiziert werden.

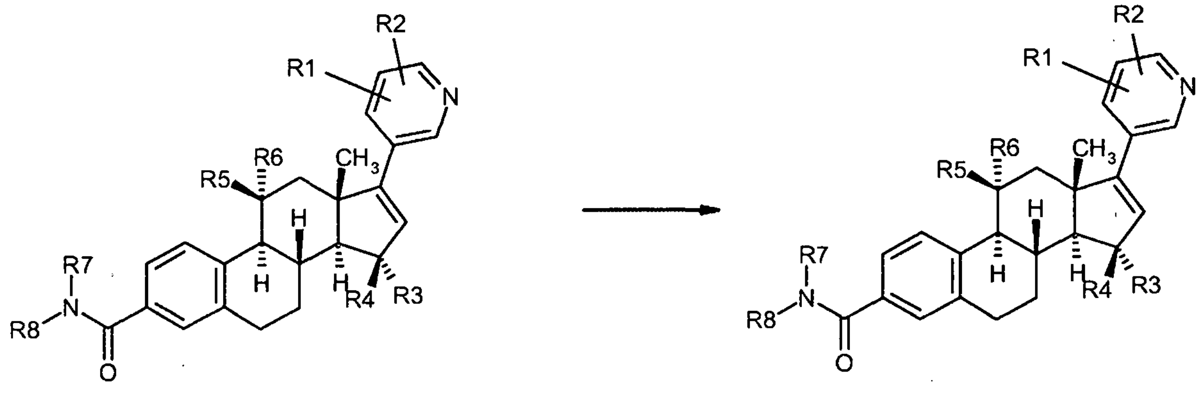

Teilmenge der Beispielverbindungen der Formel (I) mit R3 und R4 = H

Teilmenge der Beispielverbindungen der Formel (I) mit R3 = OH und R4 = H, oder R3 = H und R4 = OH

**Syntheseschema 3**

Die erfindungsgemäßen Verbindungen zeigen in nicht vorhersehbarer Weise ein wertvolles pharmakologisches Wirkspektrum und vorteilhafte pharmakokinetische Eigenschaften. Sie eignen sich daher zur Verwendung als Arzneimittel zur Behandlung und/oder Prophylaxe von Krankheiten bei Menschen und Tieren. Der Begriff "Behandlung" im Rahmen der vorliegenden Erfindung schließt die Prophylaxe ein. Die pharmazeutische Wirksamkeit der erfindungsgemäßen Verbindungen lässt sich durch ihre Wirkung als AKR1C3 Inhibitor erklären. Die erfindungsgemäßen Verbindungen sind daher besonders zur Behandlung und/oder Prophylaxe der Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes geeignet.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Verwendung in einem Verfahren zur Behandlung und/oder Prophylaxe der zuvor genannten Erkrankungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt: selektive Estrogen Rezeptor Modulatoren (SERMs), Estrogenrezeptor (ER) Antagonisten, Aromatasesinhibitoren, 17 β-HSD1 Inhibitoren, Steroid Sulfatase (STS)-Inhibitoren, GnRH-Agonisten und -Antagonisten, Kisspeptin Rezeptor (KISSR)-Antagonisten, selektive Androgen Rezeptor Modulatoren (SARMs), Androgene, 5α-Reduktase Inhibitoren, selektive Progesteron Rezeptor Modulatoren (SPRMs), Gestagene, Antigestagene, orale Kontrazeptiva, Inhibitoren der Mitogen Aktivierten Protein (MAP) Kinasen sowie Inhibitoren der MAP Kinasen Kinasen (Mkk3/6, Mekl/2, Erk1/2), Inhibitoren der Proteinkinasen B (PKBα/β/γ; Akt1/2/3), Inhibitoren der Phosphoinositid-3-Kinasen (PI3K), Inhibitoren der Cyclinabhängigen Kinase (CDK1/2), Inhibitoren des Hypoxie Induzierten Signalweges (HIF1alpha Inhibitoren,

Aktivatoren der Prolylhydroxylasen), Histon Deacetylase (HDAC)-Inhibitoren, Prostaglandin F Rezeptor (FP) (PTGFR)-Antagonisten, und Nicht-steroidale Enzündungshemmer (NSAIDs).

Die Erfindung betrifft auch pharmazeutische Präparate, die mindestens eine Verbindung der allgemeinen Formel I (oder physiologisch verträgliche Additionssalze mit organischen und anorganischen Säuren davon) enthalten und die Verwendung dieser Verbindungen zur Herstellung von Arzneimitteln, insbesondere für die zuvor genannten Indikationen.

Die Verbindungen können, sowohl nach oraler als auch parenteraler Gabe, für die zuvor genannten Indikationen eingesetzt werden.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granula Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate, intrauterine Spiralen, Vaginalringe oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Bei oraler Applikation beträgt die Menge pro Tag etwa 0,01 bis 100 mg/kg Körpergewicht. Die zu verabreichende Menge einer Verbindung der allgemeinen Formel I schwankt innerhalb eines weiten Bereichs und kann jede wirksame Menge abdecken. In Abhängigkeit des zu behandelnden Zustands und der Art der Verabreichung kann die Menge der verabreichten Verbindung 0,01 - 100 mg / kg Körpergewicht je Tag betragen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

## Abkürzungsverzeichnis Chemie

Abkürzungen und Akronyme:)

| | |
|---|---|
| DMAP | 4-Dimethylaminopyridin |
| DMF | Dimethylformamid |
| DMSO | Dimethylsulfoxid |
| d. Th. | der Theorie (bei Ausbeute) |
| h | Stunde(n) |
| HPLC | Hochdruck-, Hochleistungsflüssigkeitschromatographie |
| LC-MS | Flüssigkeitschromatographie-gekoppelte Massenspektroskopie |
| ES-MS | Elektrospray Massenspektroskopie |
| min | Minute(n) |
| MS | Massenspektroskopie |
| NMR | Kernresonanzspektroskopie |
| RT | Raumtemperatur |
| TFA | Trifluoressigsäure |
| THF | Tetrahydrofuran |

## Reinigung der erfindungsgemäßen Verbindungen

In einigen Fällen konnten die erfindungsgemäßen Verbindungen durch präparative HPLC zum Beispiel durch ein Autopurifier-Gerät der Firma Waters (Detektion der Verbindungen durch UV-Detektion sowie Elektrospray-Ionisation) in Kombination mit kommerziell erhältlichen, vorgepackten HPLC Säulen (zum Beispiel Säule XBridge (Firma Waters), C18, 5$\mu$m, 30 x 100mm) gereinigt werden. Als Lösemittelsystem wurde Acetonitril / Wasser mit Zusatz von Ameisensäure verwendet. Weitere, dem Fachmann bekannte Zusätze wie zum Beispiel Ammoniak, Ammoniumacetat oder Trifluoressigsäure können verwendet werden. Statt Acetonitril kann beispielsweise auch Methanol verwendet werden.

In einigen Fällen wurde folgende Methode für die präparative HPLC Trennung verwendet:

| | |
|---|---|
| *System:* | Waters Autopurificationsystem: Pump 2545, Sample Manager 2767, CFO, DAD 2996, ELSD 2424, SQD |
| *Säule:* | XBrigde C18 5$\mu$m 100x30 mm |
| *Lösemittel:* | A = $H_2O$ + 0.1% Vol. Ameisensäure (99%) |
| | B = Acetonitril |
| *Gradient:* | 0-1 min 1% B, 1-8 min 1-99% B, 8-10 min 99% B |
| *Fluss:* | 50 ml/min |
| *Temperatur:* | RT |
| *Injektion:* | 1 x 2.5 ml |
| *Detektion:* | DAD scan range 210-400 mm |
| | MS ESI+, ESI-, scan range 160-1000 m/z |

Zum Entfernen des HPLC-Lösemittelgemisches wurde eine Gefriertrocknung oder eine Vakuumzentrifugation verwendet. Liegen die so erhaltenen Verbindungen als TFA-Salze bzw. Formiatsalze vor, so können diese in die jeweiligen freien Basen mittels dem Fachmann bekannten Standard-Laborprozeduren überführt werden.

In einigen Fällen konnten die erfindungsgemäßen Verbindungen durch Chromatographie an Kieselgel gereinigt werden. Hierbei wurden zum Beispiel vorgepackte Silica Gel Cartridges (zum Beispiel von der Firma Separtis, *Isolute® Flash silica gel*) in Kombination mit dem Flashmaster II Chromatograhiegerät (ArgonautIBiotage) und Chromatographielösemittel bzw. -gemische wie zum Beispiel Hexan, Ethylacetat sowie Dichlormethan und Methanol in Betracht.

**Strukturanalytik der erfindungsgemäßen Verbindungen:**

In einigen Fällen wurden die erfindungsgemäßen Verbindungen durch LC-MS analysiert:

In einigen Fällen wurde folgende Analytikmethode verwandt:

Instrument: Waters Acquity UPLC-MS SQD; Säule: Acquity UPLC BEH C18 1.7 50x2.1mm; Eluent A: Wasser + 0.1% Vol. Ameisensäure (99%), Eluent B: Acetonitril; Gradient: 0-1.6 min 1-99% B, 1.6-2.0 min 99% B; Fluss 0.8 ml/min; Temperatur: 60 °C; Injektion: 2 μl; DAD scan: 210-400 nm

Bei den NMR-Daten der erfindungsgemäßen Verbindungen gelten folgende Bedeutungen:

| | |
|---|---|
| s | Singulett |
| d | Dublett |
| t | Triplett |
| q | Quartett |
| quin | Quintett |
| m | Multiplett |
| br | breit |
| mc | Zentriertes Multiplett |

**Synthese der erfindungsgemäßen Verbindungen:**

**Intermediat 1**

**Methyl-17-{[(trifluormethyl)sulfonyl]oxy}estra-1,3,5(10),16-tetraen-3-carboxylat**

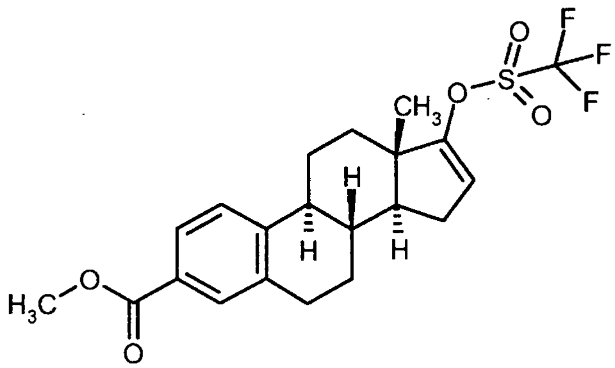

Man tropfte zu einer Mischung aus 5.00 g (16.0 mmol) Methyl-17-oxoestra-1,3,5(10)-trien-3-carboxylat (Steroids, 1995, 60, 3, 299 - 306) in 100 ml Dichlormethan und 5.3 ml 2,6-Di-*tert*-butylpyridin 3.2 ml Trifluormethausulfonsäureanhydrid und rührte 20 h bei RT. Man goss vorsichtig auf 250 ml gesättigte wässrige Natriumhydrogencarbonatlösung, rührte 40 min und trennte die Phasen, extrahierte zweimal mit Dichlormethan und wusch die vereinigten organischen Phasen mit gesättigter Natriumhydrogencarbonatlösung und Natriumchloridlösung, trocknete über Natriumsulfat und engte ein. Nach Ausrühren mit Hexan erhielt man 4.55 g der Titelverbindung als Feststoff.
$^1$H-NMR (300MHz, CHLOROFORM-d): δ [ppm]= 1.01 (s, 3H), 1.37 - 1.74 (m, 5H), 1.81 (td, 1H), 1.88 - 2.02 (m, 2H),

2.05 - 2.19 (m, 1H), 2.27 - 2.55 (m, 3H), 2.83 - 3.11 (m, 2H), 3.90 (s, 3H), 5.63 (dd, 1H), 7.32 (d, 1H), 7.68 - 7.90 (m, 2H).

**Intermediat 2-a**

**Methyl-17-(5-fluorpyridin-3-yl)estra-1,3,5(10),16-tetraen-3-carboxylat**

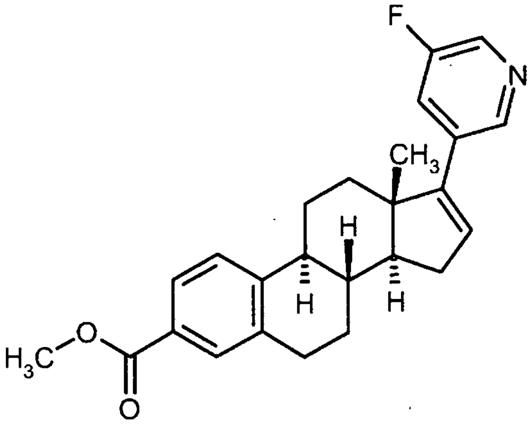

8.00 g (2.25 mmol) Methyl-17-{[(trifluormethyl)sulfonyl]oxy}estra-1,3,5(10),16-tetraen-3-carboxylat und 3.55 g (1.4 Äquiv.) 5-Fluorpyridin-3-boronsäure wurden in 60 ml Toluol und 40 ml Ethanol vorgelegt. Dann wurden 1.53 g (2.0 Äquiv.) Lithiumchlorid, 24 ml 2M wässrige Natriumcarbonatlösung und 1.04 g (5 mol%) Tetrakis(triphenylphosphin)-palladium(0) zugegeben und man erwärmte 3.5 h auf 100°C. Man addierte Wasser, extrahierte dreimal mit Ethylacetat, wusch mit gesättigter Natriumhydrogencarbonatlösung, Kochsalzlösung und engte ein. Nach säulenchromatographischer Reinigung an Kieselgel (Hexan/Ethylacetat) erhielt man 5.5 g (78% d. Th.) der Titelverbindung.
$^1$H-NMR (400MHz, CHLOROFORM-d): δ [ppm]= 1.06 (s, 3H), 1.47 - 1.63 (m, 1H), 1.63 - 1.78 (m, 3H), 1.84 (td, 1H), 1.98 - 2.06 (m, 1H), 2.13 - 2.26 (m, 2H), 2.35 - 2.51 (m, 3H), 2.98 (dd, 2H), 3.90 (s, 3H), 6.10 (dd, 1H), 7.32 - 7.44 (m, 2H), 7.76 - 7.86 (m, 2H), 8.36 (br. s., 1H), 8.48 (s, 1H).

**Intermediat 2-b**

**Methyl-17-(5-methoxypyridin-3-yl)estra-1,3,5(10),16-tetraen-3-carboxylat**

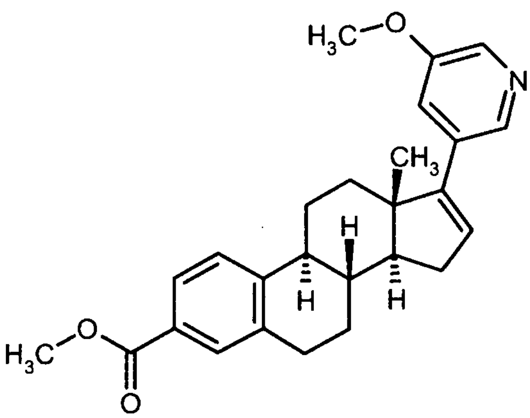

Analog zur Herstellung von Intermediat 2-a wurden 2.00 g (4.50 mmol) Intermediat 1 mit 0.96 g (1.4 Äquiv.) (5-Methoxypyridin-3-yl)boronsäure in Gegenwart von 260 mg Tetrakis(triphenylphosphin)palladium(0) bei 100°C über Nacht zu 1.4 g (76% d. Th.) der Titelverbindung umgesetzt.
$^1$H-NMR (300MHz, CHLOROFORM-d): δ [ppm]= 1.05 (s, 3H), 1.43 - 1.60 (m, 1H), 1.62 - 1.89 (m, 4H), 1.95 - 2.08 (m,

1H), 2.10 - 2.25 (m, 2H), 2.30 - 2.53 (m, 3H), 2.98 (dd, 2H), 3.88 (s, 3H), 3.90 (s, 3H), 6.00 - 6.08 (m, 1H), 7.16 - 7.22 (m, 1H), 7.35 (d, 1H), 7.75 - 7.83 (m, 2H), 8.20 (d, 1H), 8.28 (d, 1H).

**Intermediat 2-c**

**Methyl-17-(pyrimidin-5-yl)estra-1,3,5(10),16-tetraen-3-carboxylat**

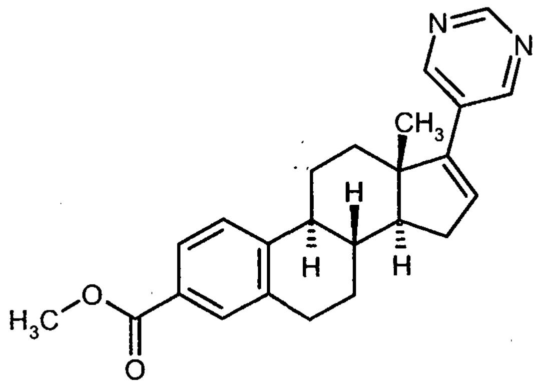

Analog zur Herstellung von Intermediat 2-a wurden 3.00 g (6.75 mmol) Intermediat 1 mit 1.17 g (1.4 Äquiv.) Pyrimidin-5-ylboronsäure in Gegenwart von 390 mg Tetrakis-(triphenylphosphin)palladium(0) bei 100°C über Nacht zu 1.70 g (64% d. Th.) der Titelverbindung umgesetzt.
$^1$H-NMR (400MHz, CHLOROFORM-d): δ [ppm]= 1.06 (s, 3H), 1.47 - 1.59 (m, 1H), 1.65 - 1.80 (m, 3H), 1.85 (td, 1H), 1.98 - 2.06 (m, 1H), 2.12 - 2.25 (m, 2H), 2.36 - 2.53 (m, 3H), 2.98 (dd, 2H), 3.90 (s, 3H), 6.14 (dd, 1H), 7.35 (d, 1H), 7.76 - 7.85 (m, 2H), 8.76 (s, 2H), 9.09 (s, 1 H).

**Intermediat 2-d**

**Methyl-17-[5-(trifluormethyl)pyridin-3-yl]estra-1,3,5(10),16-tetraen-3-carboxylat**

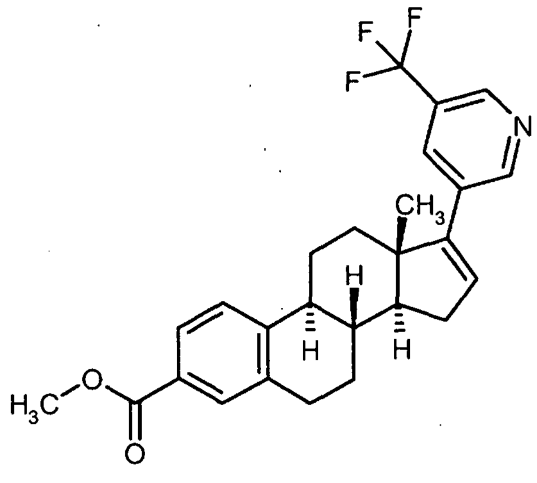

Analog zur Herstellung von Intermediat 2-a wurden 1.66 g (3.74 mmol) Intermediat 1 mit 1.00 g (1.4 Äquiv.) [5-(Trifluormethyl)pyridin-3-yl]boronsäure in Gegenwart von 216 mg Tetrakis(triphenylphosphin)palladium(0) bei 100°C über Nacht zu 1.20 g (73% d. Th.) der Titelverbindung umgesetzt.
$^1$H-NMR (300MHz, CHLOROFORM-d): δ [ppm]= 1.08 (s, 3H), 1.49 - 1.89 (m, 6H), 1.97 - 2.09 (m, 1H), 2.09 - 2.28 (m, 2H), 2.35 - 2.54 (m, 3H), 2.98 (dd, 2H), 3.90 (s, 3H), 6.15 (dd, 1H), 7.36 (s, 1H), 7.77 - 7.85 (m, 2H), 7.88 (s, 1H), 8.83 (s, 2H).

**Intermediat 2-e**

**Methyl-17-(6-methylpyridazin-4-yl)estra-1,3,5(10),16-tetraen-3-carboxylat**

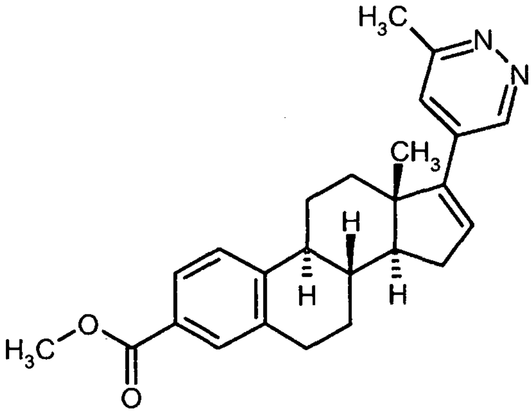

Analog zur Herstellung von Intermediat 2-a wurden 180 mg (3.74 mmol) Intermediat 1 mit 125 mg (1.4 Äquiv.) 3-Methyl-5-(4,4,5,5-tetramethyl-1,3,2-dioxaborolan-2-yl)pyridazin in Gegenwart von 14 mg Bis(triphenylphosphin)palladium(II)chlorid bei 100°C umgesetzt. Man erhielt nach wässriger Aufarbeitung wie bei der Herstellung von Intermediat 2-a beschrieben 201 mg eines Rohproduktes, welches ohne weitere Reinigung zur Herstellung von Intermediat 3-e eingesetzt wurde.

**Intermediat** 2-f

**Methyl-17-(pyridin-3-yl)estra-1,3,5(10),16-tetraen-3-carboxylat**

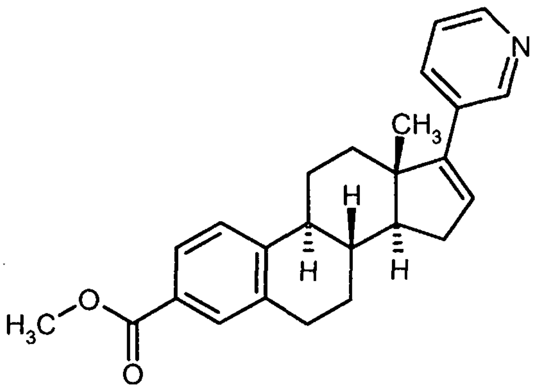

Analog zur Herstellung von Intermediat 2-a wurden 500 mg (1.13 mmol) Intermediat 1 mit 194 mg (1.4 Äquiv.) Pyridin-3-ylboronsäure in Gegenwart von 39 mg Bis(triphenylphosphin)palladium(II)chlorid bei 100°C innerhalb von 18 h umgesetzt. Man erhielt nach wässriger Aufarbeitung 462 mg eines Rohproduktes, welches ohne weitere Reinigung zur Herstellung von Intermediat 3-f eingesetzt wurde.

**Intermediat 3-a**

**17-(5-Fluorpyridin-3-yl)estra-1,3,5(10),16-tetraen-3-carbonsäure**

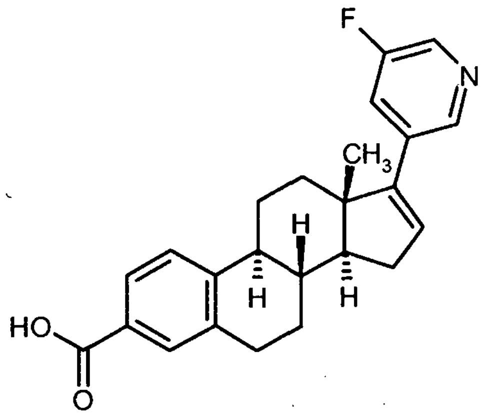

372 mg (0.95 mmol) Intermediat 2-a wurden in 50 ml THF und 3 ml Methanol vorgelegt. Man addierte eine Lösung aus 120 mg Lithiumhydroxid in 3 ml Wasser und ließ 18 h bei RT rühren. Man addierte erneut 5 Äquiv. Lithiumhydroxid, rührte 24 h bei RT und 18 h 40°C. Man verdünnte mit Wasser, säuerte mit 10%iger wässriger Zitronensäurelösung auf pH 4 an, setzte Ethylacetat zu, filtrierte Feststoff ab und erhielt nach Waschen des Feststoffes mit Ethylacetat und Wasser und Trocknen 153 mg (43% d. Th.) der Titelverbindung. Die organische Phase des Filtrats wurde abgetrennt und die wässrige Phase zweimal mit Ethylacetat extrahiert. Nach Waschen der vereinigten organischen Phasen mit Natriumchloridlösung, Trocknen über Natriumsulfat und Einengen erhielt man einen Rückstand, der aus Diethylether ausgerührt wurde. Nach Trocknen erhielt man weitere 143 mg (40% d. Th.) der Titelverbindung. $^1$H-NMR (300MHz, DMSO-$d_6$): δ [ppm]= 0.99 (s, 3H), 1.38 - 1.78 (m, 5H), 1.83 - 1.97 (m, 1H), 2.05 - 2.21 (m, 2H), 2.25 - 2.43 (m, 3H), 2.89 (dd, 2H), 6.27 (dd, 1H), 7.36 (d, 1H), 7.58 - 7.72 (m, 3H), 8.43 (d, 1H), 8.49 (t, 1H).

**Intermediat 3-b**

**17-(5-Methoxy pyridin-3-yl)estra-1,3,5(10),16-tetraen-3-carbonsäure**

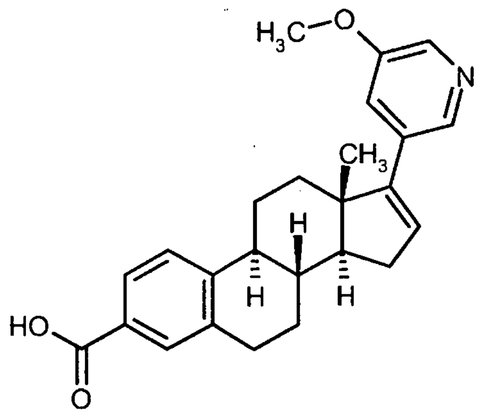

Eine Lösung aus 1.4 g (3.47 mmol) Methyl-17-(5-methoxypyridin-3-yl)estra-l,3,5(10),16-tetraen-3-carboxylat in 30 ml THF, 4 ml Methanol und 8.7 ml 2M Natronlauge wurde über Nacht bei RT gerührt und dann für 8.5 h auf 40°C erwärmt. Man verdünnte mit Wasser, säuerte mit 10%iger Zitronensäurelösung auf pH = 4 an, extrahierte dreimal mit Ethylacetat, wusch mit Kochsalzlösung und engte ein. Nach Ausrühren des Rohproduktes mit Ether erhielt man 1.2 g (89% d. Th.) der Titelverbindung.

$^1$H-NMR (300MHz, DMSO-$d_6$): δ [ppm]= 0.98 (s, 3H), 1.34 - 1.81 (m, 5H), 1.84 - 1.97 (m, 1H), 2.03 - 2.19 (m, 2H), 2.21 - 2.43 (m, 3H), 2.89 (dd, 2H), 3.81 (s, 3H), 6.12 - 6.20 (m, 1H), 7.20 - 7.29 (m, 1H), 7.36 (d, 1H), 7.59 - 7.70 (m, 2H), 8.15 (d, 1H), 8.20 (d, 1H).

**Intermediat 3-c**

**17-(Pyrimidin-5-yl)estra-1,3,5(10),16-tetraen-3-carbonsäure**

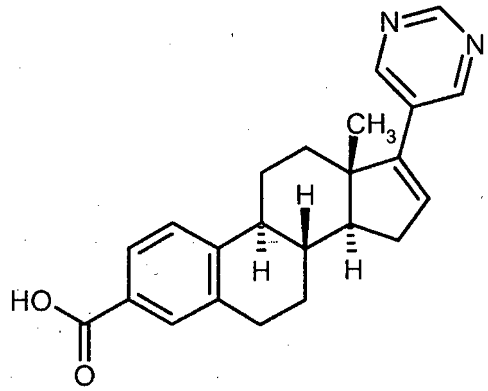

Eine Mischung aus 1.70 g (4.54 mmol) Methyl-17-(pyrimidin-5-yl)estra-1,3,5(10),16-tetraen-3-carboxylat, 40 ml THF, 11.3 ml 2M Natronlauge und 5 ml Methanol wurde bei RT über Nacht, dann 8.5 h bei 40°C 8.5 h und dann über Nacht bei RT gerührt. Man verdünnte mit Wasser, säuerte mit 10%iger Zitronensäurelösung auf pH = 4 an und gab Ethylacetat zu. Der unlösliche Feststoff wurde abfiltriert und getrocknet. Man erhielt 1.3 g (79% d. Th.) der Titelverbindung.
$^1$H-NMR (300MHz, DMSO-$d_6$): δ [ppm]= 0.99 (s, 3H), 1.39 - 1.79 (m, 5H), 1.84 - 1.97 (m, 1H), 2.06 - 2.21 (m, 2H), 2.26 - 2.44 (m, 3H), 2.89 (dd, 2H), 6.28 - 6.33 (m, 1H), 7.36 (d, 1 H), 7.59 - 7.69 (m, 2H), 8.83 (s, 2H), 9.04 (s, 1 H), 12.7 (br. s., 1 H).

**Intermediat 3-d**

**17-[5-(Trifluormethyl)pyridin-3-yl]estra-1,3,5(10),16-tetraen-3-carbonsäure**

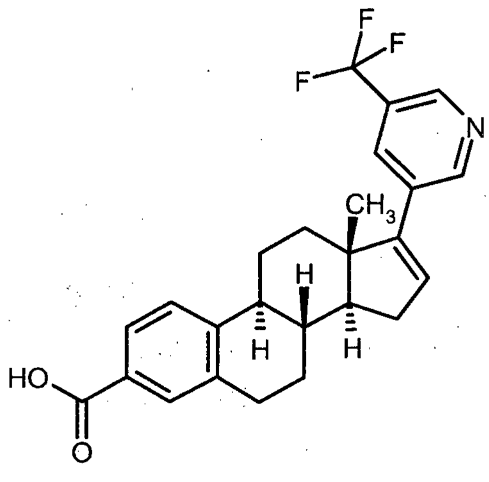

1.2 g Methyl-17-[5-(trifluoroethyl)pyridin-3-yl]estra-1,3,5(10),16-tetraen-3-carboxylat wurden in 12 ml THF vorgelegt, und man addierte eine Lösung aus 0.23 g Lithiumhydroxid in 12 ml Wasser und ließ bei 40°C über Nacht rühren. Man verdünnte mit Wasser, säuerte mit 10%iger Zitronensäurelösung auf pH = 4 an und extrahierte dreimal mit Ethylacetat. Man wusch mit Kochsalzlösung, engte ein und rührte mit Diethylether aus. Man erhielt 850 mg der Titelverbindung als Feststoff.
$^1$H-NMR (400MHz, DMSO-$d_6$): δ [ppm]= 1.01 (s, 3H), 1.37 - 1.50 (m, 1H), 1.50 - 1.69 (m, 3H), 1.76 (td, 1H), 1.86 - 1.95 (m, 1H), 2.08 - 2.19 (m, 2H), 2.27 - 2.44 (m, 3H), 2.90 (dd, 2H), 6.36 (dd, 1H), 7.36 (d, 1H), 7.61 - 7.68 (m, 2H), 8.04 (s, 1H), 8.82 - 8.86 (m, 1H), 8.90 (d, 1 H), 12.7 (br. s., 1 H).

**Intermediat 3-e**

**17-(6-Methylpyridazin-4-yl)estra-1,3,5(1.0),16-tetraen-3-carbonsäure**

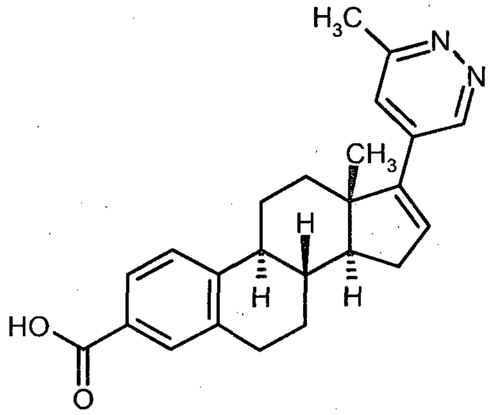

201 mg Methyl-17-(6-methylpyridazin-4-yl)estra-1,3,5(10), 16-tetraen-3-carboxylat (Rohprodukt) wurden in 3 ml THF und 0.5 ml Methanol vorgelegt, man addierte 1.3 ml einer 2M Natronlaugelösung und ließ bei 40°C über Nacht rühren. Man verdünnte mit Wasser, säuerte mit 10%iger Zitronensäurelösung auf pH = 4 an, extrahierte dreimal mit Ethylacetat und engte ein. Nach Reinigung des Rückstandes durch präparative HPLC erhielt man 42 mg der Titelverbindung als Rohprodukt.
$C_{24}H_{26}N_2O_2$ (374.5). MS-ES+ gefundene Masse: 374.20.

**Intermediat 3-f**

**17-(Pyridin-3-yl)estra-1,3,5(10),16-tetraen-3-carbonsäure**

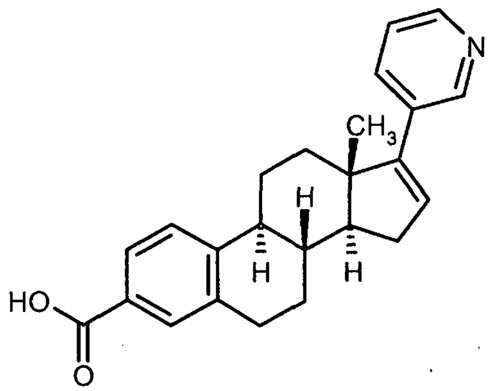

462 mg Methyl-17-(pyridin-3-yl)estra-1,3,5(10),16-tetraen-3-carboxylat (Rohprodukt) wurden in 4 ml THF und 1 ml Methanol gelöst, man addierte 3 ml einer 2M Natronlaugelösung und ließ bei 40°C über Nacht rühren. Man verdünnte mit Wasser, säuerte mit 10%iger Zitronensäurelösung auf pH = 4 an und addierte Ethylacetat. Der verbleibende unlösliche Feststoff wurde abfiltriert, mit Wasser und Ethylacetat gewaschen und im Vakuum getrocknet. Man erhielt 375 mg (84% d. Th.) der Titelverbindung.
$C_{24}H_{25}NO_2$ (359.47). MS-ES+ gefundene Masse: 359.00.
$^1$H-NMR(300MHz, DMSO-$d_6$): δ [ppm]= 0.99 (s, 3H), 1.35 - 1.78 (m, 6H), 1.84 - 1.96 (m, 1H), 2.03 - 2.18 (m, 2H), 2.21 - 2.44 (m, 4H), 2.89 (dd, 2H), 6.10 - 6.14 (m, 1H), 7.29 - 7.39 (m, 2H), 7.57 - 7.68 (m, 2H), 7.77 (dt, 1H), 8.42 (dd, 1H), 8.59 (d, 1H).

**Intermediat 4**

**17-Oxoestra-1,3,5(10)-trien-3,11α-diyldiacetat**

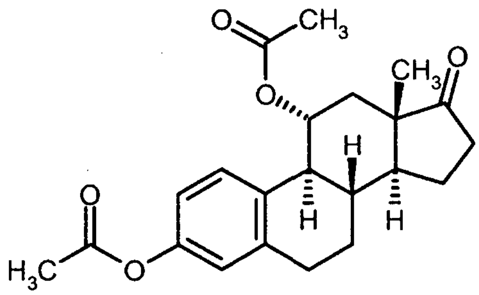

Zu einer Lösung aus 10.0 g (34.9 mmol) 3,11α-Dihydroxyestra-1,3,5(10)-trien-17-on in 100 ml Dichlormethan tropfte man bei RT 13.2 ml (4.0 Äquiv.) Essigsäureanhydrid zu und kühlte das Reaktionsgemisch auf 5°C ab. Danach tropfte man 14.1 ml Pyridin zu und ließ nach 10 min auf RT kommen und rührte 4 h. Man addierte eine Spatelspitze DMAP und ließ 72 h bei RT rühren. Man goss auf 500 ml Wasser, trennte die Phasen, extrahierte mit Dichlormethan, wusch mit 1M Salzsäure, Wasser, Natriumchloridlösung, trocknete über Natriumsulfat und engte ein. Man erhielt 12.9 g (99% d. Th.) eines weißen Feststoffes.
$^1$H-NMR (400MHz, DMSO-$d_6$): δ [ppm]= 0.81 (s, 3H), 1.29 (t, 1H), 1.43 - 1.72 (m, 4H), 1.79 - 2.00 (m, 2H), 2.00 - 2.06 (m, 3H), 2.06 - 2.19 (m, 2H), 2.19 - 2.25 (m, 3H), 2.42 - 2.57 (m, überlagert durch DMSO Signal), 2.76 (t, 2H), 5.26 (td, 1H), 6.82 - 6.89 (m, 2H), 6.97 (d, 1H).

**Intermediat 5**

**3-Hydroxy-17-oxoestra-1,3,5(10)-trien-11α-yl-acetat**

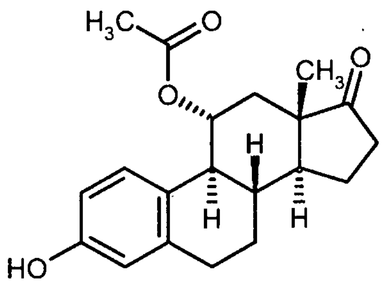

12.9 g (34.7 mmol) 17-Oxoestra-1,3,5(10)-trien-3,11α-diyl-diacetat in 100 ml Methanol wurden mit 14.6 g (5 Äquiv.) Natriumhydrogencarbonat versetzt und die Mischung wurde über Nacht bei RT gerührt. Man versetzte mit 100 ml Wasser und 1 ml 1M Salzsäure und ließ 30 min rühren. Man extrahierte viermal mit Ethylacetat. Dabei sammelte sich in der organischen Phase ein Feststoff, der abgesaugt und getrocknet wurde. Man erhielt 3.74 g (33% d. Th.) der Titelverbindung. Zusätzlich wurden 6.39 g (56% d. Th.) der Titelverbindung isoliert durch Waschen der organischen Phase mit gesättigter Natriumchloridlösung, Trockenen über Natriumsulfat, Einengen, Ausrühren des Rückstandes aus Ethylacetat, Absaugen und Trocknen im Vakuum.
$^1$H-NMR (300MHz, DMSO-$d_6$): δ [ppm]= 0.79 (s, 3H), 1.25 (t, 1H), 1.36 - 1.69 (m, 4H), 1.75 - 1.98 (m, 2H), 1.98 - 2.18 (m, 5H), 2.34 - 2.43 (m), 2.68 (t, 2H), 5.16 (td, 1H), 6.43 - 6.55 (m, 2H), 6.76 (d, 1H), 9.07 (s, 1H).

**Intermediat 6**

**3-{[(1,1,2,2,3,3,4,4,4-Nonafluorbutyl)sulfonyl]oxy}-17-oxoestra-1,3,5(10)-trien-11α-yl-acetat**

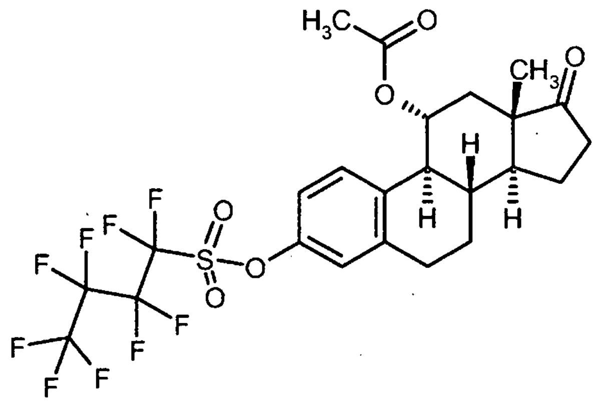

Eine Lösung aus 10.1 g (31 mmol) 3-Hydroxy-17-oxoestra-1,3,5(10)-trien-11α-yl-acetat in 20 ml THF wurde mit 12.8 g (3 Äquiv.) Kaliumcarbonat und 6.5 ml (1.2 Äquiv.) 1,1,2,2,3,3,4,4,4-Nonafluorbutan-1-sulfonylfluorid versetzt und man erhitzte 4 h unter Rückfluß und rührte 18 h bei RT. Man setzte erneut 1 ml 1,1,2,2,3,3,4,4,4-Nonafluorbutan-1-sulfonylfluorid zu und erhitzte 3 h unter Rückfluß. Man versetzte mit Wasser und gesättigter Kochsalzlösung, ließ 20 min rühren, trennte die Phasen und extrahierte die wässrige Phase dreimal mit je 50 ml Ethylacetat. Die vereinigten organischen Phasen wurden zweimal mit je 50 ml Wasser und zweimal mit 50 ml gesättigter Kochsalzlösung gewaschen, über Natriumsulfat getrocknet, filtriert und eingeengt. Nach säulenchromatographischer Reinigung an Kieselgel (Hexan/Ethylacetat) erhielt man 18.1 g (96% d. Th.) 3-{[(1,1,2,2,3,3,4,4,4-Nonafluorbutyl)sulfonyl]oxy}-17-oxoestra-1,3,5(10)-trien-11α-yl-acetat.

$^1$H-NMR(400MHz, DMSO-$d_6$): δ [ppm]= 0.85 (s, 3H), 1.26 - 1.37 (m, 1H), 1.47 - 1.76 (m, 4H), 1.83 - 2.02 (m, 2H), 2.03 - 2.25 (m, 5H, enthält s bei 2.06 ppm), 2.41 - 2.47 (m), 2.59 (t, 1H), 2.77 - 2.95 (m, 2H), 5.29 (td, 1H), 7.15 (d, 1H), 7.23 - 7.29 (m, 2H).

**Intermediat 7**

**Methyl-11α-acetoxy-17-oxoestra-1,3,5(10)-trien-3-carboxylat**

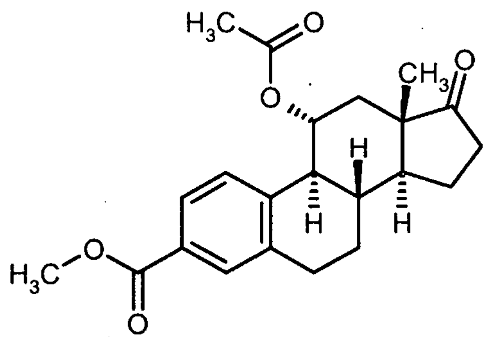

10.0 g (16.4 mmol) 3-{[(1,1,2,2,3,3,4,4,4-Nonafluorbutyl)sulfonyl]oxy}-17-oxoestra-1,3,5(10)-trien-11α-yl-acetat, 230 mg (6 mol%) Palladium(II)acetat und 440 mg (6 mol%) 1,3-Bis(diphenylphoshino)propan wurden unter Argon in einem Autoklaven vorgelegt und mit 36 ml Methanol, 54 ml DMSO und 6 ml Triethylamin versetzt. Das Reaktionsgemisch wurde dreimal mit Kohlenmonoxid gespült und bei 7.5 bar Kohlenmonoxiddruck 30 min bei RT gerührt. Danach wurde der Autoklav entspannt, evakuiert und bei 6.8 bar Kohlenmonoxiddruck bei 70°C 3.5 h gerührt. Man engte ein, und der Rückstand wurde in Wasser und Ethylacetat aufgenommen. Man trennte die Phasen und extrahierte die wässrige Phase zweimal mit Ethylacetat. Die vereinigten organischen Phasen wurden mit 1M Salzsäure und gesättigter Natriumhydrogencarbonatlösung gewaschen, über Natriumsulfat getrocknet und eingeengt. Nach Reinigung des Rückstandes durch Säulenchromatographie an Kieselgel (Hexan/Ethylacetat) erhielt man 5.96 g (98% d. Th.) der Titelverbindung als Feststoff.

$^1$H-NMR (300MHz, DMSO-$d_6$): δ [ppm]= 0.81 (s, 3H), 1.29 (t, 1H), 1.40 - 1.76 (m, 4H), 1.78 - 2.00 (m, 2H), 2.00 - 2.21 (m, 5H, enthält s bei 2.03 ppm), 2.37 - 2.52 (m, verdeckt durch DMSO Signal), 2.59 (t, 1H), 2.72 - 2.93 (m, 2H), 3.79 (s, 3H), 5.29 (td, 1H), 5.23 - 5.38 (m, 1H), 7.08 (d, 1H), 7.68 - 7.75 (m, 2H).

**Intermediat 8**

**Methyl-11α-acetoxy-17-{[(frifluormethyl)sulfonyl]oxy}estra-1,3,5(10),16-tetraen-3-carboxylat**

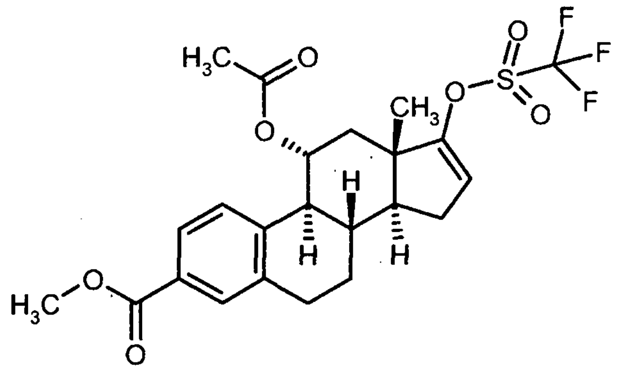

2.96 g (7.99 mmol) Methyl-11α-acetoxy-17-oxoestra-1,3,5(10)-trien-3-carboxylat wurden analog zur Herstellung von Intermediat 1 zu 5.13 g der Titelverbindung als Rohprodukt (enthielt noch 2,6-Di-*tert*-butylpyridin) umgesetzt. $^1$H-NMR (300MHz, DMSO-$d_6$): δ [ppm]= 0.93 (s, 3H), 1.41 - 1.71 (m, 3H), 1.71 - 1.87 (m, 1H), 1.87 - 2.16 (m, 5H, enhält s bei 2.03 ppm), 2.16 - 2.40 (m, 2H), 2.67 (t, 1H), 2.74 - 2.93 (m, 2H), 3.79 (s, 3H), 5.34 (td, 1H), 5.75 - 5.82 (m, 1H), 7.03 (d, 1H), 7.67 - 7.75 (m, 2H).

**Intermediat 9**

**Methyl-11α-acetoxy-17-(5-fluorpyridin-3-yl)estra-1,3,5(10),16-tetraen-3-carboxylat**

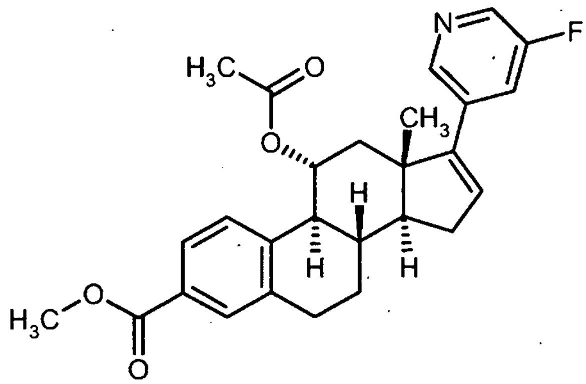

2.50 g (4.98 mmol) Methyl-11α-acetoxy-17-{[(trifluormethyl)sulfonyl]oxy}estra-1,3,5(10),16-tetraen-3-carboxylat wurden mit 981 mg (1.4 Äquiv.) 5-Fluorpyridin-3-boronsäure in Gegenwart von 170 mg (5 mol%) [1,3-Bis(2,6-Diisopropylphenyl)imidazol-2-yliden](3-chloropyridyl)palladium(II) dichlorid (PEPPSI™-IPr, CAS 905459-27-0) analog zu Intermediat 2-a innerhalb von 5 h bei Rückflußtemperatur umgesetzt. Man erhielt 2.62 g der Titelverbindung als Rohprodukt.

**Intermediat 10**

**Methyl-17-(5-fluorpyridin-3-yl)-11α-hydroxyestra-1,3,5(10),16-tetraen-3-carboxylat**

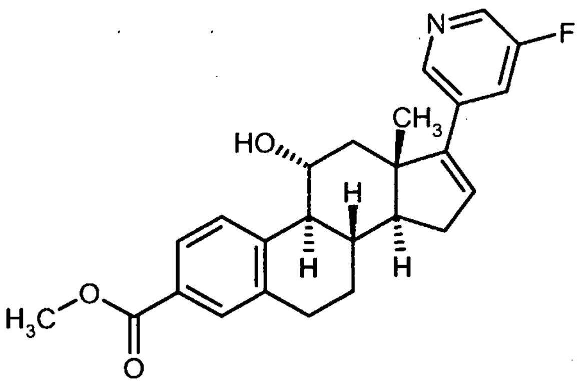

Zu 2.62 g (5.83 mmol) Methyl-11α-acetoxy-17-(5-fluorpyridin-3-yl)estra-1,3,5(10),16-tetraen-3-carboxylat in 40 ml Methanol wurden 4.0 g (5 Äquiv.) Kaliumcarbonat gegeben und die Mischung wurde 3 h bei RT gerührt. Man verdünnte mit Wasser und 1M Salzsäure und extrahierte dreimal mit Ethylacetat. Die vereinigten organischen Phasen wurden mit Wasser und gesättigter Natriumchloridlösung gewaschen, über Natriumsulfat getrocknet und eingeengt. Nach Säulenchromatographie an Kieselgel (Hexan/Ethylacetat) erhielt man 1.19 g (50% d. Th.) der Titelverbindung.
$^1$H-NMR (300MHz, DMSO-$d_6$, ausgewählte Signale): δ [ppm]= 0.95 (s, 3H), 1.40 - 1.61 (m, 3H), 2.78 - 2.97 (m, 2H), 3.79 (s, 3H), 4.06 - 4.21 (m, 1H), 4.79 - 4.92 (m, 1H), 6.26 (br. s., 1H), 7.59 - 7.74 (m, 3H), 8.07 (d, 1H), 8.39 - 8.54 (m, 2H).

**Intermediat 11**

**Methyl-11β-fluor-17-(5-fluorpyridin-3-yl)estra-1,3,5(10),16-tetraen-3-carboxylat**

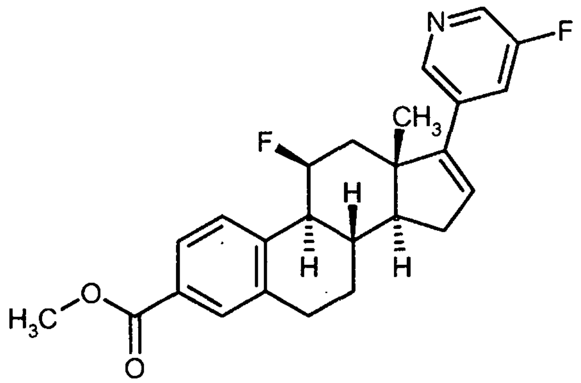

Zu einer eiskalten Lösung aus 531 mg (3.49 mmol) Methyl-17-(5-fluorpyridin-3-yl)-11α-hydroxyestra-1,3,5(10),16-tetraen-3-carboxylat in 15 ml THF wurden 0.52 ml (1.65 Äquiv.) 1,8-Diazabicyclo[5.4.0]undec-7-en und 0.58 ml (1.5 Äquiv.) 1,1,2,2,3,3,4,4,4-Nonafluor-butan-1-sulfonylfluorid getropft und es wurde unter Eisbadkühlung 3 h gerührt. Man engte ein und reinigte durch Säulenchromatographie an Kieselgel (Hexan/Ethylacetat). Man erhielt 747 mg (84% d. Th.) der Titelverbindung als Rohprodukt.
$^1$H-NMR (300MHz, DMSO-$d_6$, ausgewählte Signale): δ [ppm]= 2.86 - 2.97 (m, 2H), 5.57 - 5.83 (m, 1H), 6.26 - 6.32 (m, 1H), 7.45 - 7.53 (m, 1H), 7.65 - 7.78 (m, 3H), 8.39 - 8.53 (m, 2H).

**Intermediat 12**

**11β-Fluor-17-(5-fluorpyridin-3-yl)estra-1,3,5(10),16-tetraen-3-carbonsäure**

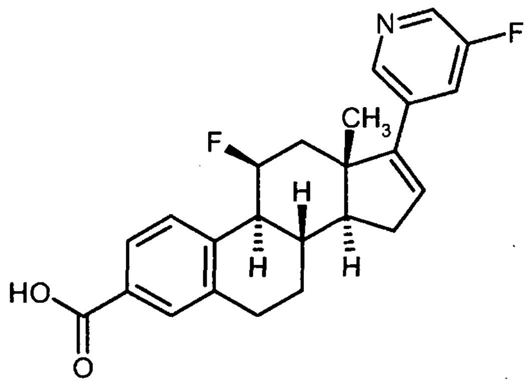

Eine Mischung aus 862 mg (2.11 mmol) Methyl-11β-fluor-17-(5-fluorpyridin-3-yl)estra-1,3,5(10),16-tetraen-3-carboxylat in 10 ml THF wurde mit 5 ml Methanol und 442 mg Lithiumhydroxid Monohydrat in 5 ml Wasser versetzt und bei Raumtemperatur über Nacht gerührt. Das Reaktionsgemisch wurde mit Wasser versetzt und mit 10%iger wässriger Zitronensäurelösung auf pH = 4 eingestellt. Der ausgefallene Niederschlag wurde abgesaugt und mit Ethylacetat gewaschen und getrocknet. Man erhielt 498 mg (60% d. Th.) eines weißen Feststoffes.
$^1$H-NMR (500MHz, DMSO-$d_6$): δ [ppm]= 1.19 (s, 3H), 1.44 - 1.59 (m, 1H), 1.80 - 1.96 (m, 2H), 1.96 - 2.08 (m, 2H), 2.18 - 2.29 (m, 1H), 2.32 - 2.42 (m, 1H), 2.59 (td, 1H), 2.74 (dd, 1H), 2.77 (br. s., 1H), 2.86 - 3.00 (m, 2H), 5.66 - 5.80 (m, 1H), 6.32 (dd, 1H), 7.48 (d, 1H), 7.65 - 7.78 (m, 3H), 8.47 (d, 1H), 8.54 (t, 1H).

**Beispiel 1**

**4-[({[17-(5-Fluorpyridin-3-yl)estra-1,3,5(10),16-tetraen-3-yl]carbonyl}amino)methyl]-3,4,5,6-tetrahydro-2*H*-pyran-4-carbonsäure**

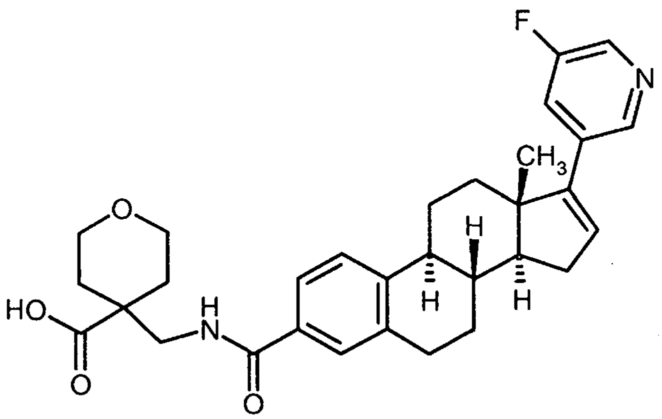

Stufe A: 100 mg (0.26 mmol) 17-(5-Fluorpyridin-3-yl)estra-1,3,5(10),16-tetraen-3-carbonsäure wurden in 1 ml DMF und 3 ml THF vorgelegt. Dann wurden 119 mg (2.0 Äquiv.) Ethyl-4-(aminomethyl)-3,4,5,6-tetrahydro-2*H*-pyran-4-carboxylat Hydrochlorid, 41 mg (2.0 Äquiv.) 1-Hydroxy-1*H*-benzotriazol Hydrat, 102 mg (2.0 Äquiv.) 1-(3-Dimethylaminopropyl)-3-ethylcarbodiimid Hydrochlorid und 0.11 ml Triethylamin zugegeben und die Mischung wurde über Nacht bei RT gerührt.
Stufe B: Danach wurden 0.66 ml einer 2M Natronlaugelösung und 0.50 ml Methanol zugesetzt und über Nacht bei RT gerührt. Das Reaktionsgemisch wurde mit Wasser versetzt und anschließend mit einer 10%igen wässrigen Zitronensäurelösung auf einen pH-Wert von 3 bis 4 angesäuert. Die wässrige Phase wurde dreimal mit Ethylacetat extrahiert, die vereinigten org. Phasen wurden eingeengt und der Rückstand durch präparative HPLC (Acetonitril/Wasser/Ameisensäure) aufgereinigt. Man erhielt 76 mg (55% d. Th.) eines Feststoffes.
$C_{31}H_{35}FN_2O_4$ (518.6). MS-ES+ gefundene Masse 518.26.
$^1$H NMR (300 MHz, DMSO-$d_6$, ausgewählte Signale) δ ppm 0.99 (s, 3 H), 1.36 - 1.99 (m, 10 H), 2.05 - 2.21 (m, 2 H), 2.25 - 2.44 (m, 3 H), 2.82 - 2.95 (m, 2 H), 3.39 (d, 2 H), 3.67 - 3.76 (m, 2 H), 6.25 - 6.29 (m, 1 H), 7.31 (d, 1 H), 7.49 -

7.60 (m, 2 H), 7.68 (dt, 1 H), 8.27 (t, 1 H), 8.43 (d, 1 H), 8.49 (s, 1 H), 12.5 (br. s).

**Beispiel 2**

***N*-{[17-(5-Fluorpyridin-3-yl)estra-1,3,5(10),16-tetraen-3-yl]carbonyl}-*N*-methyl-β-alanin**

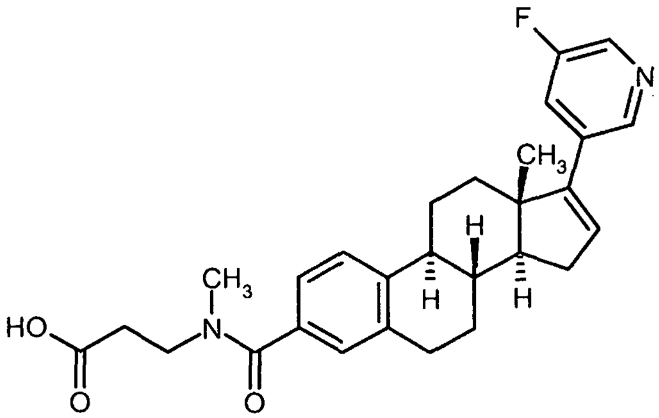

Stufe A: Eine Mischung aus 4.00 g (10.6 mmol) 17-(5-Fluorpyridin-3-yl)estra-1,3,5(10),16-tetraen-3-carbonsäure und 3.38 g (2 Äquiv.) *tert*-Butyl-*N*-methyl-β-alaninat in 100 ml THF und 5 ml DMF wurde mit 1.62 g (1.0 Äquiv.) 1-Hydroxy-1*H*-benzotriazol Hydrat, 4.06 g (2.0 Äquiv.) 1-(3-Dimethylaminopropyl)-3-ethylcarbodiimid Hydrochlorid (EDC) und 4.4 ml Triethylamin versetzt und 18 h bei RT gerührt. Man verdünnte mit Wasser, extrahierte dreimal mit Ethylacetat, wusch mit Kochsalzlösung und engte ein. Nach Reinigung des Rückstandes mit Kieselgel (Hexan/Ethylacetat) wurden 5.1 g *tert*-Butyl-*N*-{[17-(5-fluorpyridin-3-yl)estra-1,3,5(10),16-tetraen-3-yl]carbonyl}-*N*-methyl-β-alaninat (93% d. Th.) als Feststoff erhalten.

Stufe B: 1.00 g (1.93 mmol) *tert*-Butyl-*N*-{[17-(5-fluorpyridin-3-yl)estra-1,3,5(10),16-tetraen-3-yl]carbonyl}-*N*-methyl-β-alaninat wurden in 15 ml Dichlormethan vorgelegt, 1.5 ml Trifluoressigsäure wurde zugegeben und die Mischung wurde bei 40°C über Nacht gerührt, auf Eiswasser gegossen, kurz gerührt und dreimal mit Dichlormethan extrahiert. Die vereinigten organischen Phasen wurden mit gesättigter Kochsalzlösung gewaschen, über einen wasserabweisenden Filter filtriert und eingeengt. Das Rohprodukt wurde mit Diethylether versetzt, gerührt, abgesaugt und mit Diethylether gewaschen und getrocknet. Man erhielt 0.79 g (89% d. Th.) *N*-{[17-(5-Fluorpyridin-3-yl)estra-1,3,5(10),16-tetraen-3-yl]carbonyl}-*N*-methyl-β-alanin.

$C_{28}H_{31}FN_2O_3$ (462.6). MS-ES+ gefundene Masse 462.23.

$^1$H-NMR (300MHz, DMSO-d6): δ [ppm]= 1.00 (s, 3H), 1.33 - 1.79 (m, 5H), 1.82 - 1.99 (m, 1H), 2.08 - 2.21 (m, 2H), 2.21 - 2.43 (m, 3H), 2.50 (s), 2.74 - 2.88 (5H, enthält s bei 2.88 ppm), 3.36 - 3.71 (m), 6.27 (s., 1H), 6.99 - 7.16 (m, 2H), 7.28 (d, 1H), 7.68 (dt, 1H), 8.43 (d, 1H), 8.49 (s, 1H), 8.48 - 8.56 (m, 1H), 12.28 (br. s.).

**Beispiel 3**

**1-[({[17-(5-Fluorpyridin-3-yl)estra-1,3,5(10),16-tetraen-3-yl]carbonyl}amino)methyl]-cyclopropan-1-carbonsäure**

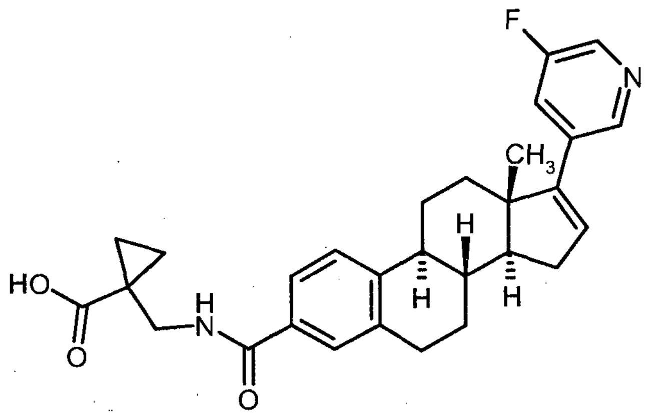

100 mg (0.26 mmol) 17-(5-Fluorpyridin-3-yl)estra-1,3,5(10),16-tetraen-3-carbonsäure wurden mit 88 mg (2.0 Äquiv.) Methyl-1-(aminomethyl)cyclopropan-1-carboxylat Hydrochlorid analog zu Beispiel 1 zu 72 mg (57% d. Th.) der Titelverbindung umgesetzt.
$C_{29}H_{31}FN_2O_3$ (474.6). MS-ES+ gefundene Masse 474.23.
$^1$H-NMR (300MHz, DMSO-d6): δ [ppm]= 0.82 - 0.90 (m, 2H), 0.95 - 1.06 (m, 5H), 1.33 - 1.81 (m, 5H), 1.56 (d, 3H), 1.84 - 1.97 (m, 1H), 1.84 - 1.97 (m, 1H), 2.05 - 2.44 (m), 2.80 - 2.94 (m, 2H), 3.44 - 3.54 (m, 2H), 6.27 (s., 1H), 7.31 (d, 1H), 7.50 - 7.60 (m, 2H), 7.68 (dt, 1H), 8.18 (t, 1H), 8.43 (d, 1H), 8.49 (s, 1H), 12.3 (s, 1H).

**Beispiel 4**

**1-[({[17-(5-Fluorpyridin-3-yl)estra-1,3,5(10),16-tetraen-3-yl]carbonyl}amino)methyl]cyclopentan-1-carbonsäure**

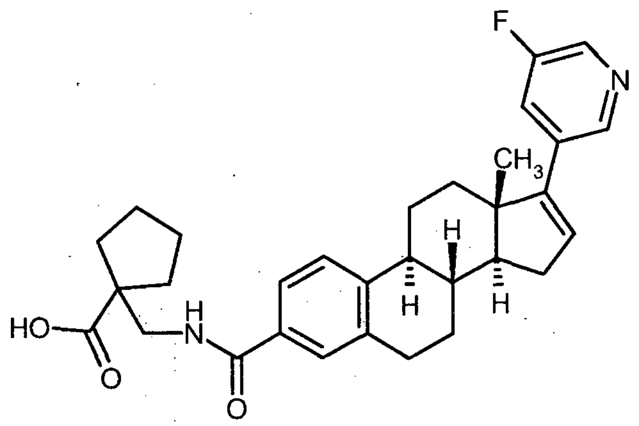

100 mg (0.26 mmol) 17-(5-Fluorpyridin-3-yl)estra-1,3,5(10),16-tetraen-3-carbonsäure wurden mit 103 mg (2.0 Äquiv.) Methyl-1-(aminomethyl)cyclopentan-1-carboxylat Hydrochlorid analog zu Beispiel 1, (die Stufe B erfolgte bei 50°C über Nacht und nach Zugabe von weiteren 5 Equiv. 2M Natronlauge durch Rühren bei 60°C über Nacht) zu 63 mg (48% d. Th.) der Titelverbindung umgesetzt.
$C_{31}H_{35}FN_2O_3$ (502.6). MS-ES+ gefundene Masse 502.26.
$^1$H-NMR (400MHz, DMSO-d6): δ [ppm]= 0.99 (s, 3H), 1.36 - 1.66 (m), 1.73 (td, 1H), 1.81 - 1.95 (m, 3H), 2.07 - 2.20 (m, 2H), 2.25 - 2.44 (m), 2.83 - 2.95 (m, 2H), 3.45 (d, 2H), 6.25 - 6.29 (m, 1H), 7.31 (d, 1H), 7.49 - 7.56 (m, 2H), 7.69 (dt, 1H), 8.14 (t, 1H), 8.43 (d, 1H), 8.49 (s, 1H), 12.2 (s).

**Beispiel 5**

**3-({[17-(5-Fluorpyridin-3-yl)estra-1,3,5(10),16-tetraen-3-yl]carbonyl}amino)-2,2-dimethylpropansäure**

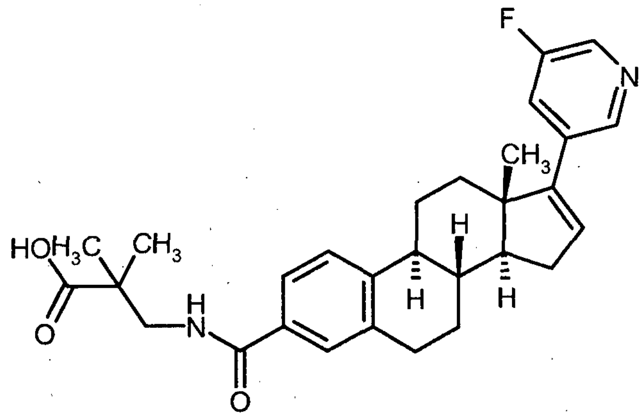

100 mg (0.26 mmol) 17-(5-Fluorpyridin-3-yl)estra-1,3,5(10),16-tetraen-3-carbonsäure wurden mit 89 mg (2.0 Äquiv.) Methyl-3-amino-2,2-dimethylpropanoat Hydrochlorid analog zu Beispiel 1 (die Stufe B erfolgte bei 50°C über Nacht und nach Zugabe von weiteren 5 Äquiv. 2M Natronlauge durch Rühren bei 60°C über Nacht) zu 63 mg (50% d. Th.) der Titelverbindung umgesetzt.
$C_{29}H_{33}FN_2O_3$ (476.6). MS-ES+ gefundene Masse 476.25.
$^1$H-NMR (400MHz, DMSO-d6): $\delta$ [ppm]= 0.99 (s, 3H), 1.06 (s, 6H), 1.36 - 1.66 (m, 4H), 1.73 (td, 1H), 1.86 - 1.95 (m, 1H), 2.07 - 2.20 (m, 2H), 2.25 - 2.45 (m, 3H), 2.84 - 2.92 (m, 2H), 3.37 (d, überlagert von Wassersignal), 6.25 - 6.29 (m, 1H), 7.31 (d, 1H), 7.50 - 7.57 (m, 2H), 7.69 (dt, 1H), 8.15 (t, 1H), 8.43 (d, 1H), 8.49 (t, 1H), 12.2 (s).

**Beispiel 6**

**1-{[17-(5-Fluorpyridin-3-yl)estra-1,3,5(10),16-tetraen-3-yl]carbonyl}piperidin-4-carbonsäure**

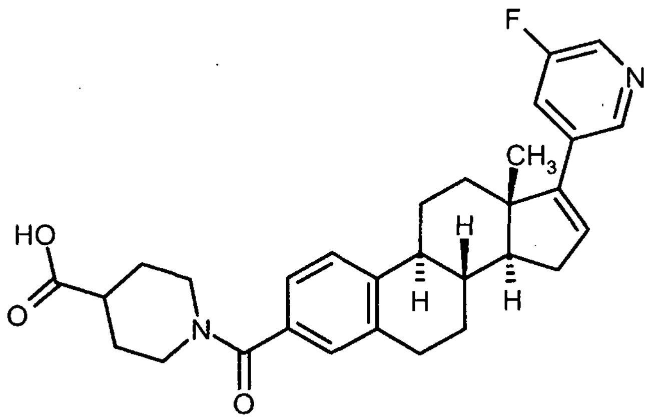

100 mg (0.26 mmol) 17-(5-Fluorpyridin-3-yl)estra-1,3,5(10),16-tetraen-3-carbonsäure wurden mit 83 mg (2.0 Äquiv.) Ethylpiperidin-4-carboxylat analog zu Beispiel 1 (die Stufe B erfolgte bei 50°C innerhalb von 5 h) zu 65 mg (50% d. Th.) der Titelverbindung umgesetzt.
$C_{30}H_{33}FN_2O_3$ (488.6). MS-ES+ gefundene Masse 488.25.
$^1$H-NMR (400MHz, DMSO-$d_6$): $\delta$ [ppm]= 1.00 (s, 3H), 1.33 - 1.96 (m, 10H), 2.03 - 2.22 (m, 2H), 2.24 - 2.43 (m, 3H), 2.79 - 2.94 (m, 3H), 3.02 (br. s., 1H), 3.57 (br. s., 1H), 4.26 (br. s., 1H), 6.25 - 6.29 (m, 1H), 7.00 - 7.11 (m, 2H), 7.29 (d, 1H), 7.69 (dt, 1H), 8.43 (d, 1H), 8.47 - 8.51 (m, 1H), 12.3 (s).

**Beispiel 7**

***N*-{[17-(5-Fluorpyridin-3-yl)estra-1,3,5(10),16-tetraen-3-yl]carbonyl}-2-methylalanin**

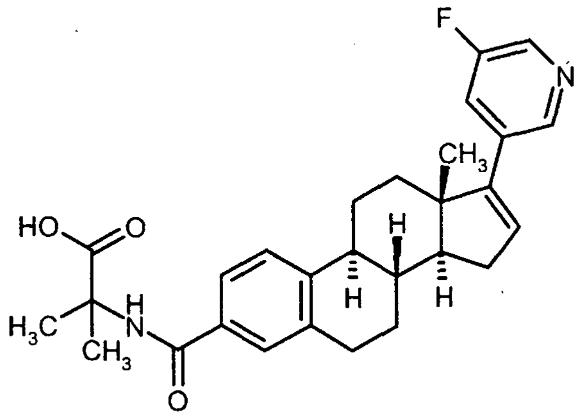

100 mg (0.26 mmol) 17-(5-Fluorpyridin-3-yl)estra-1,3,5(10),16-tetraen-3-carbonsäure wurden mit 81 mg (2.0 Äquiv.) Methyl-3-amino-2,2-dimethylpropanoat Hydrochlorid analog zu Beispiel 1 (zur Durchführung von Stufe B wurde über Nacht auf 50°C erwärmt und nach Zugabe von weiteren 5 Äquiv. 2M Natronlauge wurde bei 60°C über Nacht gerührt) zu 81 mg (66% d. Th.) der Titelverbindung umgesetzt.
$C_{28}H_{31}FN_2O_3$ (462.6). MS-ES+ gefundene Masse 462.23.
$^1$H-NMR (300MHz, DMSO-d6): $\delta$ [ppm]= 0.99 (s, 3H), 1.39 (s, 6H), 1.4 - 1.82 (m), 1.86 - 1.98 (m, 1H), 2.05 - 2.43 (m, 2H), 2.20 - 2.50 (m), 2.83 - 2.94 (m, 2H), 6.27 (s, 1H), 7.31 (d, 1H), 7.52 - 7.61 (m, 2H), 7.65 - 7.72 (m, 1H), 8.29 (s, 1H), 8.43 (d, 1H), 8.46 - 8.53 (m, 1H), 12.1 (s).

**Beispiel 8**

**4-({[17-(5-Fluorpyridin-3-yl)estra-1,3,5(10),16-tetraen-3-yl]carbonyl}amino)butansäure**

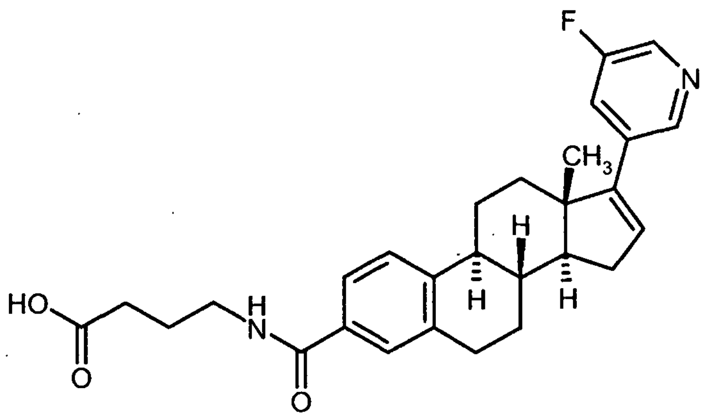

100 mg (0.26 mmol) 17-(5-Fluorpyridin-3-yl)estra-1,3,5(10),16-tetraen-3-carbonsäure wurden mit 81 mg (2.0 Äquiv.) Methyl-4-aminobutanoat Hydrochlorid analog zu Beispiel 1 zu 58 mg (48% d. Th.) der Titelverbindung umgesetzt.
$C_{28}H_{31}FN_2O_3$ (462.6). MS-ES+ gefundene Masse 462.23.
$^1$H-NMR (300MHz, DMSO-d6): $\delta$ [ppm]= 0.99 (s, 3H), 1.39 - 1.78 (m, 7H), 1.83 - 1.99 (m, 1H), 2.05 - 2.50 (m, überlagert durch DMSO-Signal), 3.1 - 3.4 (m, überlagert durch Wasser-Signal), 2.79 - 2.98 (m, 2H), 6.26 (s., 1H), 7.31 (d, 1H), 7.52 - 7.59 (m, 2H), 7.67 (dt, 1H), 8.32 (t, 1 H), 8.43 (d, 1H), 8.49 (s, 1 H), 12.0 (s).

**Beispiel 9**

***N*-{[17-(5-Fluorpyridin-3-yl)estra-1,3,5(10),16-tetraen-3-yl]carbonyl}-β-alanin**

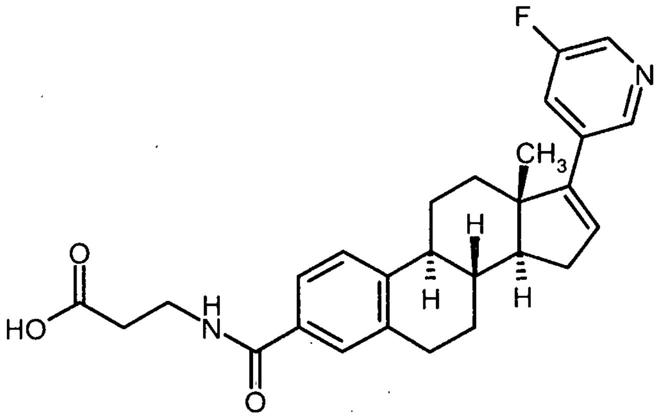

100 mg (0.26 mmol) 17-(5-Fluorpyridin-3-yl)estra-1,3,5(10),16-tetraen-3-carbonsäure wurden mit 81 mg (2.0 Äquiv.) Ethyl-β-alaninat Hydrochlorid analog zu Beispiel 1 zu 59 mg (50% d. Th.) der Titelverbindung umgesetzt.
$C_{27}H_{29}FN_2O_3$ (448.5). MS-ES+ gefundene Masse 448.22.
$^1$H-NMR (300MHz, DMSO-d6): δ [ppm]= 0.99 (s, 3H), 1.38 - 1.78 (m, 5H), 1.83 - 1.98 (m, 1H), 2.05 - 2.21 (m, 2H), 2.25 - 2.44 (m, überlagert von DMSO Signal), 2.80 - 2.94 (m, 2H), 3.35 - 3.51 (m, überlagert von Wasser-Signal), 6.26 (s., 1H), 7.31 (d, 1H), 7.50 - 7.58 (m, 2H), 7.62 - 7.74 (m, 1H), 8.36 (t, 1H), 8.43 (d, 1H), 8.49 (s, 1H), 12.2 (s).

**Beispiel 10**

***N*-{[17-(5-Fluorpyridin-3-yl)estra-1,3,5(10),16-tetraen-3-yl]carbonyl}glycin**

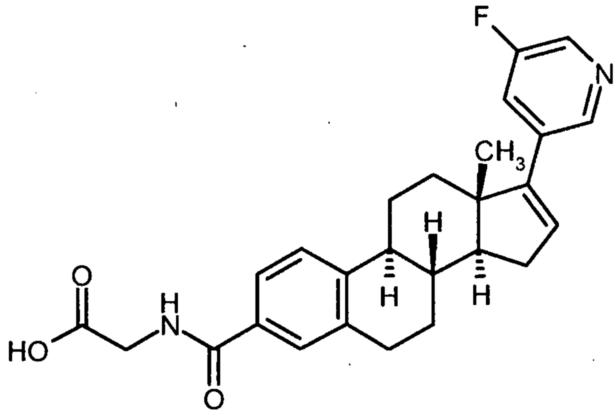

100 mg (0.26 mmol) 17-(5-Fluorpyridin-3-yl)estra-1,3,5(10),16-tetraen-3-carbonsäure wurden mit 67 mg (2.0 Äquiv.) Methylglycinat Hydrochlorid analog zu Beispiel 1 zu 58 mg (50% d. Th.) der Titelverbindung umgesetzt.
$C_{26}H_{27}FN_2O_3$ (434.5). MS-ES+ gefundene Masse 434.20.
$^1$H-NMR (400MHz, DMSO-$d_6$): δ [ppm]= 1.00 (s, 3H), 1.37 - 1.67 (m, 4H), 1.74 (td, 1H), 1.85 - 1.97 (m, 1H), 2.07 - 2.21 (m, 2H), 2.26 - 2.5 (m, überlagert von DMSO Signal), 2.84 - 2.94 (m, 2H), 3.86 (d, 2H), 6.25 - 6.29 (m, 1H), 7.34 (d, 1H), 7.55 - 7.61 (m, 2H), 7.64 - 7.72 (m, 1H), 8.43 (d, 1H), 8.49 (t, 1H), 8.64 (t, 1H), 12.5 (s).

**Beispiel 11**

**(1*R**,2*S**)-2-({[17-(5-Fluorpyridin-3-yl)estra-1,3,5(10),16-tetraen-3-yl]carbonyl}amino)cyclopentan-1-carbonsäure**

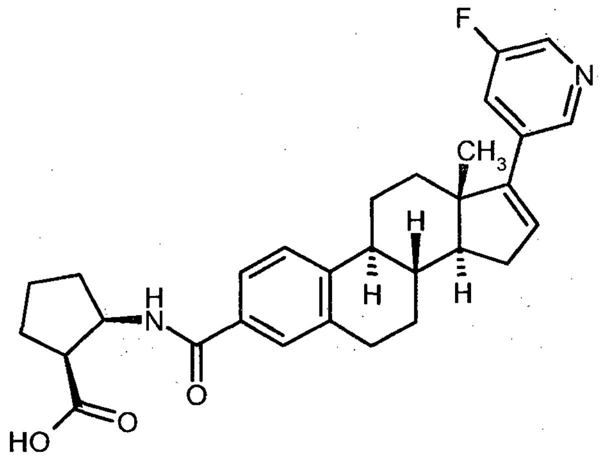

100 mg (0.26 mmol) 17-(5-Fluorpyridin-3-yl)estra-1,3,5(10),16-tetraen-3-carbonsäure wurden mit 103 mg (2.0 Äquiv.) (1*R**,2*S**)-Ethyl-2-aminocyclopentan-1-carboxylat Hydrochlorid analog zu Beispiel 1 zu 63 mg (49% d. Th.) der Titelverbindung umgesetzt.
$C_{30}H_{33}FN_2O_3$ (488.6). MS-ES+ gefundene Masse 488.25.
$^1$H-NMR (300MHz, DMSO-$d_6$): δ [ppm]= 1.00 (s, 3H), 1.39 - 1.66 (m, 5H), 1.68 - 1.96 (m, 7H), 2.02 - 2.21 (m, 2H), 2.24 - 2.41 (m), 2.78 - 2.98 (m, 3H), 4.42 - 4.57 (m, 1H), 6.27 (s., 1H), 7.30 (d, 1H), 7.46 - 7.58 (m, 2H), 7.68 (dt, 1H), 8.03 (d, 1H), 8.43 (d, 1H), 8.46 - 8.52 (m, 1H), 11.9 (s).

**Beispiel 12**

**(*S*)-3-({[17-(5-Fluorpyridin-3-yl)estra-1,3,5(10),16-tetraen-3-yl]carbonyl}amino)-butansäure**

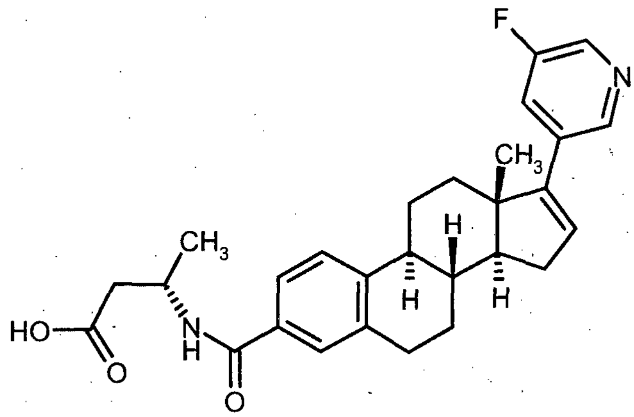

100 mg (0.26 mmol) 17-(5-Fluorpyridin-3-yl)estra-1,3,5(10),16-tetraen-3-carbonsäure wurden mit 84 mg (*S*)-3-Aminobuttersäure-*tert*-butylester analog zu Beispiel 1 (bei der Stufe B addierte man zusätzlich 5 Äquiv. 2M Natronlauge, ließ 4 h rühren, addierte erneut 5 Äquiv. 2M Natronlauge, ließ 30 min bei 110°C/300 Watt im Mikrowellenofen rühren, erhitzte nach Zugabe von 10 Äquiv. 2M Natronlauge im Mikrowellenofen 60 min bei 120°C/300 Watt und 60 min bei 130°C/300 Watt) zu 24 mg (20% d. Th.) der Titelverbindung umgesetzt.
$C_{28}H_{31}FN_2O_3$ (462.6). MS-ES+ gefundene Masse 462.23.
$^1$H-NMR (400MHz, DMSO-$d_6$): δ [ppm]= 1.02 (s, 3H), 1.16 (d, 3H), 1.38 - 1.68 (m, 4H), 1.76 (td, 1H), 1.87 - 2.01 (m, 1H), 2.09 - 2.24 (m, 2H), 2.27 - 2.46 (m, 4H), 2.51 - 2.61 (m, 1H), 2.82 - 3.00 (m, 2H), 4.31 (spt, 1H), 6.27 - 6.31 (m, 1H), 7.34 (d, 1H), 7.50 - 7.61 (m, 2H), 7.70 (dt, 1H), 8.16 (d, 1H), 8.46 (d, 1H), 8.50 - 8.54 (m, 1H), 12.2 (br. s., 1H).

**Beispiel 13**

**(*R*)-3-({[17-(5-Fluorpyridin-3-yl)estra-1,3,5(10),16-tetraen-3-yl]carbonyl}amino)-butansäure**

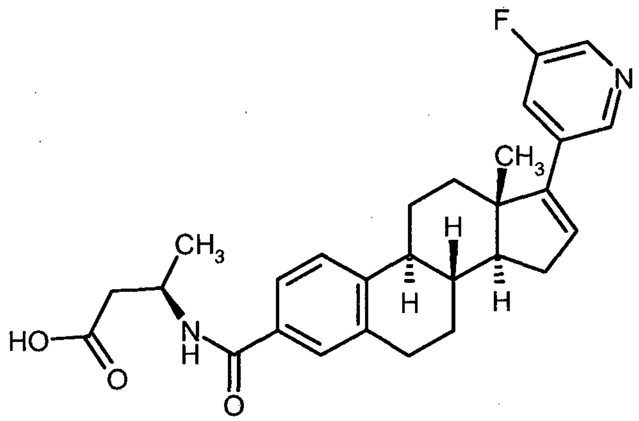

100 mg (0.26 mmol) 17-(5-Fluorpyridin-3-yl)estra-1,3,5(10),16-tetraen-3-carbonsäure wurden mit 84 mg (*R*)-3-Aminobuttersäure-*tert*-butylester analog zu Beispiel 1 (bei der Stufe B

wurden zusätzlich 5 Äquivalente 2M Natronlauge zugegeben und 30 h bei 50°C gerührt) zu 34 mg (28% d. Th.) der Titelverbindung umgesetzt.
$C_{28}H_{31}FN_2O_3$ (462.6). MS-ES+ gefundene Masse 462.23.
$^1$H-NMR (400MHz, DMSO-$d_6$): δ [ppm]= 1.02 (s, 3H), 1.16 (d, 3H), 1.39 - 1.69 (m, 4H), 1.76 (td, 1H), 1.88 - 2.00 (m, 1H), 2.09 - 2.23 (m, 2H), 2.27 - 2.47 (m, 4H), 2.51 - 2.61 (m, 1H), 2.82 - 2.98 (m, 2H), 4.32 (spt, 1H), 6.24 - 6.34 (m, 1H), 7.34 (d, 1H), 7.52 - 7.60 (m, 2H), 7.70 (dt, 1 H), 8.15 (d, 1H), 8.46 (d, 1 H), 8.50 - 8.54 (m, 1H), 12.1 (br. s., 1H).

**Beispiel 14**

**3-({[17-(5-Methoxypyridin-3-yl)estra-1,3,5(10),16-tetraen-3-yl]carbonyl}amino)-2,2-dimethylpropansäure**

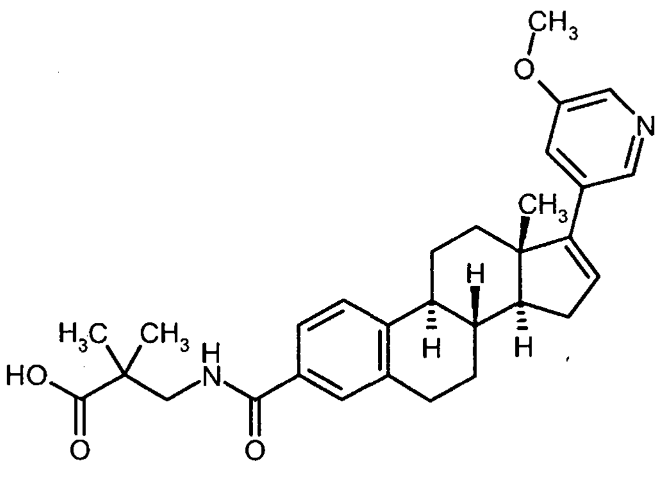

100 mg (0.26 mmol) 17-(5-Methoxypyridin-3-yl)estra-1,3,5(10),16-tetraen-3-carbonsäure wurden mit 75 mg (0.51 mmol) Ethyl-3-amino-2,2-dimethylpropanoat analog zu Beispiel 1 (zur Durchführung der Stufe B wurde bei 50°C 7 h gerührt) zu 12 mg (10% d. Th.) der Titelverbindung umgesetzt.
$C_{30}H_{36}N_2O_4$ (488.63). MS-ES+ gefundene Masse 488.27.
$^1$H-NMR (300MHz, DMSO-d6): δ [ppm]= 0.98 (s, 3H), 1.06 (s, 6H), 1.31 - 1.82 (m, 5H), 1.84 - 1.97 (m, 1H), 2.00 - 2.19 (m, 2H), 2.20 - 2.40 (m), 2.80 - 2.95 (m, 2H), 3.36 - 3.38 (m, teilweise verdeckt durch Wassersignal), 3.81 (s, 3H), 6.16

(s., 1H), 7.20 - 7.37 (m, 2H), 7.46 - 7.63 (m, 2H), 8.09 - 8.27 (m, 3H).

**Beispiel 15**

***N*-{[17-(5-Methoxypyridin-3-yl)estra-1,3,5(10),16-tetraen-3-yl]carbonyl}-β-alanin**

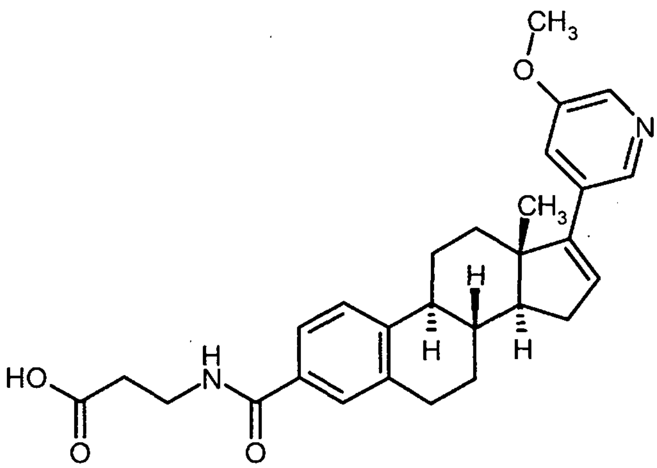

100 mg (0.26 mmol) 17-(5-Methoxypyridin-3-yl)estra-1,3,5(10),16-tetraen-3-carbonsäure wurden mit 79 mg (0.51 mmol) Ethyl-β-alaninat Hydrochlorid analog zu Beispiel 1 (zur Durchführung der Stufe B wurde 7 h bei 50°C gerührt) zu 64 mg (54% d. Th.) der Titelverbindung umgesetzt.
$C_{28}H_{32}N_2O_4$ (460.58). MS-ES+ gefundene Masse 460.24.
$^1$H-NMR (300MHz, DMSO-$d_6$): δ [ppm]= 0.98 (s, 3H), 1.38 - 1.78 (m, 5H), 1.85 - 1.96 (m, 1H), 2.04 - 2.18 (m, 2H), 2.21 - 2.41 (m, 3H), 2.76 - 2.98 (m, 2H), 3.34 - 3.50 (m, 2H), 3.81 (s, 3H), 6.14 - 6.18 (m, 1H), 7.23 - 7.28 (m, 1H), 7.31 (d, 1H), 7.46 - 7.63 (m, 2H), 8.16 (d, 1 H), 8.20 (d, 1 H), 8.38 (t, 1 H), 12.2 (br. s., 1H).

**Beispiel 16**

***N*-{[17-(5-Methoxypyridin-3-yl)estra-1,3,5(10),16-tetraen-3-yl]carbonyl}-*N*-methyl-β-alanin**

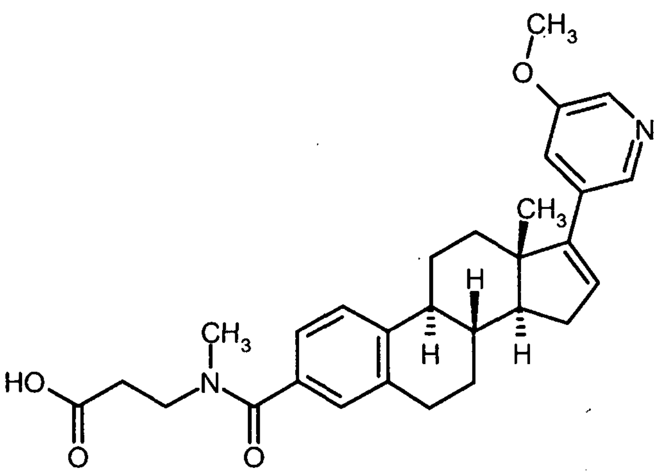

Eine Mischung aus 100 mg (0.26 mmol) 17-(5-Methoxypyridin-3-yl)estra-1,3,5(10),16-tetraen-3-carbonsäure und 82 mg (2 Äquiv.) *tert*-Butyl-*N*-methyl-β-alaninat in 3 ml THF und 1 ml DMF wurde mit 39 mg (1.0 Äquiv.) 1-Hydroxy-1*H*-benzotriazol Hydrat, 98 mg (2.0 Äquiv.) 1-[3-(Dimethylamino)propyl]-3-ethylcarbodiimid Hydrochlorid und 0.11 ml Triethylamin versetzt und 72 h bei RT gerührt. Man verdünnte mit Wasser, extrahierte dreimal mit Ethylacetat und engte

ein. Der Rückstand wurde mit 4 ml Dichlormethan und 1 ml Trifluoressigsäure versetzt und die Mischung 17 h bei Raumtemperatur gerührt. Man engte ein und nach Reinigung des Rückstandes durch präparative HPLC wurden 66 mg der Titelverbindung erhalten.
$C_{29}H_{34}N_2O_4$. (474.61). MS-ES+ gefundene Masse: 474.25.
$^1$H-NMR (300MHz, DMSO-$d_6$): δ [ppm]= 0.99 (s, 3H), 1.38 - 1.78 (m, 5H), 1.82 - 1.96 (m, 1H), 2.03 - 2.18 (m, 2H), 2.21 - 2.43 (m, 3H), 2.79 - 2.93 (m, 5H), 3.42 (br. s., 1H), 3.57 (br. s., 1H), 3.81 (s, 3H), 6.16 (s, 1H), 6.99 7.14 (m, 2H), 7.23 - 7.31 (m, 2H), 8.13 - 8.22 (m, 2H), 12.3 (br. s., 1H).

**Beispiel 17**

***N*-{[17-(Pyrimidin-5-yl)estra-1,3,5(10),16-tetraen-3-yl]carbonyl}-β-alanin**

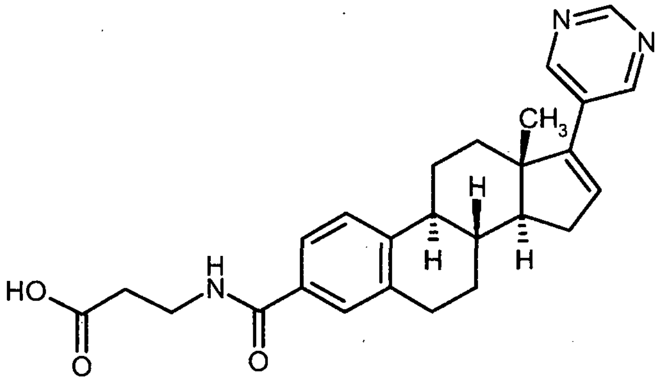

100 mg (0.28 mmol) 17-(Pyrimidin-5-yl)estra-1,3,5(10),16-tetraen-3-carbonsäure wurden mit 85 mg (2.0 Äquiv.) Ethyl-β-alaninat Hydrochlorid analog zu Beispiel 1 (zur Durchführung der Stufe B wurde 18 h bei 50°C gerührt) zu 63 mg (50% d. Th.) der Titelverbindung umgesetzt.
$C_{26}H_{29}N_3O_3$ (431.5). MS-ES+ gefundene Masse: 431.22.
$^1$H-NMR (300MHz, DMSO-$d_6$): δ [ppm]= 0.99 (s, 3H), 1.36 - 1.79 (m, 5H), 1.84 - 1.97 (m, 1H), 2.06 - 2.20 (m, 2H), 2.25 - 2.41 (m, 4H), 2.82 - 2.93 (m, 2H), 3.35 - 3.45 (m, 2H), 6.28 - 6.33 (m, 1H), 7.31 (d, 1H), 7.50 - 7.58 (m, 2H), 8.38 (t, 1H), 8.83 (s, 2H), 9.04 (s, 1H), 12.19 (br. s., 1H).

**Beispiel 18**

**4-({[17-(Pyrimidin-5-yl)estra-1,3,5(10),16-tetraen-3-yl]carbonyl}amino)butansäure**

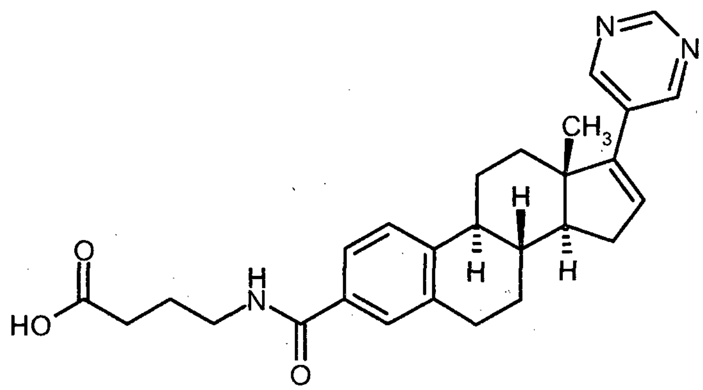

100 mg (0.28 mmol) 17-(Pyrimidin-5-yl)estra-1,3,5(10),16-tetraen-3-carbonsäure wurden mit 85 mg (2.0 Äquiv.) Methyl-4-aminobutanoat Hydrochlorid analog zu Beispiel 1 (zur Durchführung der Stufe B wurde 18 h bei 50°C gerührt)

zu 61 mg (47% d. Th.) der Titelverbindung umgesetzt.
$C_{27}H_{31}N_3O_3$ (445.6). MS-ES+ gefundene Masse: 445.24.
$^1$H-NMR (300MHz, DMSO-$d_6$): δ [ppm]= 0.99 (s, 3H), 1.39 - 1.79 (m, 7H), 1.85 - 1.98 (m, 1H), 2.05 - 2.26 (m, 4H), 2.26 - 2.41 (m, 3H), 2.81 - 2.95 (m, 2H), 3.15 - 3.25 (m, 2H), 6.27 - 6.34 (m, 1H), 7.31 (d, 1H), 7.50 - 7.61 (m, 2H), 8.34 (t, 1H), 8.83 (s, 2H), 9.04 (s, 1H), 12.04 (br. s., 1H).

**Beispiel 19**

***N*-Methyl-*N*-{[17-(pyrimidin-5-yl)estra-1,3,5(10),16-tetraen-3-yl]carbonyl}-β-alanin**

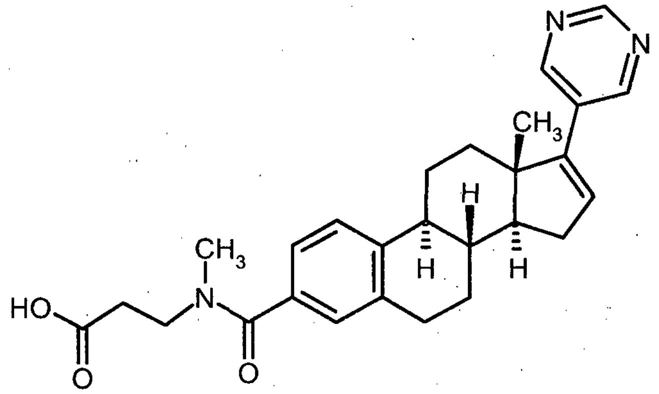

Eine Mischung aus 100 mg (0.26 mmol) 17-(Pyrimidin-5-yl)estra-1,3,5(10),16-tetraen-3-carbonsäure und 88 mg (2 Äquiv.) tert-Butyl-*N*-methyl-β-alaninat in 3 ml THF und 1 ml DMF wurde mit 42 mg (1 Äquiv.) 1-Hydroxy-1*H*-benzotriazol Hydrat, 106 mg (2.0 Äquiv.) 1-[3-(Dimethylamino)propyl]-3-ethylcarbodiimid Hydrochlorid und 0.12 ml Triethylamin versetzt und 72 h bei RT gerührt. Man verdünnte mit Wasser, extrahierte dreimal mit Ethylacetat und engte ein. Der Rückstand wurde mit 3 ml Dichlormethan und 1 ml Trifluoressigsäure versetzt und die Mischung 72 h bei Raumtemperatur gerührt. Man engte ein und nach Reinigung des Rückstandes durch präparative HPLC wurden 56 mg der Titelverbindung erhalten.
$C_{27}H_{31}N_3O_3$ (445.6). MS-ES+ gefundene Masse: 445.24.
$^1$H-NMR (300MHz, DMSO-$d_6$): δ [ppm]= 1.00 (s, 3H), 1.36 - 1.77 (m, 5H), 1.85 - 1.94 (m, 1H), 2.07 - 2.20 (m, 2H), 2.26 - 2.5 (m, verdeckt), 2.80 - 2.92 (m, 5H), 3.42 (br. s., 1H), 3.57 (br. s., 1H), 6.27 - 6.33 (m, 1H), 7.02 - 7.11 (m, 2H), 7.29 (d, 1H), 8.83 (s, 2H), 9.04 (s, 1H), 12.3 (br. s, 1H).

**Beispiel 20**

**2,2-Dimethyl-3-({[17-(pyrimidin-5-yl)estra-1,3,5(10),16-tetraen-3-yl]carbonyl}amino)-propansäure**

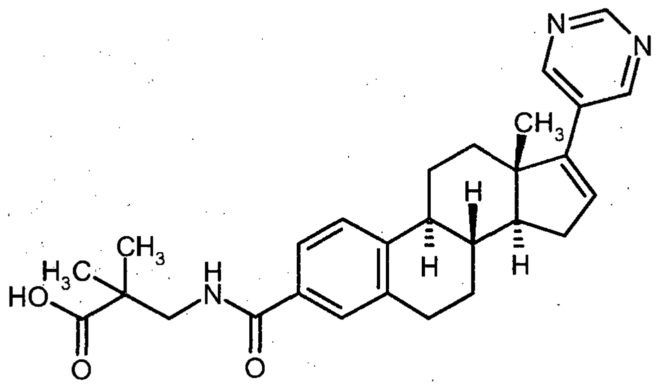

100 mg (0.28 mmol) 17-(Pyrimidin-5-yl)estra-1,3,5(10),16-tetraen-3-carbonsäure wurden mit 81 mg (0.55 mmol) Ethyl-3-amino-2,2-dimethylpropanoat analog zu Beispiel 1 (zur Durchführung der Stufe B wurde 5 h bei 50°C gerührt) zu 10 mg (8% d. Th.) der Titelverbindung umgesetzt.
$C_{28}H_{33}N_3O_3$ (459.6). MS-ES+ gefundene Masse: 459.25.
$^1$H-NMR (600MHz, DMSO-$d_6$): δ [ppm]= 1.02 (s, 3H), 1.09 (s, 6H), 1.42 - 1.50 (m, 1H), 1.53 - 1.68 (m, 3H), 1.77 (td, 1H), 1.91 - 1.97 (m, 1H), 2.13 - 2.20 (m, 2H), 2.31 - 2.39 (m, 2H), 2.41 - 2.47 (m, 1H), 2.89 - 2.94 (m, 2H), 3.40 (d, 2H), 6.33 (dd, 1H), 7.34 (d, 1H), 7.53 - 7.59 (m, 2H), 8.16- 8.21 (m., 1H), 8.85 (s, 2H), 9.07 (s, 1H), 12.25 (br. s., 1H).

**Beispiel 21**

***N*-({17-[5-(Trifluormethyl)pyridin-3-yl]estra-1,3,5(10),16-tetraen-3-yl}carbonyl)-β-alanin**

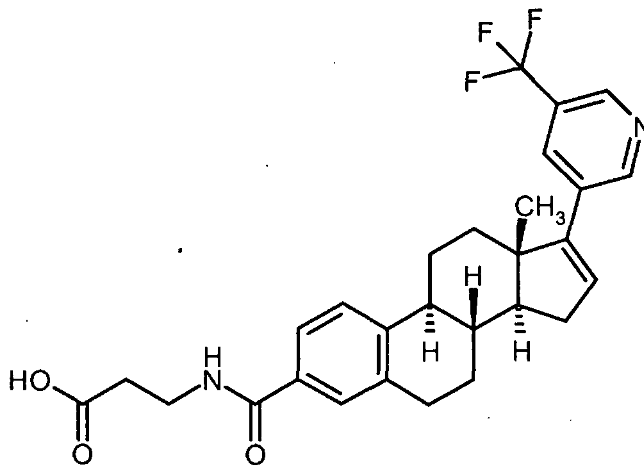

100 mg (0.23 mmol) 17-[5-(Trifluormethyl)pyridin-3-yl]estra-1,3,5(10),16-tetraen-3-carbonsäure wurden mit 72 mg (2.0 Äquiv.) Ethyl-β-alaninat Hydrochlorid analog zu Beispiel 1 (zur Durchführung der Stufe B wurde 18 h bei 50°C gerührt) zu 65 mg (56% d. Th.) der Titelverbindung umgesetzt.
$C_{28}H_{29}F_3N_2O_3$ (498.6). MS-ES+ gefundene Masse: 498.21.
$^1$H-NMR (300MHz, DMSO-$d_6$): δ [ppm]= 1.01 (s, 3H), 1.35 - 1.69 (m, 4H), 1.76 (td, 1H), 1.84 - 2.00 (m, 1H), 2.03 - 2.21 (m, 2H), 2.24 - 2.41 (m), 2.78 - 2.96 (m, 2H), 3.35 - 3.53 (m, 2H), 6.33 - 6.38 (m, 1H), 7.31 (d, 1H), 7.47 - 7.62 (m, 2H), 8.03 (s, 1H), 8.35 (t, 1H), 8.78 - 8.86 (m, 1H), 8.86 - 8.97 (m, 1H), 12.2 (br. s., 1H).

**Beispiel 22**

***N*-Methyl-*N*-({17-[5-(trifluormethyl)pyridin-3-yl]estra-1,3,5(10),16-tetraen-3-yl}carbonyl)-β-alanin**

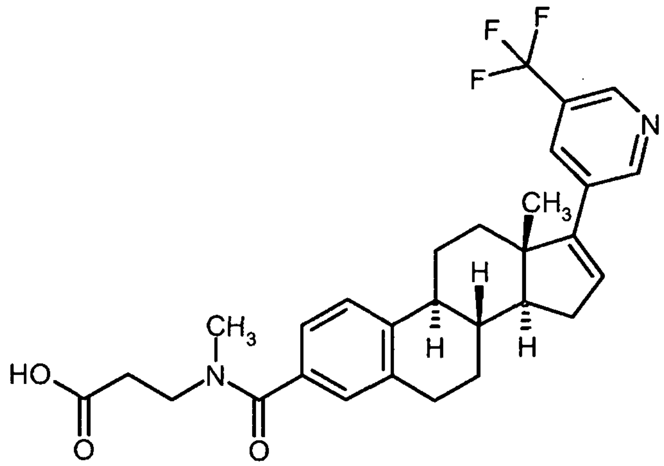

Stufe A: Eine Mischung aus 100 mg (0.23 mmol) 17-[5-(Trifluormethyl)pyridin-3-yl]estra-1,3,5(10),16-tetraen-3-carbonsäure und 74 mg (2 Äquiv.) *tert*-Butyl-*N*-methyl-β-alaninat in 3 ml THF wurde mit 36 mg (1 Äquiv.) 1-Hydroxy-1*H*-benzotriazol Hydrat, 90 mg (2.0 Äquiv.) 1-[3-(Dimethylamino)propyl]-3-ethylcarbodiimid Hydrochlorid und 98 Microliter Triethylamin versetzt und 4 h bei RT gerührt. Man verdünnte mit Wasser, extrahierte dreimal mit Ethylacetat und engte ein.

Stufe B: Man versetzte mit 2 ml Dichlormethan und 180 Microliter Trifluoressigsäure und ließ bei 40°C Badtemperatur 18 h rühren. Man addierte weitere 90 Microliter Trifluoressigsäure und rührte 5 h bei 40°C. Wasser wurde zugesetzt, die Phasen wurden getrennt und die wässrige Phase zweimal mit Dichlormethan extrahiert. Man engte ein und reinigte den Rückstand durch HPLC (Acetonitril/Wasser/Ameisensäure). Man erhielt 83 mg (69% d. Th.) der Titelverbindung.

$C_{29}H_{31}F_3N_2O_3$ (512.58). MS-ES+ gefundene Masse: 512.23.

$^1$H-NMR (300MHz, DMSO-$d_6$): δ [ppm]= 1.01 (s, 3H), 1.36 - 1.51 (m, 1H), 1.52 - 1.81 (m, 4H), 1.84 - 1.98 (m, 1H), 2.04 - 2.21 (m, 2H), 2.25 - 2.40 (m), 2.79 - 2.96 (m, 5H), 3.42 (br. s.), 3.55 (br. s.), 6.35 (s, 1H), 7.00 - 7.15 (m, 2H), 7.28 (d, 1H), 8.03 (s, 1H), 8.81 - 8.87 (m, 1H), 8.87 - 8.95 (m, 1H), 12.3 (br. s., 1H).

**Beispiel 23**

***N*-{[17-(5-Fluorpyridin-3-yl)estra-1,3,5(10),16-tetraen-3-yl]carbonyl}-L-prolin**

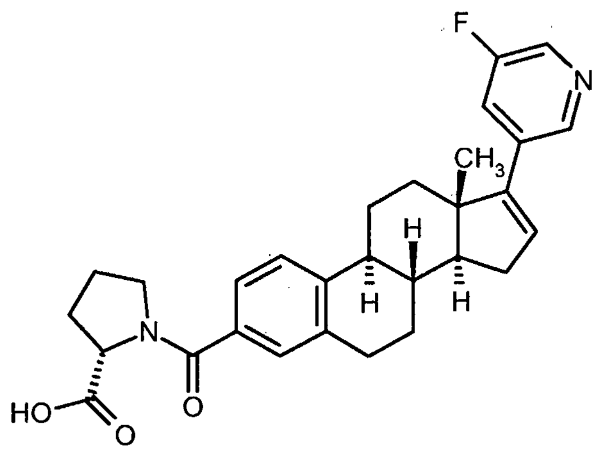

100 mg (0.26 mmol) 17-(5-Fluorpyridin-3-yl)estra-1,3,5(10),16-tetraen-3-carbonsäure wurden mit 91 mg (2.0 Äquiv.) *tert*-Butyl-L-prolinat analog zu Beispiel 22 umgesetzt. Nach Reinigung durch präparative HPLC wurden 65 mg (50% d. Th.) der Titelverbindung erhalten. $C_{29}H_{31}FN_2O_3$ (474.6). MS-ES+ gefundene Masse: 474.23.

$^1$H-NMR (300MHz, DMSO-$d_6$): δ [ppm]= 1.00 (s, 3H), 1.39 - 1.94 (m), 2.05 - 2.43 (m), 2.77 - 2.94 (m, 2H), 3.40 - 3.59 (m, 2H), 4.27 - 4.40 (m, 1H), 6.27 (s., 1H), 7.04 - 7.34 (m, 3H), 7.64 - 7.73 (m, 1 H), 8.43 (d, 1 H), 8.49 (s, 1 H), 12.5

(br. s., 1 H).

Die Titelverbindung wurde durch analytische HPLC analysiert:

| | |
|---|---|
| *System:* | Waters: Alliance 2695, DAD 996 |
| *Säule* | Chiralpak AS-RH 5µm 150x4.6 mm |
| *Solvent:* | $H_2O$ (0.1% Vol. Ameisensäure) / Acetonitril 50:50 (v/v) |
| *Fluss:* | 1.0 ml/min |
| *Temperatur:* | 25°C |
| *Lösung* | 1.0 mg/ml Ethanol / Methanol 2:1 |
| *Injektion:* | 5.0 µl |
| *Detektion:* | DAD 254 nm |
| Peak | Rt in min |
| **1** | 8.15 |

**Beispiel 24**

***N*-{[17-(5-Fluorpyridin-3-yl)estra-1,3,5(10),16-tetraen-3-yl]carbonyl}-D-prolin**

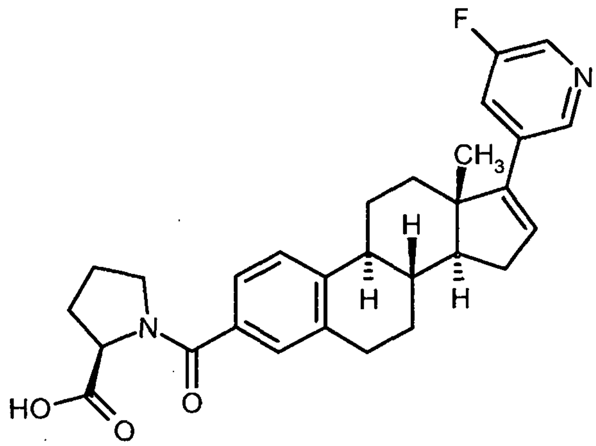

100 mg (0.26 mmol) 17-(5-Fluorpyridin-3-yl)estra-1,3,5(10),16-tetraen-3-carbonsäure wurden mit 91 mg (2.0 Äquiv.) *tert*-Butyl-D-prolinat analog zu Beispiel 22 umgesetzt. Nach Reinigung durch präparative HPLC wurden 66 mg (52% d. Th.) der Titelverbindung erhalten. $C_{29}H_{31}FN_2O_3$ (474.6). MS-ES+ gefundene Masse: 474.23.
$^1$H-NMR (300MHz, DMSO-$d_6$): $\delta$ [ppm]= 1.00 (s, 3H), 1.34 - 1.97 (m), 2.05 - 2.43 (m), 2.75 - 2.94 (m, 2H), 3.42 - 3.59 (m, 2H), 4.25 - 4.40 (m, 1H), 6.27 (s., 1H), 7.02 - 7.36 (m, 3H), 7.68 (d, 1H), 8.43 (d, 1H), 8.49 (s, 1H), 12.5 (br. s., 1H).

Die Titelverbindung wurde durch analytische HPLC analysiert:

| | |
|---|---|
| *System:* | Waters: Alliance 2695, DAD 996 |
| *Säule:* | Chiralpak AS-RH 5µm 150x4.6 mm |
| *Solvent:* | $H_2O$ (0.1% Vol. Ameisensäure) / Acetonitril 50:50 (v/v) |
| *Fluss:* | 1.0 ml/min |
| *Temperatur:* | 25°C |
| *Lösung:* | 1.0 mg/ml Ethanol / Methanol 2:1 |
| *Injektion:* | 5.0 µl |

(fortgesetzt)

| Detektion: | DAD 254 nm |
|---|---|
| Peak | Rt in min |
| **2** | 9.50 |

**Beispiel 25**

**4-({[11β-Fluor-17-(5-fluorpyridin-3-yl)estra-1,3,5(10),16-tetraen-3-yl]carbonyl}amino) butansäure**

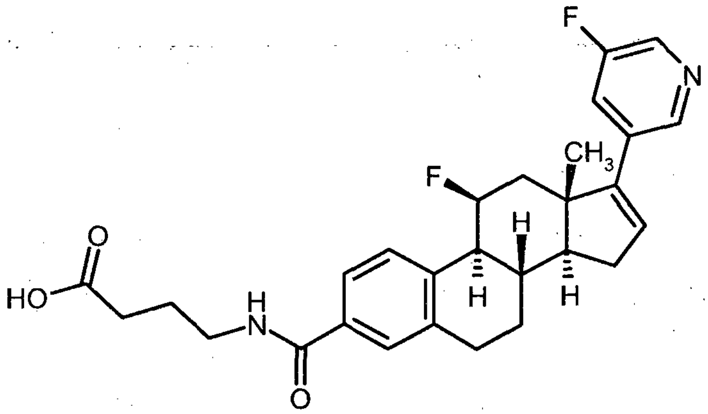

100 mg 11β-Fluor-17-(5-fluorpyridin-3-yl)estra-1,3,5(10),16-tetraen-3-carbonsäure wurden analog zu Beispiel 1 mit 78 mg (2.0 Äquiv.) Methyl-4-aminobutanoat Hydrochlorid zu 80 mg (66% d. Th.) der Titelverbindung umgesetzt. $C_{28}H_{30}F_2N_2O_3$ (480.6). MS-ES+ gefundene Masse: 480.22.
$^1$H-NMR (400MHz, DMSO-$d_6$): δ [ppm]= 1.14 (s, 3H), 1.40 - 1.55 (m, 1H), 1.70 (quin, 2H), 1.74 - 2.03 (m, 4H), 2.13 - 2.27 (m, 3H), 2.27 - 2.37 (m, 1H), 2.49 - 2.60 (m, 1H), 2.60 - 2.77 (m, 1H), 2.81 - 2.97 (m, 2H), 3.22 (q, 2H), 5.58 - 5.80 (m, 1H), 6.21 - 6.34 (m, 1H), 7.40 (d, 1H), 7.48 - 7.61 (m, 2H), 7.72 (dt, 1H), 8.36 (t, 1H), 8.45 (d, 1H), 8.50 (s, 1H), 12.0 (br. s., 1H).

**Beispiel 26**

***N*-{[17-(5-Fluorpyridin-3-yl)-15α-hydroxyestra-1,3,5(10),16-tetraen-3-yl]carbonyl}-*N*-methyl-β-alanin**

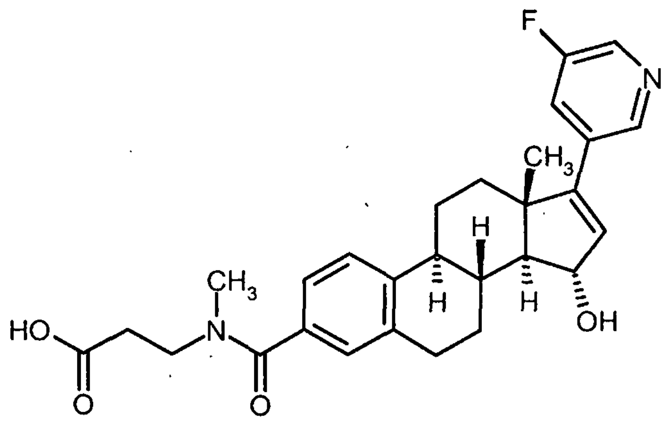

Ein 100-ml-Erlenmeyerkolben, der 20 ml einer 20 Minuten bei 121°C im Autoklaven sterilisierten wässrige Nährlösung aus 1% Maisquellwasser und 1% Sojabohnenmehl (auf pH 6.2 eingestellt) enthielt, wurde mit 0,2 ml DMSO-Eiskultur des Stammes *Calonectria decora* (ATCC No. 14767) beimpft und 48 Stunden bei 21°C auf einem Rotationsschüttler mit 165 Umdrehungen pro Minute geschüttelt. Mit 8 ml dieser Vorzucht wurde ein 500-ml-Erlenmeyerkolben beimpft, der mit 100 ml sterilem Medium aus der gleichen Endzusammensetzung, wie für die Vorkultur beschrieben, beschickt worden war. Dieser Kolben wurde für 48 Stunden bei 21 °C auf einem Rotationsschüttler mit 165 Umdrehungen pro Minute geschüttelt. Mit je 50 ml dieser Vorzucht wurden zwei 2-L Erlenmeyerkolben, die 1-L einer sterilen Nährlösung aus 3% Glucose-Monohydrat, 1% Ammoniumchlorid, 0.2% Natriumnitrat, 0.1% Kaliumdihydrogenphosphat, 0.2% Dikaliumhydrogenphosphat, 0.05% Kaliumchlorid, 0.05% Magnesiumsulfat Heptahydrat und 0.002% Eisen(II)sulfat Heptahydrat enthielten, beimpft. Nach einer Anwachsphase von 6 Stunden bei 27 °C auf einem Rotationsschüttler mit 165 Umdrehungen bei einer Temperatur von 27°C wurde eine Lösung aus 50 mg *N*-{[17-(5-Fluorpyridin-3-yl)estra-1,3,5(10),16-tetraen-3-yl]carbonyl}-*N*-methyl-β-alanin in 10 ml DMF auf beide Kolben verteilt. Die Kolben wurden weitere 43 Stunden geschüttelt und dann aufgearbeitet. Die beiden Kulturbrühen wurden vereinigt und mit 1 1 Isobutylmethylketon 19 Stunden bei 40 Umdrehungen pro Minute in einem 5 1 Glasaüsrührgefäß extrahiert. Die organische Phase wurde über Natriumsulfat getrocknet und bis zur Trockene eingeengt. Der Rückstand wurde mit Methanol gewaschen, um das Siliconöl abzutrennen. Es wurden 328 mg eines Rohproduktes erhalten. Das Rohprodukt wurde auf Diatominerde aufgezogen und chromatographiert: Methode: Biotage Isolera, 10 g SNAP Säule, Lösungsmittel: Gradient von 2 bis 20% Methanol in Ethylacetat (mit 1% Eisessig versetzt). Es wurden 42 mg der Zielverbindung erhalten.
HPLC Rt = 4.8 min
HPLC-Bedingungen: A: Wasser mit 0.05% Ameisensäure; B: Acetonitril mit 0.1% Ameisensäure; Gradient: 0 min: 60:40 A/B; 12 min: 30:70 A/B; Fluß: 0.8 ml/min; Säule: Luna C18 (2) 5μ 125x4,6; Detektions-Wellenlänge: 244 nm
$^1$H-NMR (400 MHz, DMSO-d6): δ [ppm]= 1.08 (s, 3 H), 1.50 - 1.58 (m, 2 H), 1.61 - 1.67 (m, 2 H), 1.76 - 1.85 (m, 1 H), 2.08 - 2.12 (m, 1 H), 2.30 - 2.35 (m, 2 H), 2.40 - 2.45 (m, 1 H), 2.55 (2H überlagert durch DMSO-Signal), 2.85 - 2.89 (m, 2 H), 2.91 (s, 3 H), 3.45 (br. s, 1 H), 3.62 (br. s, 1 H), 4.62 (d, 1 H), 4.95 (br. s, 1 H), 6.15 (s, 1 H), 7.05 (s, 1 H), 7.11 (d, 1 H), 7.31 (d, 1 H), 7.71 (d, 1 H), 8.49 (d, 1 H), 8.51 (s, 1 H), 12.1 (br. s, 1 H).

**Beispiel 27**

***N*-{[17-(5-Fluorpyridin-3-yl)-15β-hydroxyestra-1,3,5(10),16-tetraen-3-yl]carbonyl}-*N*-methyl-β-alanin:**

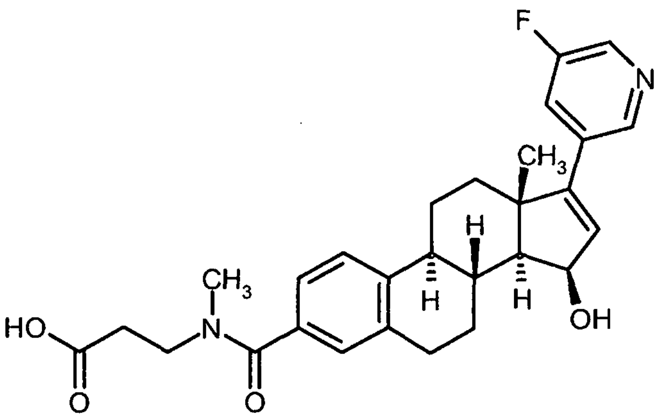

Ein 100-ml-Erlenmeyerkolben, der 20 ml einer 20 Minuten bei 121 °C im Autoklaven sterilisierten wässrigen Nährlösung aus 3% Glucose-Monohydrat, 1% Maisquellwasser, 0.2% Natriumnitrat, 0.1% Kaliumdihydrogenphosphat, 0.2% Dikaliumhydrogenphosphat, 0.05% Kaliumchlorid, 0.05% Magnesiumsulfat Heptahydrat und 0.002% Eisen(II)sulfat Heptahydrat (auf pH 6.0 eingestellt) enthielt, wurde mit 0.2 ml DMSO-Eiskultur des Stammes *Mucor plumbeus* (CBS No. 29563) beimpft und 65 Stunden bei 27 °C auf einem Rotationsschüttler mit 165 Umdrehungen pro Minute geschüttelt. Mit 8 ml dieser Vorzucht wurde ein 500-ml-Erlenmeyerkolben beimpft, der mit 100 ml sterilem Medium aus der gleichen Endzusammensetzung, wie für die Vorkultur beschrieben, beschickt worden war. Dieser Kolben wurde für 72 Stunden bei 27 °C auf einem Rotationsschüttler mit 165 Umdrehungen pro Minute geschüttelt. Mit je 50 ml dieser Vorzucht wurden zwei 2-L Erlenmeyerkolben, die 1-L einer sterilen Nährlösung aus 3% Glucose-Monohydrat, 1% Ammoniumchlorid, 0.2% Natriumnitrat, 0.1% Kaliumdihydrogenphosphat, 0.2% Dikaliumhydrogenphosphat, 0.05% Kaliumchlorid, 0.05% Magnesiumsulfat Heptahydrat und 0.002% Eisen(II)sulfat Heptahydrat enthielten, beimpft. Nach einer Anwachsphase

von 6 Stunden bei 27 °C auf einem Rotationsschüttler mit 165 Umdrehungen bei einer Temperatur von 27 °C wurde eine Lösung aus 50 mg *N*-{[17-(5-Fluorpyridin-3-yl)estra-1,3,5(10),16-tetraen-3-yl]carbonyl}-*N*-methyl-β-alanin in 10 ml DMF auf beide Kolben verteilt. Die Kolben wurden weitere 43 Stunden geschüttelt und dann aufgearbeitet. Die beiden Kulturbrühen wurden vereinigt und mit 1 Liter Isobutylmethylketon 19 Stunden bei 40 Umdrehungen pro Minute in einem 5 Liter Glasausrührgefäß extrahiert. Die organische Phase wurde über Natriumsulfat getrocknet und bis zur Trockene eingeengt. Der Rückstand wurde mit Methanol gewaschen, um das Siliconöl abzutrennen. Es wurden 236 mg Rohprodukt als braunes Öl erhalten. Das Rohprodukt wurde auf Diatominerde aufgezogen und chromatographiert: Gerät Biotage Isolera; 10 g SNAP Säule; Lösungsmittel: Gradient von 2 bis 20% Methanol in Ethylacetat (mit 1% Eisessig versetzt). Es wurden 35 mg der Zielverbindung erhalten. HPLC Rt = 5.4 min

HPLC-Bedingungen: A: Wasser mit 0.05% Ameisensäure; B: Acetonitril mit 0.1% Ameisensäure; Gradient: 0 min: 60:40 A/B; 12 min: 30:70 A/B; Fluß: 0.8 ml/min; Säule: Luna C18 (2) 5μ 125x4,6;

Detektions-Wellenlänge: 244 nm

$^1$H-NMR (400 MHz, DMSO-d6): δ [ppm]= 1.28 (s, 3 H); 1.38 - 1.60 (m, 4 H); 1.69 - 1.78 (m, 1 H); 2.01 - 2.08 (m, 1 H); 2.20 - 2.28 (m, 1 H); 2.30 - 2.40 (m, 1 H); 2.55 (2H überlagert durch DMSO-Signal); 2.85 - 2.90 (m, 5 H), 3.10 (s, 1 H); 3.45 (br. s, 1 H); 3.57 (br. s, 1 H); 4.50 (s, 1 H); 4.69 (br. s, 1 H); 6.30 (s, 1 H); 7.05 (s, 1 H); 7.08 (d, 1 H); 7.28 (d, 1 H); 7.71 (d7, 1 H); 8.47 (d, 1 H); 8.52 (s, 1 H); 12.1 (br. s, 1 H).

**Beispiel 28**

**N-Methyl-N-{[17-(6-methylpyridazin-4-yl)estra-1,3,5(10),16-tetraen-3-yl]carbonyl}-β-alanin**

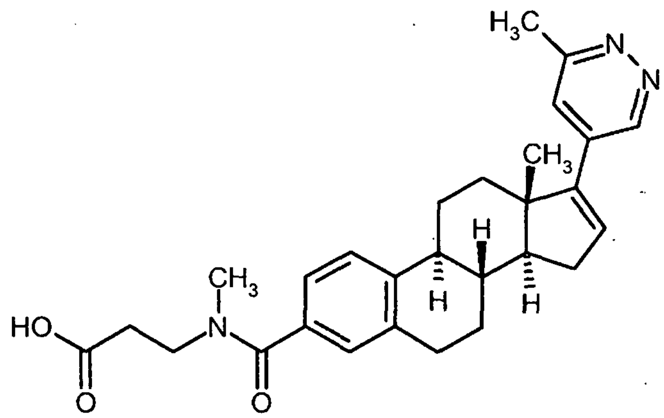

42 mg 17-(6-Methylpyridazin-4-yl)estra-1,3,5(10),16-tetraen-3-carbonsäure (verunreinigt) und 36 mg *tert*-Butyl-*N*-methyl-β-alaninat (2 Äquiv.) wurden in 2.5 ml THF und 0.5 ml DMF gelöst. Es wurden 43 mg 1-[3-(Dimethylamino)propyl]-3-ethylcarbodiimid Hydrochlorid (EDC), 17 mg 1-Hydroxy-1*H*-benzotriazol Hydrat und 0.047 ml Triethylamin zugegeben und 18 h bei Raumtemperatur gerührt. Das Reaktionsgemisch wurde mit Wasser verdünnt und dreimal mit Ethylacetat extrahiert. Die vereinigten organischen Phasen wurden eingeengt und der Rückstand mit 2 ml Dichlormethan und 0.5 ml Trifluoressigsäure versetzt und bei Raumtemperatur 6 h gerührt. Man engte ein und reinigte durch präparative HPLC. Man erhielt 18 mg der Titelverbindung.

$C_{28}H_{33}N_3O_3$ (459.59). MS-ES+ gefundene Masse: 459.25.

$^1$H-NMR (400 MHz, DMSO-d6): δ [ppm]= 1.02 (s, 3H), 1.35 - 1.77 (m, 5H), 1.84 - 1.95 (m, 1H), 2.07 - 2.42 (m, 6H), 2.58 (s, 3H), 2.78 - 2.95 (m, 5H), 3.41 (br. s), 3.57 (br. s), 6.54 - 6.59 (m, 1H), 7.02 - 7.13 (m, 2H), 7.29 (d, 1 H), 7.49 (d, 1H), 9.10 (d, 1H), 12.3 (br. s, 1H).

**Beispiel 29**

**N-Methyl-N-{[17-(pyridin-3-yl)estra-1,3,5(10),16-tetraen-3-yl]carbonyl}-β-alanin**

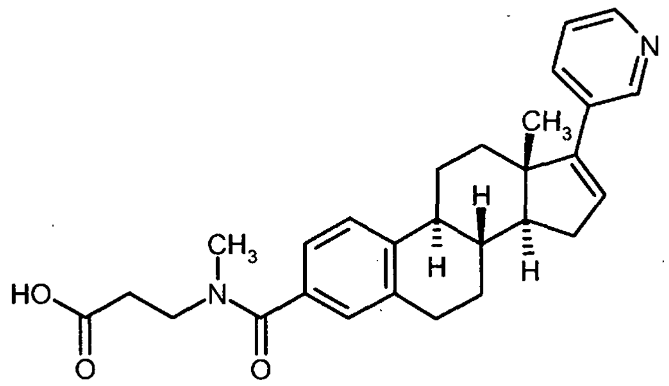

100 mg 17-(Pyridin-3-yl)estra-1,3,5(10),16-tetraen-3-carbonsäure und 89 mg *tert*-Butyl-*N*-methyl-β-alaninat (2 Äquiv.) wurden in 3 ml THF und 0.5 ml DMF gelöst. Es wurden 107 mg 1-[3-(Dimethylamino)propyl]-3-ethylcarbodiimid Hydrochlorid (EDC), 43 mg 1-Hydroxy-1*H*-benzotriazol Hydrat und 0.116 ml Triethylamin zugegeben und 18 h bei Raumtemperatur gerührt. Das Reaktionsgemisch wurde mit Wasser verdünnt und dreimal mit Ethylacetat extrahiert. Die vereinigten organischen Phasen wurden eingeengt und der Rückstand mit 3 ml Dichlormethan und 1 ml Trifluoressigsäure versetzt und bei Raumtemperatur 20 h gerührt. Man engte ein und reinigte durch präparative HPLC. Man erhielt 78 mg der Titelverbindung.
$C_{28}H_{32}N_2O_3$ (444.58). MS-ES+ gefundene Masse: 444.24.
$^1$H-NMR (400MHz, DMSO-$d_6$): δ [ppm]= 0.99 (s, 3H), 1.37 - 1.67 (m, 4H), 1.73 (td, 1H), 1.86 - 1.94 (m, 1H), 2.06 - 2.17 (m, 2H), 2.24 - 2.45 (m, 3H), 2.80 - 2.93 (m, 5H), 3.42 (br. s.), 3.57 (br. s.), 6.12 (dd, 1H), 7.02 - 7.11 (m, 2H), 7.26 - 7.35 (m, 2H), 7.77 (dt, 1H), 8.42 (dd, 1H), 8.59 (d, 1H), 12-3 (br. s., 1H).

**Beispiel 30**

**4-({[17-(5-Methoxypyridin-3-yl)estra-1,3,5(10),16-tetraen-3-yl]carbonyl}amino)butansäure**

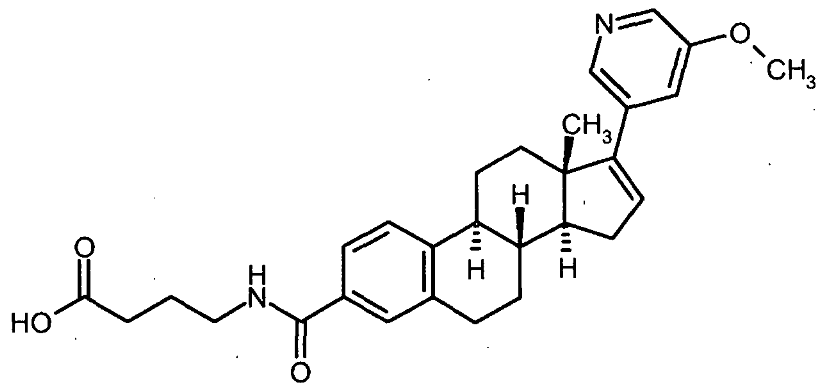

100 mg (0.26 mmol) 17-(5-Methoxypyridin-3-yl)estra-1,3,5(10),16-tetraen-3-carbonsäure wurden mit 79 mg Methyl-4-aminobutanoat Hydrochlorid analog zu Beispiel 1 zu 64 mg (53% d. Th.) der Titelverbindung umgesetzt.
$C_{29}H_{34}N_2O_4$ (474.61). MS-ES+ gefundene Masse: 474.25.
$^1$H-NMR (300MHz, DMSO-$d_6$): δ [ppm]= 0.99 (s, 3H), 1.38 - 1.78 (m, 7H), 1.86 - 1.96 (m, 1H), 2.04 - 2.43 (m, 7H), 2.83 - 2.92 (m, 2H), 3.15 - 3.26 (m, 2H), 3.81 (s, 3H), 6.16 (s, 1H), 7.23 - 7.34 (m, 2H), 7.51 - 7.59 (m, 2H), 8.16 (d, 1H), 8.20 (d, 1H), 8.33 (t, 1H), 12.0 (br. s., 1H).

<u>**Pharmakologische Untersuchung der erfindungsgemäßen Verbindungen *in vitro***</u>

**Beispiel 31 (AKR1C3-inhibitorische Wirkung)**

Die AKR1C3-inhibitorische Wirkung der Substanzen dieser Erfindung wurde in dem in den folgenden Absätzen

beschriebenen AKR1C3-Assay gemessen.

Im Wesentlichen wird die Enzymaktivität durch Quantifizierung des gebildeten Coumberols aus Coumberon gemessen (Halim, M., Yee, D.J., and Sames, D., J. AM. CHEM. SOC. 130, 14123-14128 (2008) und Yee, D.J., Balsanek, V., Bauman, D.R., Penning, T.M., and Sämes, D., Proc. Natl. Acad. Sci. USA 103, 13304 - 13309 (2006)). Bei diesem Versuch kann die Zunahme des stark fluoreszierenden Coumberols durch NADPH (Nicotinamid-Adenin-Dinukleotidphosphat)-abhängige Reduktion des nicht fluoreszierenden Coumberons durch AKR1C3 bestimmt werden.

Als Enzym wurde rekombinantes humanes AKR1C3 (Aldo-keto reductase family 1 member C3) (GenBank Accession No. NM_003739) verwendet. Dieses wurde als GST (Glutathion-S-Transferase)-Fusionsprotein in *E.coli* exprimiert und mittels Gluthation-Sepharose-Affinitätschromatographie gereinigt. Durch Thrombinverdau mit anschließender Größenausschlusschromatographie wurde das GST entfernt (Dufort, I., Rheault, P., Huang, XF., Soucy, P., and Luu-The, V.,Endocrinology 140, 568-574 (1999)).

Für den Assay wurden 50 nl einer 100-fach konzentrierten Lösung der Testsubstanz in DMSO in eine schwarze low-volume 384well-Mikrotiterplatte (Greiner Bio-One, Frickenhausen, Deutschland) pipettiert, 2,0 μl einer Lösung von AKR1C3 in Assaypuffer [50 mM Kalium-Phosphatpuffer pH 7, 1 mM DTT, 0,0022% (w/v) Pluronic F-127, 0,01% BSA (w/v) und Proteaseinhibitor-Cocktail (Complete, EDTA-free Protease Inhibitor Cocktail von Roche)] hinzugegeben und die Mischung für 15 min inkubiert, um eine Vorbindung der Substanzen an das Enzym vor der Enzymreaktion zu ermöglichen. Dann wurde die Enzymreaktion durch Zugabe von 3 μl einer Lösung von NADPH (16,7 μM → Endkonzentration in 5 μl Assayvolumen ist 10 μM) und Coumberon (0,5 μM → Endkonzentration in 5 μl Assayvolumen ist 0,3 μM) in Assaypuffer gestartet und die resultierende Mischung für die Reaktionszeit von 90 min bei 22°C inkubiert. Die Konzentration der AKR1C3 wurde an die jeweilige Aktivität des Enzym-Präparats angepasst und so eingestellt, dass der Assay im linearen Bereich arbeitete. Typische Konzentrationen lagen im Bereich von 1 nM. Die Reaktion wurde durch Zugabe von 5 μl einer Stopplösung bestehend aus dem Inhibitor EM-1404 [F. Labrie et al. US Patent 6,541,463, 2003] (2 μM → Endkonzentration in 5 μl Assayvolumen ist 1 μM) gestoppt. Anschließend wurden die Fluoreszenz des Coumberols bei 520 nm (Anregung bei 380 nm) mit einem geeigneten Messgerät (Pherastar von BMG Labtechnologies) gemessen. Die Intensität der Fluoreszenz wurde als Maß für die Menge des gebildeten Coumberols und damit für die Enzymaktivität der AKR1C3 verwendet. Die Daten wurden normalisiert (Enzymreaktion ohne Inhibitor = 0 % Inhibition; alle anderen Assaykomponenten, aber kein Enzym = 100 % Inhibition). Üblicherweise wurden die Testsubstanzen auf derselben Mikrotiterplatten bei 11 verschiedenen Konzentrationen im Bereich von 20 μM bis 96,8 pM (20 μM, 5,9 μM, 1,7 μM, 0,5 μM, 0,15 μM, 44 nM, 12,9 nM, 3,8 nM, 1,1 nM, 0,3 nM und 96,8 pM, die Verdünnungsreihen wurden vor dem Assay auf der Ebene der 100-fach konzentrierten Lösung durch serielle 1:3 Verdünnungen mit 100% DMSO hergestellt) in Doppelwerten für jede Konzentration getestet, und $IC_{50}$-Werte wurden mit einem 4-Parameter-Fit kalkuliert.

Wie beschrieben wurden die beanspruchten pharmakologischen Substanzen auf ihre inhibitorische Wirkung auf das AKR1C3 Enzym untersucht (siehe Tabelle 1). Über den Großteil des beanspruchten Strukturbereichs zeigen diese Verbindungen eine starke Inhibition von AKR1C3 in vitro ($IC_{50}$-Werte < 50 nM) und überwiegend sogar $IC_{50}$-Werte < 20 nM.

**Tabelle 1:** Inhibition von AKR1C3 der erfindungsgemäßen Verbindungen (angegeben sind für den Großteil der Verbindungen die Werte für zwei experimentelle Bestimmungen)

| Beispielverbindung | AKR1C3 Enzym Inhibition $IC_{50}$ [nmol/l]) | Beispielverbindung | AKR1C3 Enzym Inhibition $IC_{50}$ [nmol/l]) | Beispielverbindung | AKR1C3 Enzym Inhibition $IC_{50}$ [nmol/l]) |
|---|---|---|---|---|---|
| 1 | 1.4 | 9 | 8.9 | 19 | 17.8 |
| 1 | 1.9 | 10 | 7.3 | 19 | 26.7 |
| 2 | 1.3 | 10 | 4.9 | 20 | 45.5 |
| 2 | 1.4 | 11 | 2.2 | 20 | 46.3 |
| 3 | 9.2 | 11 | 1.8 | 21 | 8.5 |
| 3 | 10.2 | 12 | 1.2 | 22 | 4.2 |
| 4 | 13.9 | 12 | 1.7 | 23 | 1.9 |
| 4 | 8.9 | 13 | 0.8 | 24 | 5.3 |
| 5 | 9.1 | 13 | 1.8 | 25 | 29.1 |
| 5 | 8.0 | 14 | 9.8 | 25 | 16.8 |

(fortgesetzt)

| Beispielverbindung | AKR1C3 Enzym Inhibition $IC_{50}$ [nmol/l]) | Beispielverbindung | AKR1C3 Enzym Inhibition $IC_{50}$ [nmol/l]) | Beispielverbindung | AKR1C3 Enzym Inhibition $IC_{50}$ [nmol/l]) |
|---|---|---|---|---|---|
| 5 | 4.3 | 14 | 17.9 | 26 | 11.0 |
| 5 | 3.6 | 15 | 6.9 | 26 | 16.6 |
| 6 | 21.1 | 15 | 7.2 | 27 | 35.3 |
| 6 | 21.0 | 16 | 4.4 | 27 | 24.2 |
| 7 | 8.5 | 16 | 6.9 | 28 | 0.5 |
| 7 | 9.6 | 17 | 29.7 | 29 | 2.5 |
| 8 | 8.5 | 17 | 37.6 | 30 | 7.3 |
| 8 | 6.8 | 18 | 23.5 | 30 | 11.2 |
| 9 | 8.3 | 18 | 31.8 | | |
| 9 | 8.9 | 19 | 26.7 | | |

**Beispiel 32 (Inhibition von Cyp17A1)**

CYP17A1 (Synonym 17α-Hydroxylase/17,20 Lyase) ist ein Enzym, das an Pregnenolon und an Progesteron eine Hydroxyl-Gruppe an Position 17 des steroidalen D-Rings addiert, wodurch 17α-Hydroxyprogesteron und 17α-Hydroxypregnenolon gebildet wird. Nachfolgend wird Dehydroepiandrosteron und Androstendion gebildet. Der bekannte CYP17A1 Inhibitor *Abirateron* wird zum Beispiel für die Therapie von metastasierten, kastrationsrefraktären Prostatakarzinom nach Versagen einer Docetaxel-basierten Chemotherapie eingesetzt (Urologe 2010, 49, 64-68). Abirateron blockiert die Androgensynthese und Estrogensynthese im gesamten Körper und senkt demnach die Hormonprduktion nicht gewebespezifisch, was zu unerwünschten Nebenwirkungen führt (vgl. Pressemitteilung der *FDA, U.S. Food and Drug Admistration* vom 28. April 2011).

Überaschenderweise wurde gefunden, dass die erfindungsgemäßen Verbindungen, obwohl sie einen aromatischen stickstoffhaltigen Heterocyclus an Position 17 des steroidalen Gerüstes zeigen, CYP17A1 nicht oder nur sehr schwach inhibieren.

Assaybeschreibung:

Die Inhibierung der CYP17A1 durch die Testverbindungen wurde mittels rekombinantem Enzym evaluiert. Humane CYP17A1 wurde in E. coli exprimiert (Ehmer, P. B. et al.; J. Steroid Biochem. Mol. Biol., 75, 57-63 (2000)). Die Microsomale Fraktion und 140 μL Phosphatpuffer (50 mM Na-Phosphat, lmM $MgCl_2$, 0.1 mM EDTA, 0.1 mM Dithiothreitol, pH 7.4) mit einer Mischung aus Progesteron (24.95 μM) und $^3$H-Progesterone (0.05 μM, 101.3 Ci/mmol), 50 μM eines NADPH Regenerationssystems (in Phosphatpuffer mit 10 mM NADP+, 100 mM Glukose-6-Phosphat und 2.5 U Glukose-6-Phosphat Dehydrogenase) und den entsprechenden Testsubstanzen (in 5 μL DMSO) wurden einzeln bei 37°C für 5 Minuten präinkubiert. Die Reaktion wurde durch Zugabe des Enzyms gestartet und nach 30 Minuten Inkubation bei 37°C durch Zugabe von 50 μL 1 N Salzsäure gestoppt.

Die Steroide wurden mit Ethylacetat extrahiert. Nach Verdampfen der organischen Phase wurden die Steroide in Acetonitril aufgenommen. 16α-Hydroxyprogesteron, 17α-Hydroxyprogesteron und Progesteron wurden mit Acetonitril/Wasser (45:55) als mobile Phase auf einer C18 *reverse phase* Chromatographiesäule (Nucleodur C18 Gravity, 3 μm, Macherey-Nagel, Düren) auf einem HPLC System (Agilent 1100 Series, Agilent Technologies, Waldbronn) getrennt. Detektion und Quantifizierung der Steroide wurde mittels eines Radioflow Detektors (Berthold Technologies, Bad Wildbad) durchgeführt. Die Inhibition wurde nach folgender Formel berechnet:

$$\%Inhibition = \frac{\%(17\alpha - Hydroxyprogesteron + 16\alpha - Hydroxyprogesteron)}{\%(17\alpha - Hydroxyprogesteron + 16\alpha - Hydroxyprogesteron) + Progesteronsteron} \cdot 100$$

Jeder Wert wurde aus mindestens drei unabhängigen Experimenten berechnet. Der finale $IC_{50}$ Wert wurde

als Mittelwert aus 3 oder 4 unabhängigen $1C_{50}$ Werten berechnet.

Die erfindungsgemäßen Verbindungen zeigen keine oder nur eine sehr schwach ausgeprägte Inhibition von Cyp17A1 (Tabelle 2) mit $1C_{50}$-Werten größer als 10 μM im Vergleich zu dem bekannten Cyp17A1 Inhibitor Abirateron (als freie Base eingesetzt).

**Tabelle 2:** Inhibierung von *humaner* CYP17

| **Beispielverbindung** | **$IC_{50}$ ± SD (μM)** |
|---|---|
| | **CYP17** |
| Abirateron | 0.029 ± 0.004 |
| 5 | 85.5 ± 7.7 |
| 2 | ~ 200 (49%)[a] |
| 9 | Keine Inhibition |
| 10 | 15.19 ± 1.58 |
| [a]% Inhibierung bei einer Konzentration von 200 μM Substanz | |

**Beispiel 33 (Löslichkeit in wässrigem Puffer pH 6.5):**

**Bestimmung der thermodynamischen Löslichkeit in wässrigem Puffer pH 6.5 (Shake-Flask Method)**

Die thermodynamische Löslichkeit wurde nach der Shake-Flask Methode bestimmt. [Literatur: Edward H. Kerns and Li Di (2008) Solubility Methods in: Drug-like Properties: Concepts, Structure Design and Methods, p276-286. Burlington, MA, Academic Press].

Hierbei wurde eine gesättigte Lösung des Wirkstoffs in Puffer pH 6.5 hergestellt und für 24 h gerührt, um sicher zu stellen, dass sich das Gleichgewicht zwischen dem Feststoff und dein Stoff in Lösung eingestellt hatte. Danach wurde die Lösung zentrifugiert, und die Konzentration der gewonnenen Lösung mit Hilfe einer Kalibrationsgerade quantifiziert.

Für die Probe wurden 2 mg Festsubstanz in ein 4 ml Gläschen genau eingewogen. 1 ml Phosphatpuffer pH 6.5 wurde hinzugefügt. Diese Suspension ließ man 24 h auf einem Rührer bei Raumtemperatur rühren. Anschließend wurde die Lösung zentrifugiert. Für die Erstellung des Vergleichs für die Kalibration wurden 2 mg Festsubstanz genau eingewogen und in 30 ml Acetonitril gelöst. Nach kurzer Ultraschallbehandlung wurde die Lösung mit Wasser auf 50 ml verdünnt.

Probe und Vergleich wurden mittels HPLC mit UV-Detektion quantifiziert. Jede Probe wurde dreimal pro Injektionsvolumen (5 und 50 μl) eingespritzt. Für den Vergleich wurden drei Injektionsvolumina (5 μl, 10 μl und 20 μl) eingespritzt.

Folgende Chromatographie-Bedingungen wurden gewählt:

| | |
|---|---|
| HPLC Säule: | Xterra MS C18 2.5 μm 4.6 x 30 mm |
| Injektionsvolumina: | Probe: 3x5μl and 3x50μl |
| | Vergleich: 5μl, 10μl, 20μl |
| Flußrate: | 1.5 ml/min |
| Mobile Phase: | saurer Gradient: |
| | A: Wasser / 0.01% Trifluoressigsäure (TFA) |
| | B: Acetonitril / 0.01 % TFA |
| | 0 min → 95%A 5%B |
| | 0-3 min → 35%A 65%B, linearer Gradient |
| | 3-5 min → 35%A 65%B, isokratisch |
| | 5-6 min → 95%A 5%B, isokratisch |
| UV Detektor: eine Wellenlänge in der Nähe des Absorptionsmaximums (zwischen 200 und 400 nm) | |

Die Flächen der Probe- und Vergleichsinjektionen, sowie die Berechnung der Löslichkeit (in mg/l) wurden mit der HPLC Software (Waters Empower 2 FR) bestimmt.

Für die erfindungsgemäße Verbindung Beispiel 2 wurde eine Löslichkeit von 354 mg/l gemessen, der bekannte AKR1C3 Inhibitor EM-1404 zeigte eine Löslichkeit von 0.1 mg/l.

**Beispiel 34 (Endometriosemodell)**

Zur Untersuchung der *in vivo* Wirksamkeit der Beispielverbindung Beispiel 2 wurde ein Endometriosemodell in Weissbüschelaffen verwendet. Dabei werden weibliche Weissbüschelaffen im Alter zwischen 4 und 8 Jahren eingesetzt (Körpergewicht zwischen 340 und 460 g). In diesen Tieren wurde Endometriose induziert, indem während einer Laparotomie der Uterus punktiert und mit sterilem Medium gespült wurde, so dass dabei uterine Zellen über die Eileiter in den Abdomen gelangen [Einspanier et al., MolHum Reprod 2006]. Die Prozedur wird nach 3 Monaten wiederholt. Vor dem eigentlichen Behandlungsbeginn werden die Tiere einer Laparotomie unterzogen und auf das Vorhandensein endometriotischer Läsionen auf der Blase, am Uterus und auf den Ovarien hin untersucht. 6 Wochen später war Behandlungsbeginn. Es wurden zwei Behandlungsgruppen eingesetzt bei einer Gruppengröße von n=6 Tieren pro Gruppe. Gruppe 1 wurde nur mit Vehikel (Erdbeer-/Bananensaft) behandelt, Gruppe 2 mit der im Vehikel verabreichten Testsubstanz. Es wurden 30 mg/kg der Testsubstanz einmal pro Tag per os verabreicht. Die Behandlungsdauer betrug 6 Wochen. Unmittelbar am Ende der Behandlung wurde eine 2. Laparoskopie durchgeführt und die Anzahl und Größe der Läsionen auf Uterus, Ovarien und Blase erneut bestimmt. Da auf den Ovarien sowohl vor als auch nach der Behandlung kaum Läsionen gefunden wurden, wurde das Ovar als Läsionsart bei der Auswertung nicht berücksichtigt.

**Patentansprüche**

**1.** Verbindungen der Formel (I),

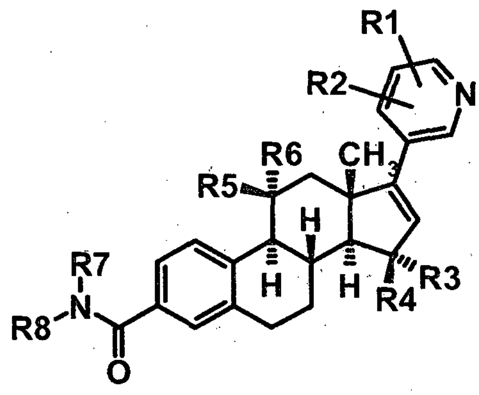

(I)

worin

R1 und R2 unabhängig voneinander Wasserstoff, Fluor, Chlor, Nitril, Trifluormethyl, Pentafluorethyl, Methoxy, Ethoxy, Trifluormethoxy, $-OCH_2CF_3$, $CH_3SO_2$-, $CH_3CH_2SO_2$-,
-(C=O)$CH_3$, Carboxyl, $C_1$-$C_4$-Alkyl, Hydroxyl, $-CH_2OH$, $-C(CH_3)_2OH$, $-CONH_2$, -(C=O)NHAlkyl, -(C=O)$N(CH_3)_2$, $-SO_2NH_2$, $-SO_2NH(CH_3)$, $-SO_2N(CH_3)_2$ oder den Ersatz einer C-H Gruppe im Pyridinring durch ein Stickstoffatom und
R3 und R4 Wasserstoff oder
R3 Hydroxyl, Fluor, Methoxy oder Ethoxy und R4 Wasserstoff oder
R3 Wasserstoff und R4 für Hydroxyl, Fluor, Methoxy oder Ethoxy und
R5 und R6 Wasserstoff oder
R5 Fluor, Hydroxyl, Methoxy oder Ethoxy und R6 Wasserstoff oder
R5 Wasserstoff und R6 Fluor und
R7 Wasserstoff, $C_1$-$C_4$-Alkyl, $C_3$-$C_6$-Cycloalkyl, Cyclopropylmethyl, Trifluormethyl oder 2,2,2-Trifluorethyl und
R8 $-CR^aR^b$ -COOH wobei
$R^a$ und $R^b$ unabhängig voneinander Wasserstoff, Methyl, Ethyl oder
$R^a$ und $R^b$ gemeinsam $-(CH_2)_n$- mit n = 2, 3, 4 oder 5, wobei bis zu 4 Wasserstoffatome der $CH_2$-Gruppen durch Fluoratome ersetzt sein können oder
$R^a$ und $R^b$ gemeinsam $-CH_2$-O-$CH_2$-, $-CH_2$-NH-$CH_2$-, $-CH_2$-$N(CH_3)$-$CH_2$-, $-CH_2CH_2$-O-$CH_2CH_2$-, $-CH_2CH_2$-NH-$CH_2CH_2$-, $-CH_2CH_2$-$N(CH_3)$-$CH_2CH_2$- oder

$R^a$ Wasserstoff, Methyl oder Ethyl und $R^b$ gemeinsam mit R7 $-(CH_2)_n-$ mit n = 1, 2, 3, 4, wobei bis zu 4 Wasserstoffatome der $CH_2$-Gruppen durch Fluoratome ersetzt sein können oder
$R^a$ gemeinsam mit R7 $-CH_2-O-CH_2CH_2-$, $-CH_2-N(CH_3)-CH_2CH_2-$ und $R^b$ Wasserstoff, Methyl oder Ethyl oder $-CR^cR^d-CR^eR^f-COOH$ wobei
$R^c$, $R^d$, $R^e$ $R^f$ Wasserstoff oder,
$R^c$, $R^d$ unabhängig voneinander Methyl, Ethyl oder gemeinsam $-(CH_2)_n-$ mit n = 2, 3, 4, 5 oder $-CH_2CH_2-O-CH_2CH_2-$ und $R^e$, $R^f$ Wasserstoff oder
$R^c$, $R^d$ Wasserstoff und $R^e$, $R^f$ unabhängig voneinander Methyl, Ethyl oder gemeinsam $-(CH_2)n-$ mit n = 2, 3, 4, 5, $-CH_2CH_2-O-CH_2CH_2-$, $-CH_2CH_2-NH-CH_2CH_2-$, $-CH_2CH_2-N(CH_3)-CH_2CH_2-$ oder $-CH_2-O-CH_2-$ oder
$R^c$ Methyl, Ethyl, Trifluormethyl und $R^d$, $R^c$ und $R^f$ Wasserstoff oder
$R^c$, $R^d$ und $R^f$ Wasserstoff und $R^e$ Methyl, Ethyl, Trifluormethyl, Hydroxyl oder Methoxy oder
$R^c$ und $R^e$ gemeinsam $-(CH_2)_n-$ mit n = 1,2,3 oder 4 und $R^d$ und $R^f$ Wasserstoff oder
$-CH_2-CH_2-CHR^g-COOH$ wobei
$R^g$ Wasserstoff oder
$R^g$ und R7 gemeinsam $-CH_2-$ oder $-CH_2CH_2-$

bedeuten und deren Salze, Solvate und Solvate der Salze.

2. Verbindungen nach Anspruch 1 der Formel (II) und der Formel (III)

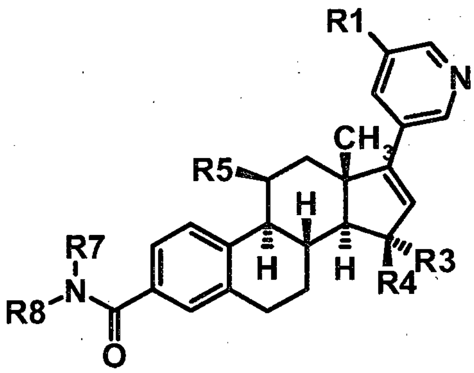

(II)

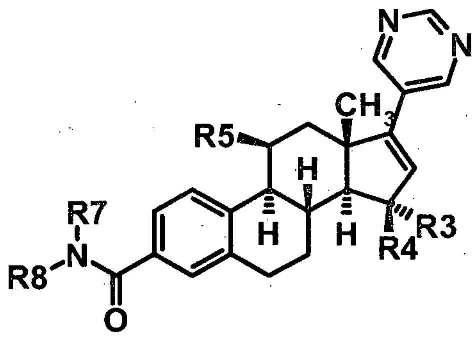

(III)

worin

R1 Wasserstoff, Fluor, Chlor, Nitril, Methoxy, Ethoxy, Trifluormethoxy, Methyl, Ethyl, Trifluormethyl, $-(C=O)CH_3$ und
R3 und R4 Wasserstoff oder
R3 Hydroxyl und R4 Wasserstoff oder
R3 Wasserstoff und R4 Hydroxyl und

R5 Wasserstoff oder Fluor und
R7 Wasserstoff oder $C_1$-$C_4$-Alkyl und
R8 -$CR^aR^b$ -COOH wobei
$R^8$ und $R^b$ unabhängig voneinander Wasserstoff, Methyl oder Ethyl oder
$R^a$ und $R^b$ gemeinsam -$(CH_2)_n$- mit n = 2, 3, 4 oder 5 oder
$R^a$ Wasserstoff und $R^b$ gemeinsam mit R7 --$(CH_2)_n$- mit n = 3 oder 4 oder
-$CR^cR^d$-$CR^eR^f$-COOH wobei
$R^c$, $R^d$, $R^e$ $R^f$ Wasserstoff oder
$R^c$, $R^d$ Wasserstoff und $R^e$, $R^f$ unabhängig voneinander Methyl, Ethyl oder gemeinsam -$(CH_2)$n- mit n = 2, 3, 4, 5 oder -$CH_2CH_2$-O-$CH_2CH_2$- oder
$R^c$ Methyl oder Ethyl und $R^d$, $R^e$ und $R^f$ Wasserstoff oder
$R^c$ und $R^e$ gemeinsam -$(CH_2)_n$- mit n = 1, 2, 3 oder 4 und $R^d$ und $R^f$ Wasserstoff oder
-$CH_2$-$CH_2$-$CHR^g$-COOH wobei
$R^g$ Wasserstoff oder
$R^g$ und R7 gemeinsam-$CH_2CH_2$-

bedeuten und deren Salze, Solvate und Solvate der Salze.

**3.** Verbindungen nach Anspruch 1 oder 2 der Formel (II) und der Formel (III) worin

R1 Wasserstoff, Fluor, Chlor, Nitril, Methoxy, Trifluormethyl und
R3 und R4 Wasserstoff oder
R3 Hydroxyl und R4 Wasserstoff oder
R3 Wasserstoff und R4 Hydroxyl und
R5 Wasserstoff oder Fluor und
R7 Wasserstoff, Methyl oder Ethyl und
R8 -$CR^aR^b$-COOH wobei
$R^a$ und $R^b$ unabhängig voneinander Wasserstoff, Methyl oder Ethyl oder
$R^a$ Wasserstoff und $R^b$ gemeinsam mit R7 -$(CH_2)_n$- mit n = 3 oder 4 oder
-$CR^cR^d$-$CR^eR^f$-COOH wobei
$R^c$, $R^d$, $R^e$ $R^f$ Wasserstoff oder
$R^c$, $R^d$ Wasserstoff und $R^e$, $R^f$ unabhängig voneinander Methyl oder Ethyl oder gemeinsam -$(CH_2)$n- mit n = 2, 4, 5 oder -$CH_2CH_2$-O-$CH_2CH_2$- oder
$R^c$ Methyl und $R^d$, $R^e$ und $R^f$ Wasserstoff oder
$R^c$ und $R^e$ gemeinsam -$(CH_2)_n$- mit n = 3 oder 4 und $R^d$ und $R^f$ Wasserstoff oder
-$CH_2$-$CH_2$-$CHR^g$-COOH wobei
$R^g$ Wasserstoff oder
$R^g$ und R7 gemeinsam -$CH_2CH_2$-

bedeuten und deren Salze, Solvate und Solvate der Salze.

**4.** Verbindungen nach Anspruch 1, 2 oder 3der Formel (II) und der Formel (III) worin

R1 Wasserstoff, Fluor, Methoxy, Trifluormethyl und
R3 und R4 Wasserstoff oder
R3 Hydroxyl und R4 Wasserstoff oder
R3 Wasserstoff und R4 Hydroxyl und
R5 Wasserstoff oder Fluor und
R7 Wasserstoff oder Methyl und
R8 -$CR^aR^b$ -COOH worin
$R^a$ und $R^b$ unabhängig voneinander Wasserstoff oder Methyl oder
$R^a$ Wasserstoff und $R^b$ gemeinsam mit R7 -$(CH_2)_3$- oder
-$CR^cR^d$-$CR^cR^f$-COOH
$R^c$, $R^d$, $R^e$ $R^f$ Wasserstoff, oder
$R^c$ und $R^d$ Wasserstoff und $R^e$ und $R^f$ Methyl oder gemeinsam -$(CH_2)$n- mit n = 2 oder 4 oder -$CH_2CH_2$-O-$CH_2CH$ oder
$R^c$ Methyl und $R^d$, $R^e$ und $R^f$ Wasserstoff oder
$R^c$ und $R^c$ gemeinsam -$(CH_2)_3$- und $R^d$ und $R^f$ Wasserstoff oder

$-CH_2-CH_2-CHR^g-COOH$
$R^g$ Wasserstoff oder
$R^g$ und R7 $-CH_2CH_2-$

bedeuten und deren Salze, Solvate und Solvate der Salze.

**5.** Verbindungen nach Anspruch 1 bis 4 mit der Bezeichnung

4-[({[17-(5-Fluorpyridin-3-yl)estra-1,3,5(10),16-tetraen-3-yl]carbonyl}amino) methyl]-3,4,5,6-tetrahydro-2*H*-pyran-4-carbonsäure
*N*-{[17-(5-Fluorpyridin-3-yl)estra-1,3,5(10),16-tetraen-3-yl]carbonyl}-*N*-methyl-β-alanin
1-[({[17-(5-Fluorpyridin-3-yl)estra-1,3,5(10),16-tetraen-3-yl]carbonyl}amino) methyl]cyclopropan-1-carbonsäure
1-[({[17-(5-Fluorpyridin-3-yl)estra-1,3,5(10),16-tetraen-3-yl]carbonyl}amino)methyl] cyclopentan-1-carbonsäure
3-({[17-(5-Fluorpyridin-3-yl)estra-1,3,5(10),16-tetraen-3-yl]carbonyl}amino)-2,2-dimethylpropansäure
1-{[17-(5-Fluorpyridin-3-yl)estra-1,3,5(10),16-tetraen-3-yl]carbonyl}piperidin-4-carbonsäure
*N*-{[17-(5-Fluorpyridin-3-yl)estra-1,3,5(10),16-tetraen-3-yl]carbonyl}-2-methylalanin
4-({[17-(5-Fluorpyridin-3-yl)estra-1,3,5(10),16-tetraen-3-yl]carbonyl}amino)butansäure
*N*-{[17-(5-Fluorpyridin-3-yl)estra-1,3,5(10),16-tetraen-3-yl]carbonyl}-β-alanin
*N*-{[17-(5-Fluorpyridin-3-yl)estra-1,3,5(10),16-tetraen-3-yl]carbonyl}glycin
(1*R**,2*S**)-2-({[17-(5-Fluorpyridin-3-yl)estra-1,3,5(10),16-tetraen-3-yl]carbonyl} amino)cyclopentan-1-carbonsäure
(*S*)-3-({[17-(5-Fluorpyridin-3-yl)estra-1,3,5(10),16-tetraen-3-yl]carbonyl}amino) butansäure
(*R*)-3-({[17-(5-Fluorpyridin-3-yl)estra-1,3,5(10),16-tetraen-3-yl]carbonyl}amino) butansäure
3-({[17-(5-Methoxypyridin-3-yl)estra-1,3,5(10),16-tetraen-3-yl]carbonyl}amino)-2,2-dimethylpropansäure
*N*-{[17-(5-Methoxypyridin-3-yl)estra-1,3,5(10),16-tetraen-3-yl]carbonyl}-β-alanin *N*-{[17-(5-Methoxypyridin-3-yl)estra-1,3,5(10),16-tetraen-3-yl]carbonyl}-*N*-methyl-β-alanin
*N*-{[17-(Pyrimidin-5-yl)estra-1,3,5(10),16-tetraen-3-yl]carbonyl}-β-alanin
4-({[17-(Pyrimidin-5-yl)estra-1,3,5(10),16-tetraen-3-yl]carbonyl}amino)butansäure
*N*-Methyl-*N*-{[17-(pyrimidin-5-yl)estra-1,3,5(10),16-tetraen-3-yl]carbonyl}-β-alanin
2,2-Dimethyl-3-({[17-(pyrimidin-5-yl)estra-1,3,5(10),16-tetraen-3-yl]carbonyl}amino) propansäure
*N*-({17-[5-(Trifluormethyl)pyridin-3-yl]estra-1,3,5(10),16-tetraen-3-yl}carbonyl)-β-alanin
*N*-Methyl-*N*-({17-[5-(trifluormethyl)pyridin-3-yl]estra-1,3,5(10),16-tetraen-3-yl} carbonyl)-β-alanin
*N*-{[17-(5-Fluorpyridin-3-yl}estra-1,3,5(10),16-tetraen-3-yl]carbonyl}-L-prolin
*N*-{[17-(5-Fluorpyridin-3-yl)estra-1,3,5(10),16-tetraen-3-yl]carbonyl}-D-prolin
4-({[11β-Fluor-17-(5-fluorpyridin-3-yl)estra-1,3,5(10),16-tetraen-3-yl]carbonyl} amino)butansäure
*N*-{[17-(5-Fluorpyridin-3-yl)-15α-hydroxyestra-1,3,5(10),16-tetraen-3-yl]carbonyl}-*N*-methyl-β-alanin
*N*-{[17-(5-Fluorpyridin-3-yl)-15β-hydroxyestra-1,3,5(10),16-tetraen-3-yl]carbonyl}-*N*-methyl-β-alanin
N-Methyl-N-{[17-(6-methylpyridazin-4-yl)estra-1,3,5(10),16-tetraen-3-yl]carbonyl}-β-alanin
N-Methyl-N-{[17-(3-pyridin-3-yl)estra-1,3,5(10),16-tetraen-3-yl]carbonyl}-β-alanin
4-({[17-(5-Methoxypyridin-3-yl)estra-1,3,5(10),16-tetraen-3-yl]carbonyl}amino)butansäure

und deren Salze, Solvate und Solvate der Salze.

**6.** Verbindungen nach Anspruch 1, 2, 3, 4 oder 5 zur Behandlung und/oder Prophylaxe von Krankheiten.

**7.** Verwendung einer Verbindung nach Anspruch 1, 2, 3, 4 oder 5 zur Herstellung eines Arzneimittels zur Behandlung und/oder Prophylaxe von Krankheiten.

**8.** Verbindungen nach Anspruch 1, 2, 3, 4 oder 5 zur Behandlung und/oder Prophylaxe der Endometriose, von uterinen Leiomyomen, von uterinen Blutungsbeschwerden, der Dysmenorrhö, des Prostata Karzinoms, der Prostata Hyperplasie, der Akne, der Seborrhö, des Haarausfalls, der frühzeitigen Geschlechtsreife, des polyzystischen ovariellen Syndroms, des Brustkrebses, des Lungenkrebses, des Endometriumkarzinoms, des Nierenzellkarzinoms, des Blasenkarzinoms, der Non-Hodgkin-Lymphome, der chronisch obstruktiven Lungenerkrankungen (COPD), der Adipositas, oder des inflammatorischen Schmerzes.

**9.** Arzneimittel enthaltend eine Verbindung nach Anspruch 1, 2, 3, 4 oder 5 in Kombination mit einem oder mehreren

weiteren Wirkstoff(en), insbesondere mit selektiven Estrogen Rezeptor Modulatoren (SERMs), Estrogenrezeptor (ER) Antagonisten, Aromataseinhibitoren, 17β HSD1 Inhibitoren, Steroid Sulfatase (STS)-Inhibitoren, GnRH-Agonisten und -Antagonisten, Kisspeptin Rezeptor (KISSR)-Antagonisten, selektiven Androgen Rezeptor Modulatoren (SARMs), Androgenen, 5α-Reduktase Inhibitoren, selektiven Progesteron Rezeptor Modulatoren (SPRMs), Gestagenen, Antigestagenen, orale Kontrazeptiva, Inhibitoren der Mitogen Aktivierten Protein (MAP) Kinasen sowie Inhibitoren der MAP Kinasen Kinasen (Mkk3/6, Mek1/2, Erkl/2), Inhibitoren der Proteinkinasen B (PKBα/β/γ; Akt1/2/3), Inhibitoren der Phosphoinositid-3-Kinasen (PI3K), Inhibitoren der Cyclin-abhängigen Kinase (CDK1/2), Inhibitoren des Hypoxie Induzierten Signalweges (HIF1alpha Inhibitoren, Aktivatoren der Prolylhydroxylasen), Histon Deacetylase (HDAC)-Inhibitoren, Prostaglandin F Rezeptor (FP) (PTGFR)-Antagonisten und Nicht-steroidale Entzündungshemmern (NSAIDs).

## Claims

**1.** Compounds of the formula (I)

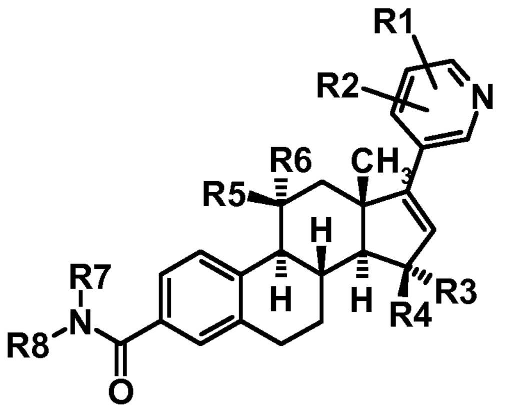

(I) ,

in which

R1 and R2 independently of one another represent hydrogen, fluorine, chlorine, nitrile, trifluoromethyl, pentafluoroethyl, methoxy, ethoxy, trifluoromethoxy, $-OCH_2CF_3$, $CH_3SO_2$-, $CH_3CH_2SO_2$-, - (C=O)$CH_3$, carboxyl, $C_1$-$C_4$-alkyl, hydroxy, $-CH_2OH$, $-C(CH_3)_2OH$, $-CONH_2$, -(C=O)NH-alkyl, -(C=O)$N(CH_3)_2$, $-SO_2NH_2$, $-SO_2NH(CH_3)$, $-SO_2N(CH_3)_2$ or the replacement of a C-H group in the pyridine ring by a nitrogen atom and
R3 and R4 represent hydrogen or
R3 represents hydroxy, fluorine, methoxy or ethoxy and R4 represents hydrogen or
R3 represents hydrogen and R4 represents hydroxy, fluorine, methoxy or ethoxy and
R5 and R6 represent hydrogen or
R5 represents fluorine, hydroxy, methoxy or ethoxy and R6 represents hydrogen or
R5 represents hydrogen and R6 represents fluorine and
R7 represents hydrogen, $C_1$-$C_4$-alkyl, $C_3$-$C_6$-Cycloalkyl, cyclopropylmethyl, trifluoromethyl or 2,2,2-trifluoroethyl and
R8 represents $-CR^aR^b$ -COOH where
$R^a$ and $R^b$ independently of one another represent hydrogen, methyl, ethyl or
$R^a$ and $R^b$ together represent $-(CH_2)_n-$ where n = 2, 3, 4 or 5, where up to 4 hydrogen atoms of the $CH_2$ groups may be replaced by fluorine atoms or
$R^a$ and $R^b$ together represent $-CH_2-O-CH_2-$, $-CH_2-NH-CH_2-$, $-CH_2-N(CH_3)-CH_2-$, $-CH_2CH_2-O-CH_2CH_2-$, $-CH_2CH_2-NH-CH_2CH_2-$, $-CH_2CH_2-N(CH_3)-CH_2CH_2-$ or
$R^a$ represents hydrogen, methyl or ethyl and $R^b$ together with R7 represents - $(CH_2)_n$- where n = 1, 2, 3, 4, where up to 4 hydrogen atoms of the $CH_2$ groups may be replaced by fluorine atoms or
$R^a$ together with R7 represents $-CH_2-O-CH_2CH_2-$, - $CH_2-N(CH_3)-CH_2CH_2-$ and $R^b$ represents hydrogen, methyl or ethyl or
represents $-CR^cR^d-CR^eR^f-COOH$ where

$R^c$, $R^d$, $R^e$, $R^f$ represent hydrogen or
$R^c$, $R^d$ independently of one another represent methyl, ethyl or together represent $-(CH_2)_n-$where n = 2, 3, 4, 5 or $-CH_2CH_2-O-CH_2CH_2-$ and $R^e$, $R^f$ represent hydrogen or
$R^c$, $R^d$ represent hydrogen and $R^e$, $R^f$ independently of one another represent methyl, ethyl or together represent $-(CH_2)n-$ where n = 2, 3, 4, 5, $-CH_2CH_2-O-CH_2CH_2-$, $-CH_2CH_2-NH-CH_2CH_2-$, $-CH_2CH_2-N(CH_3)-CH_2CH_2-$ or $-CH_2-O-CH_2-$ or
$R^c$ represents methyl, ethyl, trifluoromethyl and $R^d$, $R^e$ and $R^f$ represent hydrogen or
$R^c$, $R^d$ and $R^f$ represent hydrogen and $R^e$ represents methyl, ethyl, trifluoromethyl, hydroxy or methoxy or
$R^c$ and $R^e$ together represent $-(CH_2)_n-$ where n = 1, 2, 3 or 4 and $R^d$ and $R^f$ represent hydrogen or
represents $-CH_2-CH_2-CHR^g-COOH$ where
$R^g$ represents hydrogen or
$R^g$ and R7 together represent $-CH_2-$ or $-CH_2CH_2-$

and their salts, solvates and solvates of the salts.

**2.** Compounds according to Claim 1 of the formula (II) and the formula (III)

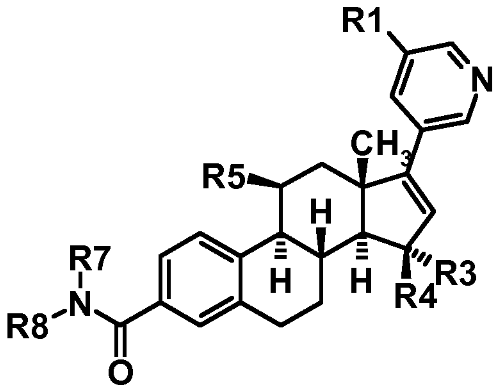

**(II)**

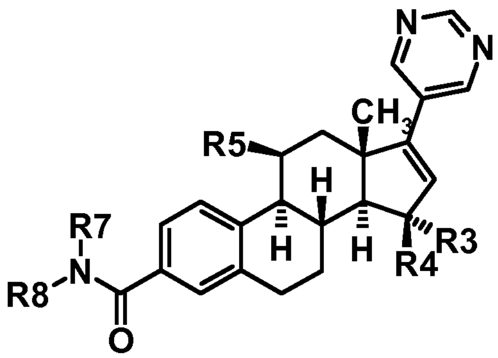

**(III)** ,

in which

R1 represents hydrogen, fluorine, chlorine, nitrile, methoxy, ethoxy, trifluoromethoxy, methyl, ethyl, trifluoromethyl, $-(C=O)CH_3$ and
R3 and R4 represent hydrogen or
R3 represents hydroxy and R4 represents hydrogen or
R3 represents hydrogen and R4 represents hydroxy and
R5 represents hydrogen or fluorine and

R7 represents hydrogen or $C_1$-$C_4$-alkyl and
R8 represents $-CR^aR^b-COOH$ where
$R^a$ and $R^b$ independently of one another represent hydrogen, methyl or ethyl or
$R^a$ and $R^b$ together represent $-(CH_2)_n-$ where n = 2, 3, 4 or 5 or
$R^a$ represents hydrogen and $R^b$ together with R7 represents $-(CH_2)_n-$ where n = 3 or 4 or
represents $-CR^cR^d-CR^eR^f-COOH$ where
$R^c$, $R^d$, $R^e$, $R^f$ represent hydrogen or
$R^c$, $R^d$ represent hydrogen and $R^e$, $R^f$ independently of one another represent methyl, ethyl or together represent $-(CH_2)n-$ where n = 2, 3, 4, 5 or $-CH_2CH_2-O-CH_2CH_2-$ or
$R^c$ represents methyl or ethyl and $R^d$, $R^e$ and $R^f$ represent hydrogen or
$R^c$ and $R^e$ together represent - $(CH_2)_n$- where n = 1, 2, 3 or 4 and $R^d$ and $R^f$ represent hydrogen or
represents $-CH_2-CH_2-CHR^g-COOH$ where
$R^g$ represents hydrogen or
$R^g$ and R7 together represent $-CH_2CH_2-$

and their salts, solvates and solvates of the salts.

**3.** Compounds according to Claim 1 or 2 of the formula (II) and the formula (III), in which

R1 represents hydrogen, fluorine, chlorine, nitrile, methoxy, trifluoromethyl and
R3 and R4 represent hydrogen or
R3 represents hydroxy and R4 represents hydrogen or
R3 represents hydrogen and R4 represents hydroxy and
R5 represents hydrogen or fluorine and
R7 represents hydrogen, methyl or ethyl and
R8 represents $-CR^aR^b-COOH$ where
$R^a$ and $R^b$ independently of one another represent hydrogen, methyl or ethyl or
$R^a$ represents hydrogen and $R^b$ together with R7 represents - $(CH_2)_n$- where n = 3 or 4 or
represents $-CR^cR^d-CR^eR^f-COOH$ where
$R^c$, $R^d$, $R^e$, $R^f$ represent hydrogen or
$R^c$, $R^d$ represent hydrogen and $R^e$, $R^f$ independently of one another represent methyl or ethyl or together represent $-(CH_2)n-$ where n = 2, 4, 5 or represent $-CH_2CH_2-O-CH_2CH_2-$ or
$R^c$ represents methyl and $R^d$, $R^e$ and $R^f$ represent hydrogen or
$R^c$ and $R^e$ together represent $-(CH_2)_n-$ where n = 3 or 4 and $R^d$ and $R^f$ represent hydrogen or
represents $-CH_2-CH_2-CHR^g-COOH$ where $R^g$ represents hydrogen or
$R^g$ and R7 together represent $-CH_2CH_2-$

and their salts, solvates and solvates of the salts.

**4.** Compounds according to Claim 1, 2 or 3 of the formula (II) and the formula (III), in which

R1 represents hydrogen, fluorine, methoxy, trifluoromethyl and
R3 and R4 represent hydrogen or
R3 represents hydroxy and R4 represents hydrogen or
R3 represents hydrogen and R4 represents hydroxy and
R5 represents hydrogen or fluorine and
R7 represents hydrogen or methyl and
R8 represents $-CR^aR^b-COOH$ where
$R^a$ and $R^b$ independently of one another represent hydrogen or methyl or
$R^a$ represents hydrogen and $R^b$ together with R7 represents $-(CH_2)_3-$ or
represents $-CR^cR^d-CR^eR^f-COOH$ where
$R^c$, $R^d$, $R^e$, $R^f$ represent hydrogen or
$R^c$ and $R^d$ represent hydrogen and $R^e$ and $R^f$ represent methyl or together represent $-(CH_2)n-$where n = 2 or 4 or represent $-CH_2CH_2-O-CH_2CH_2-$ or
$R^c$ represents methyl and $R^d$, $R^e$ and $R^f$ represent hydrogen or
$R^c$ and $R^e$ together represent $-(CH_2)_3-$ and $R^d$ and $R^f$ represent hydrogen or
represents $-CH_2-CH_2-CHR^g-COOH$ where
$R^g$ represents hydrogen or

$R^g$ and R7 represent $-CH_2CH_2-$

and their salts, solvates and solvates of the salts.

**5.** Compounds according to any of Claims 1 to 4 having the names

4-[({[17-(5-fluoropyridin-3-yl)estra-1,3,5(10),16-tetraen-3-yl]carbonyl}amino)methyl]-3,4,5,6-tetrahydro-2H-pyran-4-carboxylic acid
N-{[17-(5-fluoropyridin-3-yl)estra-1,3,5(10),16-tetraen-3-yl]carbonyl}-*N*-methyl-β-alanine
1-[({[17-(5-fluoropyridin-3-yl)estra-1,3,5(10),16-tetraen-3-yl]carbonyl}amino)methyl]cyclopropan-1-carboxylic acid
1-[({[17-(5-fluoropyridin-3-yl)estra-1,3,5(10),16-tetraen-3-yl]carbonyl}amino)methyl]cyclopentane-1-carboxylic acid
3-({[17-(5-fluoropyridin-3-yl)estra-1,3,5(10),16-tetraen-3-yl]carbonyl}amino)-2,2-dimethylpropanoic acid
1-{[17-(5-fluoropyridin-3-yl)estra-1,3,5(10),16-tetraen-3-yl]carbonyl}piperidine-4-carboxylic acid
*N*-{[17-(5-fluoropyridin-3-yl)estra-1,3,5(10),16-tetraen-3-yl]carbonyl}-2-methylalanine
4-({[17-(5-fluoropyridin-3-yl)estra-1,3,5(10),16-tetraen-3-yl]carbonyl}amino)butanoic acid
*N*-{[17-(5-fluoropyridin-3-yl)estra-1,3,5(10),16-tetraen-3-yl]carbonyl}-β-alanine
*N*-{[17-(5-fluoropyridin-3-yl)estra-1,3,5(10),16-tetraen-3-yl]carbonyl}glycine
(1*R**, 2*S**)-2-({[17-(5-fluoropyridin-3-yl)estra-1,3,5(10),16-tetraen-3-yl]carbonyl}amino)cyclopentane-1-carboxylic acid
(*S*)-3-({[17-(5-fluoropyridin-3-yl)estra-1,3,5(10),16-tetraen-3-yl]carbonyl}amino)butanoic acid
(*R*)-3-({[17-(5-fluoropyridin-3-yl)estra-1,3,5(10),16-tetraen-3-yl]carbonyl}amino)butanoic acid
3-({[17-(5-methoxypyridin-3-yl)estra-1,3,5(10),16-tetraen-3-yl]carbonyl}amino)-2,2-dimethylpropanoic acid
*N*-{[17-(5-methoxypyridin-3-yl)estra-1,3,5(10),16-tetraen-3-yl]carbonyl}-β-alanine
*N*-{[17-(5-methoxypyridin-3-yl)estra-1,3,5(10),16-tetraen-3-yl]carbonyl}-*N*-methyl-β-alanine
*N*-{[17-(pyrimidin-5-yl)estra-1,3,5(10),16-tetraen-3-yl]carbonyl}-β-alanine
4-({[17-(pyrimidin-5-yl)estra-1,3,5(10),16-tetraen-3-yl]carbonyl}amino)butanoic acid
*N*-methyl-N-{[17-(pyrimidin-5-yl)estra-1,3,5(10),16-tetraen-3-yl]carbonyl}-β-alanine
2,2-dimethyl-3-({[17-(pyrimidin-5-yl)estra-1,3,5(10),16-tetraen-3-yl]carbonyl}amino)propanoic acid
*N*-({17-[5-(trifluoromethyl)pyridin-3-yl]estra-1,3,5(10),16-tetraen-3-yl}carbonyl)-β-alanine
*N*-methyl-*N*-({17-[5-(trifluoromethyl)pyridin-3-yl]estra-1,3,5(10),16-tetraen-3-yl}carbonyl)-β-alanine
*N*-{[17-(5-fluoropyridin-3-yl)estra-1,3,5(10),16-tetraen-3-yl]carbonyl}-L-proline
*N*-{[17-(5-fluoropyridin-3-yl)estra-1,3,5(10),16-tetraen-3-yl]carbonyl}-D-proline
4-({[11β-fluoro-17-(5-fluoropyridin-3-yl)estra-1,3,5(10),16-tetraen-3-yl]carbonyl}amino)butanoic acid
*N*-{[17-(5-fluoropyridin-3-yl)-15α-hydroxyestra-1,3,5(10),16-tetraen-3-yl]carbonyl}-*N*-methyl-β-alanine
*N*-{[17-(5-fluoropyridin-3-yl)-15β-hydroxyestra-1,3,5(10),16-tetraen-3-yl]carbonyl}-*N*-methyl-β-alanine
N-methyl-N-{[17-(6-methylpyridazin-4-yl)estra-1,3,5(10),16-tetraen-3-yl]carbonyl}-β-alanine
N-methyl-N-{[17-(3-pyridin-3-yl)estra-1,3,5(10),16-tetraen-3-yl]carbonyl}-β-alanine
4-({[17-(5-methoxypyridin-3-yl)estra-1,3,5(10),16-tetraen-3-yl]carbonyl}amino)butanoic acid and their salts, solvates and solvates of the salts.

**6.** Compounds according to any of Claims 1, 2, 3, 4 or 5 for the treatment and/or prophylaxis of diseases.

**7.** Use of a compound according to any of Claims 1, 2, 3, 4 or 5 for preparing a medicament for the treatment and/or prophylaxis of diseases.

**8.** Compounds according to any of Claims 1, 2, 3, 4 or 5 for the treatment and/or prophylaxis of endometriosis, of uterine leiomyomas, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkin lymphomas, of chronic obstructive pulmonary disease (COPD), of adiposity or of inflammatory pain.

**9.** Medicament comprising a compound according to any of Claims 1, 2, 3, 4 or 5 in combination with one or more further active compound(s), in particular with selective oestrogen receptor modulators (SERMs), oestrogen receptor (ER) antagonists, aromatase inhibitors, 17β HSD1 inhibitors, steroid sulphatase (STS) inhibitors, GnRH agonists and antagonists, kisspeptin receptor (KISSR) antagonists, selective androgen receptor modulators (SARMs), androgens, 5α-reductase inhibitors, selective progesterone receptor modulators (SPRMs), gestagens, antigestagens,

oral contraceptives, inhibitors of mitogen activating protein (MAP) kinases and inhibitors of the MAP kinases kinases (Mkk3/6, Mek1/2, Erk1/2), inhibitors of the protein kinases B (PKBα/β/γ; Akt1/2/3), inhibitors of the phosphoinositide 3-kinases (PI3K), inhibitors of the cyclin-dependent kinase (CDK1/2), inhibitors of the hypoxia-induced signal path (HIF1alpha inhibitors, activators of prolyl hydroxylases), histone deacetylase (HDAC) inhibitors, prostaglandin F receptor (FP) (PTGFR) antagonists and non-steroidal antiflammatory drugs (NSAIDs).

**Revendications**

1. Composés de formule (I)

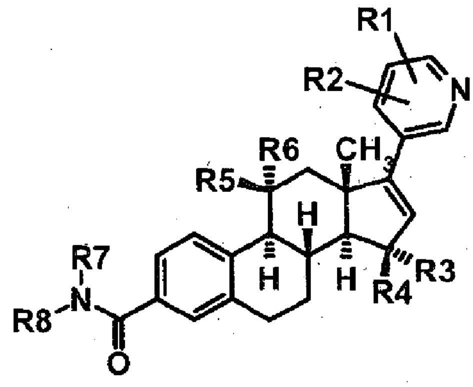

(I)

dans laquelle

R1 et R2 signifient, indépendamment l'un de l'autre, hydrogène, fluor, chlore, nitrile, trifluorométhyle, pentafluoroéthyle, méthoxy, éthoxy, trifluorométhoxy, $-OCH_2CF_3$, $CH_3SO_2$-, $CH_3CH_2SO_2$-, $-(C=O)CH_3$, carboxyle, $C_1$-$C_4$-alkyle, hydroxyle, $-CH_2OH$, $-C(CH_3)_2OH$, $-CONH_2$, -(C=O)NH-alkyle, $-(C=O)N(CH_3)_2$, $-SO_2NH_2$, $-SO_2NH(CH_3)$, $-SO_2N-(CH_3)_2$ ou le remplacement d'un groupe C-H dans le cycle pyridine par un atome d'azote et
R3 et R4 signifient hydrogène ou
R3 signifie hydroxyle, fluor, méthoxy ou éthoxy et R4 signifie hydrogène ou
R3 signifie hydrogène et R4 signifie hydroxyle, fluor, méthoxy ou éthoxy et
R5 et R6 signifient hydrogène ou

R5 signifie fluor, hydroxyle, méthoxy ou éthoxy et R6 signifie hydrogène ou
R5 signifie hydrogène et R6 signifie fluor et
R7 signifie hydrogène, $C_1$-$C_4$-alkyle, $C_3$-$C_6$-cycloalkyle, cyclopropylméthyle, trifluorométhyle ou 2,2,2-trifluoréthyle et
R8 signifie $-CR^aR^b-COOH$,
$R^a$ et $R^b$ signifiant, indépendamment l'un de l'autre, hydrogène, méthyle, éthyle ou
$R^a$ et $R^b$ signifiant, ensemble, $-(CH_2)_n-$ avec n = 2, 3, 4 ou 5, jusqu'à 4 atomes d'hydrogène des groupes $CH_2$ pouvant être remplacés par des atomes de fluor ou
$R^a$ et $R^b$ signifiant, ensemble, $-CH_2-O-CH_2-$, $-CH_2-NH-CH_2-$, $-CH_2-N(CH_3)$ $-CH_2-$, $-CH_2CH_2-O-CH_2CH_2-$, $-CH_2CH_2-NH-CH_2CH_2-$, $-CH_2CH_2-N(CH_3)-CH_2CH_2-$ ou
$R^a$ signifiant hydrogène, méthyle ou éthyle et $R^b$ signifiant, ensemble avec R7, - $(CH_2)_n$- avec n = 1, 2, 3, 4, jusqu'à 4 atomes d'hydrogène des groupes $CH_2$ pouvant être remplacés par des atomes de fluor ou $R^a$ signifiant, ensemble avec R7, $-CH_2-O-CH_2CH_2-$, $-CH_2-N(CH_3)-CH_2CH_2-$ et $R^b$ signifiant hydrogène, méthyle ou éthyle ou
$-CR^cR^d-CR^eR^f-COOH$
$R^c$, $R^d$, $R^e$, $R^f$ signifiant hydrogène ou
$R^c$, $R^d$ signifiant, indépendamment l'un de l'autre, méthyle, éthyle ou, ensemble, $-(CH_2)_n-$ avec n = 2, 3, 4, 5 ou $-CH_2CH_2-O-CH_2CH_2-$ et $R^e$, $R^f$ signifiant hydrogène ou
$R^c$, $R^d$ signifiant hydrogène et $R^e$, $R^f$ signifiant, indépendamment l'un de l'autre, méthyle, éthyle ou, ensemble, $-(CH_2)_n-$ avec n = 2, 3, 4, 5, $-CH_2CH_2-O-CH_2CH_2-$, $-CH_2CH_2-NH-CH_2CH_2-$, $-CH_2CH_2-N$ $(CH_3)-CH_2CH_2-$ ou $-CH_2-O-CH_2-$ ou

$R^c$ signifiant méthyle, éthyle, trifluorométhyle et $R^d$, $R^e$ et $R^f$ signifiant hydrogène ou
$R^c$, $R^d$ et $R^f$ signifiant hydrogène et $R^e$ signifiant méthyle, éthyle, trifluorométhyle, hydroxyle ou méthoxy ou
$R^c$ et $R^e$ signifiant, ensemble, $-(CH_2)_n-$ avec n = 1, 2, 3 ou 4 et $R^d$ et $R^f$ signifiant hydrogène ou $-CH_2-CH_2-CHR^g-COOH$
$R^g$ signifiant hydrogène ou
$R^g$ et R7 signifiant, ensemble, $-CH_2-$ ou $-CH_2CH_2-$

et leurs sels, solvates et solvates des sels.

**2.** Composés selon la revendication 1 de formule (II) et de formule (III)

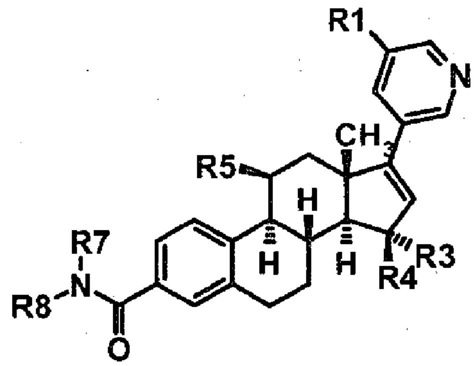

(II)

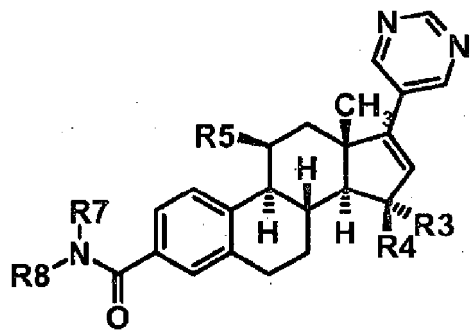

(III)

dans lesquelles

R1 signifie hydrogène, fluor, chlore, nitrile, méthoxy, éthoxy, trifluorométhoxy, méthyle, éthyle, trifluorométhyle, $-(C=O)CH_3$ et
R3 et R4 signifient hydrogène ou
R3
signifie hydroxyle et R4 signifie hydrogène ou
R3 signifie hydrogène et R4 signifie hydroxyle et
R5 signifie hydrogène ou fluor et
R7 signifie hydrogène ou $C_1$-$C_4$-alkyle et
R8 signifie - $CR^aR^b$-COOH
$R^a$ et $R^b$ signifiant, indépendamment l'un de l'autre, hydrogène, méthyle ou éthyle ou
$R^a$ et $R^b$ signifiant, ensemble, $-(CH_2)_n-$ avec n = 2, 3, 4 ou 5 ou
$R^a$ signifiant hydrogène et $R^b$ signifiant, ensemble avec R7, $-(CH_2)_n-$ avec n = 3 ou 4 ou - $CR^cR^d$-$CR^eR^f$-COOH
$R^c$, $R^d$, Re $R^f$ signifiant hydrogène ou
$R^a$, $R^d$ signifiant hydrogène et $R^e$, $R^f$ signifiant, indépendamment l'un de l'autre, méthyle, éthyle ou, ensemble,

$-(CH_2)_n-$ avec n = 2, 3, 4, 5 ou $-CH_2CH_2-O-CH_2CH_2-$ ou
$R^c$ signifiant méthyle ou éthyle et $R^d$, $R^e$ et $R^f$ signifiant hydrogène ou
$R^c$ et $R^e$ signifiant, ensemble, - $(CH_2)_n-$ avec n = 1, 2, 3 ou 4 et $R^d$ et $R^f$ signifiant hydrogène ou
$-CH_2-CH_2-CHR^g-COOH$
$R^g$ signifiant hydrogène ou
$R^g$ et R7 ensemble, signifiant $-CH_2CH_2-$

et leurs sels, solvates et solvates des sels.

**3.** Composés selon la revendication 1 ou 2 de formule (II) et de formule (III), dans lesquelles

R1 signifie hydrogène, fluor, chlore, nitrile, méthoxy, trifluorométhyle et
R3 et R4 signifient hydrogène ou

R3 signifie hydroxyle et R4 signifie hydrogène ou
R3 signifie hydrogène et R4 signifie hydroxyle et

R5 signifie hydrogène ou fluor et
R7 signifie hydrogène, méthyle ou éthyle et
R8 signifie $-CR^aR^b-COOH$
$R^a$ et $R^b$ signifiant, indépendamment l'un de l'autre, hydrogène, méthyle ou éthyle ou
$R^a$ signifiant hydrogène et $R^b$ signifiant, ensemble avec R7, $-(CH_2)_n-$ avec n = 3 ou 4 ou $-CR^cR^d-CR^eR^f-COOH$
$R^c$, $R^d$, $R^e$ $R^f$ signifiant hydrogène ou
$R^c$, $R^d$ signifiant hydrogène et $R^e$, $R^f$ signifiant, indépendamment l'un de l'autre, méthyle ou éthyle ou, ensemble, $-(CH_2)_n-$ avec n = 2, 4, 5 ou $-CH_2CH_2-O-CH_2CH_2-$ ou
$R^c$ signifiant méthyle et $R^d$, $R^e$ et $R^f$ signifiant hydrogène ou
$R^c$ et $R^e$ signifiant, ensemble, $-(CH_2)_n-$ avec n = 3 ou 4 et $R^d$ et $R^f$ signifiant hydrogène ou
$-CH_2-CH_2-CHR^g-COOH$
$R^g$ signifiant hydrogène ou
$R^g$ et R7 ensemble, signifiant $-CH_2CH_2-$

et leurs sels, solvates et solvates des sels.

**4.** Composés selon la revendication 1, 2 ou 3 de formule (II) et de formule (III), dans lesquelles

R1 signifie hydrogène, fluor, méthoxy, trifluorométhyle et
R3 et R4 signifient hydrogène ou

R3 signifie hydroxyle et R4 signifie hydrogène ou
R3 signifie hydrogène et R4 signifie hydroxyle et

R5 signifie hydrogène ou fluor et
R7 signifie hydrogène ou méthyle et
R8 signifie $-CR^aR^b-COOH$ où
$R^a$ et $R^b$ signifient, indépendamment l'un de l'autre, hydrogène ou méthyle ou
$R^a$ signifie hydrogène et $R^b$ signifie, ensemble avec R7, $-(CH_2)_3-$ ou
$-CR^cR^d-CR^eR^f-COOH$
$R^c$, $R^d$, $R^e$ $R^f$ signifiant hydrogène, ou
$R^c$ et $R^d$ signifiant hydrogène et $R^e$ et $R^f$ signifiant méthyle ou, ensemble, - $(CH_2)_n-$ avec n = 2 ou 4 ou $-CH_2CH_2-O-CH_2CH_2-$ ou
$R^c$ signifiant méthyle et $R^d$, $R^e$ et $R^f$ signifiant hydrogène ou
$R^c$ et $R^e$ signifiant, ensemble, $-(CH_2)_3-$ et $R^d$ et $R^f$ signifiant hydrogène ou
$-CH_2-CH_2-CHR^g-COOH$
$R^g$ signifiant hydrogène ou $R^g$ et R7 signifiant $-CH_2CH_2-$

et leurs sels, solvates et solvates des sels.

**5.** Composés selon la revendication 1 à 4, appelés acide 4-[({[17-(5-fluoropyridin-3-yl)estra-1,3,5(10), 16-tétraén-3-yl]carbonyl}amino)méthyl]-3,4,5,6-tétrahydro-2H-pyrane-4-carboxylique N-{[17-(5-fluoropyridin-3-yl)estra-1,3,5(10),16-tétraén-3-yl]carbonyl}-N-méthyl-β-alanine acide 1-[({[17-(5-fluoropyridin-3-yl)estra-1,3,5(10),16-tétraén-3-yl]carbonyl}amino)méthyl]cyclopropane-1-carboxylique acide 1-[({[17-(5-fluoropyridin-3-yl)estra-1,3,5(10),16-tétraén-3-yl]carbonyl}amino)méthyl]cyclopentane-1-carboxylique acide 3-({[17-(5-fluoropyridin-3-yl)estra-1,3,5(10),16-tétraén-3-yl]carbonyl}amino)-2,2-diméthylpropanoïque acide 1-{[17-(5-fluoropyridin-3-yl)estra-1,3,5(10),16-tétraén-3-yl]carbonyl}pipéridine-4-carboxylique N-{[17-(5-fluoropyridin-3-yl)estra-1,3,5(10),16-tétraén-3-yl]carbonyl}-2-méthylalanine acide 4-({[17-(5-fluoropyridin-3-yl)estra-1,3,5(10),16-tétraén-3-yl]carbonyl}amino)butanoïque N-{[17-(5-fluoropyridin-3-yl)estra-1,3,5(10),16-tétraén-3-yl]carbonyl}-β-alanine N-{[17-(5-fluoropyridin-3-yl)estra-1,3,5(10),16-tétraén-3-yl]carbonyl}glycine acide (1R*,2S*)-2-({[17-(5-fluoropyridin-3-yl)estra-1,3,5(10),16-tétraén-3-yl]carbonyl}amino)cyclopentane-1-carboxylique acide (S)-3-({[17-(5-fluoropyridin-3-yl)estra-1,3,5(10),16-tétraén-3-yl]carbonyl}amino)butanoïque acide (R)-3-({[17-(5-fluoropyridin-3-yl)estra-1,3,5(10),16-tétraén-3-yl]carbonyl}amino)butanoïque acide 3-({[17-(5-méthoxypyridin-3-yl)estra-1,3,5(10),16-tétraén-3-yl]carbonyl}amino)-2,2-diméthylpropanoïque N-{[17-(5-méthoxypyridin-3-yl)estra-1,3,5(10),16-tétraén-3-yl]carbonyl}-β-alanine N-{[17-(5-méthoxypyridin-3-yl)estra-1,3,5(10),16-tétraén-3-yl]carbonyl}-N-méthyl-β-alanine N-{[17-(pyrimidin-5-yl)estra-1,3,5(10),16-tétraén-3-yl]carbonyl}-β-alanine acide 4-({[17-(pyrimidin-5-yl)estra-1,3,5(10),16-tétraén-3-yl]carbonyl}amino)butanoïque N-méthyl-N-{[17-(pyrimidin-5-yl)estra-1,3,5(10),16-tétraén-3-yl]carbonyl}-β-alanine acide 2,2-diméthyl-3-({[17-(pyrimidin-5-yl)estra-1,3,5(10),16-tétraén-3-yl]carbonyl}amino)propanoïque N-({17-[5-(trifluorométhyl)pyridin-3-yl]estra-1,3,5(10),16-tétraén-3-yl}carbonyl)-β-alanine N-méthyl-N-({17-[5-(trifluorométhyl)pyridin-3-yl]estra-1,3,5(10),16-tétraén-3-yl]carbonyl)-β-alanine N-{[17-(5-fluoropyridin-3-yl)estra-1,3,5(10),16-tétraén-3-yl]carbonyl}-L-proline N-{[17-(5-fluoropyridin-3-yl)estra-1,3,5(10),16-tétraén-3-yl]carbonyl}-D-proline acide 4-({[11β-fluoro-17-(5-fluoropyridin-3-yl)estra-1,3,5(10),16-tétraén-3-yl]carbonyl}amino)butanoïque N-{[17-(5-fluoropyridin-3-yl)-15α-hydroxyestra-1,3,5(10),16-tétraén-3-yl]carbonyl}-N-méthyl-β-alanine N-{[17-(5-fluoropyridin-3-yl)-15β-hydroxyestra-1,3,5(10),16-tétraén-3-yl]carbonyl}-N-méthyl-β-alanine N-méthyl-N-{[17-(6-méthylpyridazin-4-yl)estra-1,3,5(10),16-tétraén-3-yl]carbonyl}-β-alanine N-méthyl-N-[17-(3-pyridin-3-yl)estra-1,3,5(10),16-tétraén-3-yl]carbonyl}-β-alanine acide 4-({[17(5-méthoxypyridin-3-yl)estra-1,3,5(10),16-tétraén-3-yl]carbonyl}amino)butanoïque et leurs sels, solvates et solvates des sels.

6. Composés selon la revendication 1, 2, 3, 4 ou 5 destinés au traitement et/ou à la prophylaxie de maladies.

7. Utilisation d'un composé selon la revendication 1, 2, 3, 4 ou 5 pour la préparation d'un médicament destiné au traitement et/ou à la prophylaxie de maladies.

8. Composés selon la revendication 1, 2, 3, 4 ou 5 pour le traitement et/ou la prophylaxie de l'endométriose, de léiomyomes utérins, de troubles hémorragiques utérins, de la dysménorrhée, du carcinome de la prostate, de l'hyperplasie prostatique, de l'acné, de la séborrhée, de l'alopécie, de la maturité sexuelle précoce, du syndrome des ovaires polykystiques, du cancer du sein, du cancer du poumon, du carcinome de l'endomètre, du carcinome à cellules rénales, du carcinome de la vessie, du lymphome non hodgkinien, des bronchopneumopathies chroniques obstructives (BPCO), de l'obésité ou de la douleur inflammatoire.

9. Médicament contenant un composé selon la revendication 1, 2, 3, 4 ou 5 en combinaison avec une ou plusieurs autres substances actives, en particulier avec des modulateurs sélectifs du récepteur des estrogènes (SERM), des antagonistes du récepteur des estrogènes (ER), des inhibiteurs d'aromatases, des inhibiteurs de 17ß HSD1, des inhibiteurs de la sulfatase stéroïde (STS), des agonistes et des antagonistes de GnRH, des antagonistes du récepteur de la kisspeptine (KISSR), des modulateurs sélectifs du récepteur des androgènes (SARM), des androgènes, des inhibiteurs de la 5α-réductase, des modulateurs sélectifs du récepteur des progestérones (SPRM), des gestagènes, des antigestagènes, des contraceptifs oraux, des inhibiteurs des protéine kinases activées par des agents mitogènes (MAP) ainsi que des inhibiteurs des MAP kinases kinases (Mkk3/6, Mek1/2, Erk1/2), des inhibiteurs des protéine kinases B (PKBα/β/γ ; Akt1/2/3), des inhibiteurs des phosphoinositide-3-kinases (PI3K), des inhibiteurs des kinases cycline-dépendantes (CDK1/2), des inhibiteurs de la voie de signalisation induite par l'hypoxie (inhibiteurs HIF1alpha, activateurs des prolylhydroxylases), des inhibiteurs de l'histone désacétylase (HDAC), des antagonistes du récepteur de la prostaglandine F (FP) (PTGFR) et des anti-inflammatoires non stéroïdiens (AINS).

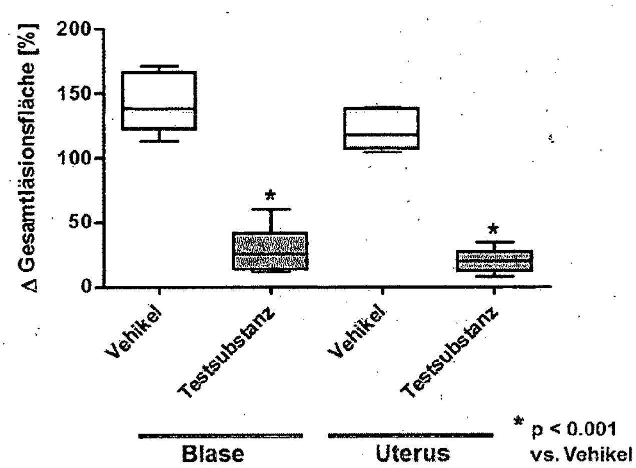


Abb. 1 Differenz der Läsionsflächen der auf dem Uterus bzw. der Blase befindlichen Läsionen in Prozent.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6541463 B, F. Labrie
- US 5604213 A, S. E. Barrie
- US 20050203075 A


### In der Beschreibung aufgeführte Nicht-Patentliteratur


- **T. M. PENNING.** Aldo-keto reductase (AKR) 1 C3: Role in prostate disease and the development of specific inhibitors. *Molecular and Cellular Endocrinology,* 2006, vol. 248 (1-2), 182-191
- **A. OSTER.** *J. Med. Chem,* 2010, vol. 53, 8176-8186
- **B. DELVOUX et al.** *J Clin Endocrinol Metab.,* 2009, vol. 94, 876-883
- **T. SMUC et al.** *Mol Cell Endocrinol.,* 25. Marz 2009, vol. 301 (1-2), 59-64
- **M.C. BYRNS ; Y. JIN ; T.M. PENNING.** *Journal of Steroid Biochemistry and Molecular Biology,* 2010
- **A. L. LOVERING.** *Cancer Res,* vol. 64 (5), 1802-1810
- **K. M. FUNG et al.** *Endocr Relat Cancer,* vol. 13 (1), 169-180
- **R. O. ROBERTS et al.** *Prostate,* vol. 66 (4), 392-404
- **T. L. RIZNER et al.** *Mol Cell Endocrinol,* 2006, vol. 248 (1-2), 126-135
- **K. QIN et al.** *J Endocrinol Metab,* 2006, vol. 91 (1), 270-276
- **Q. LAN et al.** *Carcinogenesis,* 2004, vol. 25 (11), 2177-2181
- **Q. LAN et al.** *Hum Genet,* 2007, vol. 121 (2), 161-168
- **L. COLOMBE et al.** *Exp Dermatol,* 2007, vol. 16 (9), 762-769
- **A. SVENSSON et al.** *Cell Mol Biol Lett,* 2008, vol. 13 (4), 599-613
- **J. D. FIGUEROA.** *Carcinogenesis,* 2008, vol. 29 (10), 1955-1962
- **J. BIRTHWISTLE.** *Mutat Res,* 2009, vol. 662 (1-2), 67-74
- **J. T. AZZARELLO.** *Int J Clin Exp Pathol,* 2009, vol. 3 (2), 147-155
- **M. C. BYMS.** *J Steroide Biochem Mol Biol,* 2010, vol. 118 (3), 177-187
- **C. HE.** *Hum Genet,* 2010, vol. 128 (5), 515-527
- **S. PIERROU.** *Am J Respir Crit Care,* 2007, vol. 175 (6), 577-586
- **JOANNA M DAY ; HELENA J TUTILL ; ATUL PUROHIT ; MICHAEL J REED.** *Endocrine-Related Cancer,* 2008, vol. 15, 665-692
- **P. BYDAL, VAN LUU-THE ; F. LABRIE ; D. POIRIER.** *European Journal of Medicinal Chemistry,* 2009, vol. 44, 632-644
- **D. DELUCA ; G. MOLLER ; A. ROSINUS ; W. ELGER ; A. HILLISCH ; J. ADAMSKI.** *Mol. Cell. Endocrinol.,* 2006, vol. 248, 218-224
- **V. M. MOREIRA et al.** *Current Medicinal Chemistry,* 2008, vol. 15 (9
- *Steroids,* 1995, vol. 60 (3), 299-306
- *J. Med. Chem.,* 1995, vol. 38, 2463-2471
- *J. Org. Chem.,* 1985, vol. 50, 1990-1992
- *JACS,* 2009, vol. 131, 9014-9019
- *Archiv der Pharmazie (Weinheim, Germany),* 2001, vol. 334 (12), 373-374
- **D. M. KNAPP et al.** *J. Am. Chem. Soc.,* 2009, vol. 131, 6961
- **G. A. MOLANDER et al.** *J. Org. Chem.,* 2009, vol. 74, 973
- **D. M. KNAPP.** *J. Am. Chem. Soc.,* 2009, vol. 131, 6961
- **G. A. MOLANDER.** *J. Org. Chem.,* 2009, vol. 74, 973
- **HALIM, M. ; YEE, D.J. ; SAMES, D.** *J. AM. CHEM. SOC.,* 2008, vol. 130, 14123-14128
- **YEE, D.J. ; BALSANEK, V. ; BAUMAN, D.R. ; PENNING, T.M. ; SÄMES, D.** *Proc. Natl. Acad. Sci. USA,* 2006, vol. 103, 13304-13309
- **DUFORT, I. ; RHEAULT, P. ; HUANG, XF. ; SOUCY, P. ; LUU-THE, V.** *Endocrinology,* 1999, vol. 140, 568-574
- *Urologe,* 2010, vol. 49, 64-68
- **EHMER, P. B. et al.** *J. Steroid Biochem. Mol. Biol.,* 2000, vol. 75, 57-63
- **EDWARD H. KERNS ; LI DI.** Solubility Methods in: Drug-like Properties: Concepts, Structure Design and Methods. Academic Press, 2008, 276-286